# EUROPÄISCHE PATENTSCHRIFT

(11) **EP 1 511 737 B1**
(45) Veröffentlichungstag und Bekanntmachung des Hinweises auf die Patenterteilung: **17.02.2010**
(21) Anmeldenummer: 03732395.3
(22) Anmeldetag: 16.05.2003
(51) Int. Cl.: C07D 237/04, C07D 401/12, A61K 31/50, A61P 37/00

(54) **ARYLOXIME**
ARYLOXIMES
ARYLOXYMES

(30) Priorität: 10.06.2002 DE 10225574
(43) Veröffentlichungstag der Anmeldung: 09.03.2005
(73) Patentinhaber: Merck Patent GmbH, 64293 Darmstadt (DE)
(72) Erfinder: EGGENWEILER, Hans-Michael, 64291 Darmstadt (DE); BEIER, Norbert, 64354 Reinheim (DE); SCHELLING, Pierre, 64367 Mühltal (DE); WOLF, Michael, 64297 Darmstadt (DE)
(86) Internationale Anmeldenummer: PCT/EP2003/005173
(87) Internationale Veröffentlichungsnummer: WO 2003/104205

(56) Entgegenhaltungen:
- WO-A-98/06704
- WO-A-99/65880

## Beschreibung

Die Erfindung betrifft Verbindungen der Formel I 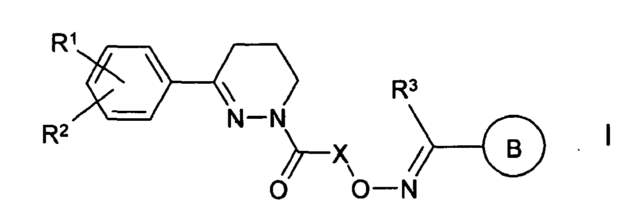 worin
- R¹, R²: jeweils unabhängig voneinander H, OH, OR⁸, -SR⁸, -SOR⁸, -SO₂R⁸ oder Hal bedeuten,
- R¹ und R²: zusammen auch -OCH₂O- oder -OCH₂CH₂O- bedeuten,
- R³: H, A"R⁷, COA"R⁷, COOA"R⁷, CONH₂, CONHA"R⁷, CON(A"R⁷)(A"'R⁷), CONR¹⁰Het, NH₂, NHA"R⁷, N(A"R⁷)(A"'R⁷), NCOA"R⁷ oder NCOOA"R⁷,
- B: Phenyl, Pyridyl, Pyridyl-N-oxid, Thienyl, Furyl, Pyrrolyl, Pyridazinyl, Pyrimidinyl, Pyrazinyl, Triazinyl, Isoxazolinyl, Oxazolinyl, Thiazolinyl, Pyrazolinyl, Imidazolinyl, Naphthyl, Chinolinyl, Isochinolinyl, Cinnolinyl, Phthalazinyl, Chinazolinyl oder Chinoxalinyl, das unsubstituiert ist oder ein-, zwei- oder dreifach durch R⁴, R⁵ und/oder R⁶ substituiert sein kann"
- X: Alkylen mit 1-10 C-Atomen oder Alkenylen mit 2-8 C-Atomen, worin eine, zwei oder drei CH₂-Gruppen durch O, S, SO, SO₂, NH oder NA"R⁷ ersetzt sein können,
1-7 H-Atome durch F und/oder Cl ersetzt sein können,
- R⁴, R⁵, R⁶: jeweils unabhängig voneinander H, A"R⁷, OH, OA"R⁷, NO₂, NH₂, NHA"R⁷, N(A"R⁷)(A"'R⁷), NHCOA"R⁷, NHCOOA"R⁷ , NHCONH₂, NHCONHA"R⁷, NHCON(A"R⁷)(A"'R⁷), Hal, COOH, COOA"R⁷, CONH₂, CONHA"R⁷, CON(A"R⁷)(A"'R⁷), 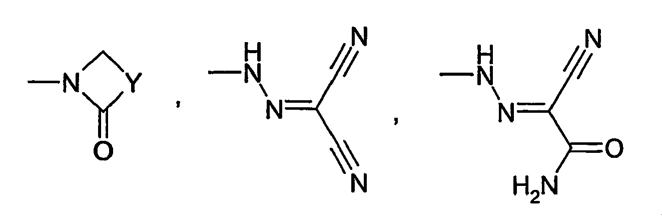 oder 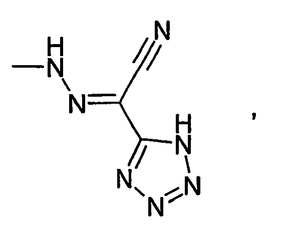
- R⁷: H, COOH, COOA, CONH₂, CONHA, CONAA', NH₂, NHA, NAA', NCOA, NCOOA, OH oder OA,
- R⁸: A, Cycloalkyl mit 3-7 C-Atomen, Alkylencycloalkyl mit 4-8 C- Atomen oder Alkenyl mit 2-8 C-Atomen,
- R⁹: Alkyl mit 1-10 Atomen, Cycloalkyl mit 3-7 C-Atomen,
Alkylencycloalkyl mit 4-8 C-Atomen oder Alkenyl mit 2-8 C- Atomen,
worin eine, zwei oder drei CH₂-Gruppen durch O, S, SO, SO₂, NH, NMe, NEt und/oder durch -CH=CH-Gruppen ersetzt sein können,
1-7 H-Atome durch F und/oder Cl ersetzt sein können und/oder 1 H-Atom durch R⁷ ersetzt sein kann,
- Y: Alkylen mit 1-10 C-Atomen oder Alkenylen mit 2-8 C-Atomen, worin eine, zwei oder drei CH₂-Gruppen durch O, S, SO, SO₂, NH oder NR⁹ und/oder
1-7 H-Atome durch F und/oder Cl ersetzt sein können,
- A, A': jeweils unabhängig voneinander Alkyl mit 1-10 C-Atomen oder Alkenyl mit 2-8 C-Atomen,
worin eine, zwei oder drei CH₂-Gruppen durch O, S, SO, SO₂, NH oder NR⁹ und/oder
1-7 H-Atome durch F und/oder Cl ersetzt sein können,
oder
Aryl oder Het,
- A und A': zusammen auch eine Alkylenkette mit 2-7 C-Atomen, worin eine, zwei oder drei CH₂-Gruppen durch O, S, SO, SO₂, NH, NR⁹, NCOR⁹ oder NCOOR⁹ ersetzt sein können,
- A", A"': jeweils unabhängig voneinander
fehlt, Alkylen mit 1-10 C-Atomen, Alkenylen mit 2-8 C-Atomen oder Cycloalkylen mit 3-7 C-Atomen,
worin eine, zwei oder drei CH₂-Gruppen durch O, S, SO, SO₂, NH oder NR⁹ und/oder
1-7 H-Atome durch F und/oder Cl ersetzt sein können,
- A" und A"': zusammen auch eine Alkylenkette mit 2-7 C-Atomen, worin eine, zwei oder drei CH₂-Gruppen durch O, S, SO, SO₂, NH, NR⁹, NCOR⁹ oder NCOOR⁹ ersetzt sein können,
- Aryl: unsubstituiertes oder ein-, zwei- oder dreifach durch Hal, R¹¹, OR¹⁰, N(R¹⁰)₂, NO₂, CN, COOR¹⁰, CON(R¹⁰)₂, NR¹⁰COR¹⁰, NR¹⁰CON(R¹⁰)₂, NR¹⁰SO₂A, COR¹⁰, SO₂N(R¹⁰)₂, S(O)ₘR¹¹ substituiertes Phenyl, Naphthyl, Fluorenyl oder Biphenyl,
- R¹⁰: H oder Alkyl mit 1-6 C-Atomen,
- R¹¹: Alkyl mit 1-6 C-Atomen,
- Het: einen ein- oder zweikernigen gesättigten, ungesättigten oder aromatischen Heterocyclus mit 1 bis 2 N-, O- und/oder S- Atomen, der unsubstituiert oder ein- oder zweifach durch Carbonylsauerstoff, Hal, R¹¹, OR¹⁰, N(R¹⁰)₂, NO₂, CN, COOR¹⁰, CON(R¹⁰)₂, NR10COR¹⁰ NR¹⁰CON(R¹⁰)₂, NR¹⁰SO₂R¹¹, COR¹⁰, SO₂NR¹⁰ und/oder S(O)ₘR¹¹ substituiert sein kann,
- Hal: F, Cl, Br oder I,
- m: 0, 1 oder 2,
bedeuten,
sowie ihre pharmazeutisch verwendbaren Derivate, Solvate, Stereoisomere und E/Z-Isomeren, einschließlich deren Mischungen in allen Verhältnissen.

1-Benzoyltetrahydropyridazine als Progesteron-Rezeptorliganden sind z.B. in J. Med. Chem. 38, 4878 (1995) beschrieben.
Weitere Arylalkanoylpyridazine sind zum Beispiel aus EP 0 922 036, EP 1 124 809 oder WO 01/04099 bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze und Solvate bei guter Verträglichkeit sehr wertvolle pharmakologische Eigenschaften besitzen.

Die Verbindungen der Formel I zeigen eine selektive Phosphodiesterase IV-Hemmung, die mit einer intrazellulären Erhöhung von cAMP verbunden ist (N. Sommer et al., Nature Medicine, 1, 244-248 (1995)). Die PDE IV-Hemmung kann z.B. analog C.W. Davis in Biochim. Biophys. Acta 797, 354-362 (1984) nachgewiesen werden.
Die Affinität der erfindungsgemäßen Verbindungen für Phosphodiesterase IV wird durch Bestimmung ihrer IC₅₀-Werte (der Inhibitorkonzentration, die zur Erreichung einer 50%igen Hemmung der Enzymaktivität erforderlich ist) gemessen.

Die erfindungsgemäßen Verbindungen können zur Behandlung von asthmatischen Erkrankungen eingesetzt werden. Die antiasthmatische Wirkung der PDE IV-Inhibitoren ist z.B. von T.J. Torphy et al. in Thorax, 46, 512-523 (1991) beschrieben und kann z. B. nach der Methode von T. Olsson, Acta allergologica 26, 438-447 (1971), bestimmt werden.

Da cAMP knochenabbauende Zellen hemmt und knochenaufbauende Zellen stimuliert (S. Kasugai et al., M 681 und K. Miyamoto, M 682, in Abstracts of the American Society for Bone and Mineral Research 18th Annual Meeting, 1996), können die erfindungsgemäßen Verbindungen zur Behandlung von Osteoporose eingesetzt werden.

Außerdem zeigen die Verbindungen eine antagonistische Wirkung auf die Produktion von TNF (Tumor Nekrose Faktor) und eignen sich daher zur Behandlung von allergischen und entzündlichen Erkrankungen, Autoimmunerkrankungen wie zum Beispiel rheumatoider Arthritis, multipler Sklerose, Morbus Crohn, Diabetes mellitus oder ulzerativer Kolitis, Transplantatabstoßungsreaktionen, Kachexie und Sepsis.

Die antünflammatorische Wirkung der erfindungsgemäßen Substanzen und ihre Wirksamkeit zur Behandlung von z.B. Autoimmunstörungen wie multipler Sklerose oder rheumatoider Arthritis, kann analog den Methoden von N. Sommer et al., Nature Medicine 1, 244-248 (1995) oder L. Sekut et al., Clin. Exp. Immunol. 100, 126-132 (1995) bestimmt werden.

Die Verbindungen können zur Behandlung von Kachexie eingesetzt werden. Die antikachektische Wirkung kann in TNF-abhängigen Modellen der Kachexie geprüft werden (P. Costelli et al., J. Clin. Invest. 95, 2367ff. (1995); J.M. Argiles et al., Med. Res. Rev. 17, 477ff. (1997)).

PDE IV-Inhibitoren können auch das Wachstum von Tumorzellen hemmen und sind deshalb für die Tumortherapie geeignet (D. Marko et al., Cell Biochem. Biophys. 28, 75ff. (1998)). Die Wirkung von PDE IV-Inhibitoren bei der Tumorbehandlung ist z.B. in der WO 95 35 281, WO 95 17 399 oder WO 96 00 215 beschrieben.

PDE IV-Inhibitoren können die Mortalität in Modellen für Sepsis verhindern und eignen sich daher für die Therapie von Sepsis (W. Fischer et al., Biochem. Pharmacol. 45, 2399ff. (1993)).

Sie können weiterhin zur Behandlung von Gedächtnisstörungen, Atherosklerose, atopische Dermatitis und AIDS eingesetzt werden.

Die Wirkung von PDE IV-Inhibitoren bei der Behandlung von Asthma, entzündlichen Erkrankungen, Diabetes mellitus, atopischer Dermatitis, Psoriasis, AIDS, Kachexie, Tumorwachstum oder Tumormetastasen ist z.B. in der EP 77 92 91 beschrieben.

Die Verbindungen der Formel I können als Arzneimittelwirkstoffe in der Human- und Veterinärmedizin eingesetzt werden. Ferner können sie als Zwischenprodukte zur Herstellung weiterer Arzneimittelwirkstoffe eingesetzt werden.

Die Erfindung betrifft weiterhin die Verwendung von Typ-4-Phosphodiesteraseinhibitoren (PDE IV-Inhibitoren) der Formel I zur Behandlung von Erkrankungen sowie Kombinationen von Verbindungen der Formel I mit anderen Arzneistoffen.

Es wird auf WO 01/57025 verwiesen, aus der spezielle Pyrimidinderivate als PDE IV-Inhibitoren, ihre Verwendung zur Behandlung von Erkrankungen sowie Kombinationen mit anderen Arzneistoffen bekannt sind.

Gegenstand der Erfindung ist dementsprechend insbesondere die Verwendung von Verbindungen der Formel I und ihren physiologisch unbedenklichen Salzen und Solvaten zur Herstellung eines Arzneimittels zur Behandlung eines Patienten, der an einer Erkrankung oder einem Leiden leidet, die vom PDE IV- Isozym in seiner Rolle bei der Regulierung der Aktivierung und Degranulation humaner Eosinophiler vermittelt wird.

Aus WO 01/57025 sind verschiedene In-vitro-Assays und Tiermodellversuche bekannt, die genug Daten bereitstellen können, um den therapeutischen Nutzen von Verbindungen der Formel I zu definieren und nachzuweisen.

Verbindungen der Formel I hemmen das PDE IV-Isozym und eignen sich daher aufgrund der wesentlichen Rolle, die die PDE-IV-Isozymfamilie in der Physiologie aller Säugetiere spielt, für verschiedenste therapeutische Anwendungen. Bei der enzymatischen Rolle der PDE IV-Isozyme handelt es sich um die intrazelluläre Hydrolyse von Adenosin-3',5'-Monophosphat (cAMP) in Leukozyten vor Erreichen des Entzündungszustands. cAMP wiederum ist für die Vermittlung der Wirkung zahlreicher Hormone im Körper verantwortlich und die Hemmung von PDE IV spielt daher eine wesentliche Rolle bei verschiedenen physiologischen Vorgängen. Es liegt umfassende Fachliteratur vor, in der die Wirkungen von PDE-Inhibitoren auf verschiedene Entzündungsreaktionen der Zelle beschrieben werden, zu denen außer der Erhöhung von cAMP auch die Hemmung der Superoxidproduktion, Degranulation, Chemotaxis und Freisetzung des Tumor Nekrose Faktors (TNF) in Eosinophilen, Neutrophilen und Monozyten zählt.

Die Erfindung betrifft daher die Verbindungen der Formel I und ein Verfahren zur Herstellung von Verbindungen der Formel I sowie deren Salzen und Solvaten, dadurch gekennzeichnet, daß man
a) eine Verbindung der Formel II 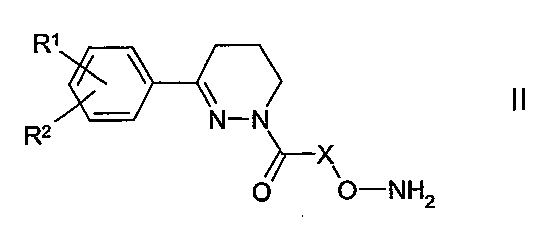 worin
   X, R¹ und R² die in Anspruch 1 angegebenen Bedeutungen haben,
   mit einer Verbindung der Formel III 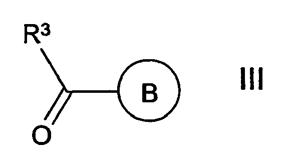 worin
   R³ und B die in Anspruch 1 angegebenen Bedeutungen haben,
   mit der Maßgabe, daß falls eine weitere OH- und/oder Aminogruppe vorliegt, diese geschützt ist,
   umsetzt,
   und anschließend gegebenenfalls eine Schutzgruppe abspaltet,
   oder
b) eine Verbindung der Formel IV 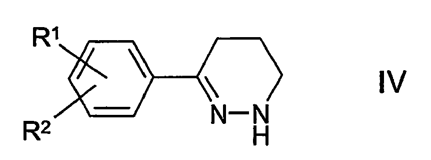 worin
   R¹ und R² die in Anspruch 1 angegebenen Bedeutungen haben, mit einer Verbindung der Formel V 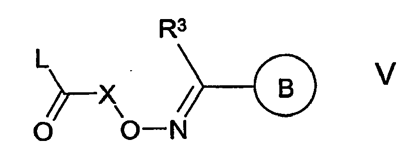 worin
   L Cl, Br, I oder eine freie oder reaktionsfähig funktionell abgewandelte OH-Gruppe bedeutet,
   und R³, X und B die in Anspruch 1 angegebenen Bedeutungen haben, mit der Maßgabe, daß falls eine weitere OH- und/oder Aminogruppe vorliegt, diese geschützt ist,
   umsetzt,
   und anschließend gegebenenfalls eine Schutzgruppe abspaltet,
   oder
c) eine Verbindung der Formel VI 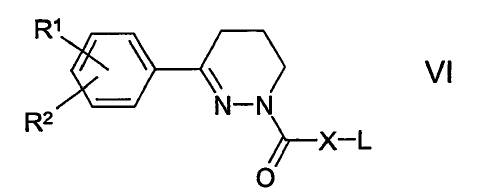 worin
   X, R¹ und R² die in Anspruch 1 angegebenen Bedeutungen haben und L Cl, Br, I oder eine freie oder reaktionsfähig funktionell abgewandelte OH-Gruppe bedeutet,
   mit einer Verbindung der Formel VII 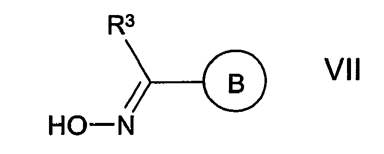 worin R³ und B die in Anspruch 1 angegebenen Bedeutungen haben, mit der Maßgabe, daß falls eine weitere OH- und/oder Aminogruppe vorliegt, diese geschützt ist,
   umsetzt,
   und anschließend gegebenenfalls eine Schutzgruppe abspaltet,
   oder
d) in einer Verbindung der Formel I einen oder mehrere Reste R¹, R² R³ und/oder B in einen oder mehrere andere Reste R¹, R², R³ und/oder B umwandelt, indem man
   i) einen Ether oder Ester spaltet,
   ii) eine OH-Funktion alkyliert oder acyliert,
   iii) eine Aminogruppe reduktiv alkyliert,
   iv) eine Aminogruppe mit Malodinitril umsetzt,
   v) eine Cyangruppe in eine Tetrazolgruppe überführt,
und/oder daß man eine basische Verbindung der Formel I durch Behandeln mit einer Säure in eines ihrer Salze überführt.

Die Erfindung betrifft außerdem die optisch aktiven Formen (Stereoisomere), die E2-Isomeren, die Enantiomere, die Racemate, die Diastereomere und die Hydrate und Solvate dieser Verbindungen. Unter Solvaten der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Monohydrate, Dihydrate oder Alkoholate.

Unter pharmazeutisch verwendbaren Derivaten werden z.B. die Salze der erfindungsgemäßen Verbindungen sowie sogenannte Prodrug-Verbindungen verstanden.
Unter Prodrug-Derivaten werden z.B. Verbindungen der Formel I verstanden, die z.B. mit Alkyl- oder Acylgruppen, Zuckern oder Oligopeptiden modifiziert wurden und die im Organismus rasch gespalten werden und so die erfindungsgemäßen Wirkstoffe abgeben.
Dazu zählen auch biologisch abbaubare Polymerderivate der erfindungsgemäßen Verbindungen wie z.B. in Int. J. Pharm. 115, 61-67 (1995) beschrieben.

### Es bedeuten nachstehend:

- Ac: Acetyl
- BOC: tert.-Butoxycarbonyl
- CBZ oder Z: Benzyloxycarbonyl
- DCCI: Dicyclohexylcarbodiimid
- DCM: Dichlormethan
- DMF: Dimethylformamid
- EDCI: N-Ethyl-N,N'-(dimethylaminopropyl)-carbodiimid
- EE: Essigester
- Et: Ethyl
- Fmoc: 9-Fluorenylmethoxycarbonyl
- HOBt: 1-Hydroxybenzotriazol
- Me: Methyl
- MBHA: 4-Methyl-benzhydrylamin
- Mtr: 4-Methoxy-2,3,6-trimethylphenyl-sulfonyl
- HONSu: N-Hydroxysuccinimid
- OBut: tert.-Butylester
- Oct: Octanoyl
- OMe: Methylester
- OEt: Ethylester
- POA: Phenoxyacetyl
- TFA: Trifluoressigsäure
- Trt: Trityl (Triphenylmethyl).

Die Bedeutungen aller Reste, die mehr als einmal vorkommen, sind jeweils voneinander unabhängig.

Vor- und nachstehend haben die Reste R¹, R², R³, X, B und L die in den Formeln I, II, III, IV, V, VI und VII angegebenen Bedeutungen, sofern nicht ausdrücklich etwas anderes angegeben ist.

Alkyl mit 1-10 C-Atomen bedeutet Alkyl mit 1, 2, 3, 4, 5, 6, 7, 8, 9 oder 10 C-Atomen, ist verzweigt oder unverzweigt, und bedeutet vorzugsweise Alkyl mit 1, 2, 3, 4, 5 oder 6 C-Atomen und bedeutet z.B. Methyl, Ethyl, Trifluormethyl, Pentafluorethyl oder Propyl, weiter bevorzugt Isopropyl, Butyl, Isobutyl, sec-Butyl oder tert.-Butyl, jedoch auch n-Pentyl, Neopentyl, Isopentyl oder n-Hexyl. Besonders bevorzugt ist Methyl, Ethyl, Trifluormethyl, Propyl, Isopropyl, Butyl, n-Pentyl, n-Hexyl oder n-Decyl.

Cycloalkyl hat vorzugsweise 3-7 C-Atome und bedeutet vorzugsweise Cyclopropyl oder Cyclobutyl, weiter bevorzugt Cyclopentyl oder Cyclohexyl, ferner auch Cycloheptyl; besonders bevorzugt ist Cyclopentyl.

Alkenyl bedeutet vorzugsweise Vinyl, Allyl, 2- oder 3-Butenyl, Isobutenyl, sec-Butenyl; ferner ist 4-Pentenyl, Isopentenyl oder 5-Hexenyl bevorzugt.

Alkylen ist vorzugsweise unverzweigt und bedeutet bevorzugt Methylen oder Ethylen, ferner bevorzugt Propylen oder Butylen.

Alkylencycloalkyl bedeutet z.B. Cyclohexylmethyl oder Cyclopentylethyl.

Alkyl mit 1-6 C-Atomen bedeutet Alkyl mit 1, 2, 3, 4, 5 oder 6 C-Atomen, ist verzweigt oder unverzweigt, und bedeutet z.B. Methyl, Ethyl, Trifluormethyl, Pentafluorethyl oder Propyl, weiter bevorzugt Isopropyl, Butyl, Isobutyl, sec-Butyl oder tert.-Butyl, jedoch auch n-Pentyl, Neopentyl, Isopentyl oder n-Hexyl. Besonders bevorzugt ist Methyl, Ethyl, Trifluormethyl, Propyl, Isopropyl, Butyl, n-Pentyl oder n-Hexyl.

Hal bedeutet vorzugsweise F, Cl oder Br, ferner auch I.

Die Reste R¹ und R² können gleich oder verschieden sein und befinden sich vorzugsweise in der 3- oder 4-Stellung des Phenylrings. Sie bedeuten beispielsweise unabhängig voneinander H, Hydroxyl, -S-CH₃, -SO-CH₃, -SO₂CH₃, F, Cl, Br oder I oder zusammen Methylendioxy. Bevorzugt bedeuten sie jedoch jeweils Methyl, Ethyl, Propyl, Methoxy, Ethoxy, Propoxy, Isopropoxy, Benzyloxy, oder aber Fluor-, Difluor oder Trifluormethoxy, oder 1-Fluor-, 2-Fluor-, 1,2-Difluor-, 2,2-Difluor-, 1,2,2-Trifluor oder 2,2,2-Trifluorethoxy.

In einer weiteren Ausführungsform bedeuten R¹, R² jeweils unabhängig voneinander Alkoxy mit 1, 2, 3, 4, 5 oder 6 C-Atomen.

Alkoxy bedeutet z.B. Methoxy, Ethoxy, Propoxy, Isopropoxy, Butoxy, Pentoxy oder Hexoxy.

R¹ bedeutet besonders bevorzugt Ethoxy, Benzyloxy, F, Propoxy oder Isopropoxy, weiterhin Difluormethoxy oder Cycloalkoxy, z.B. Cyclopentoxy. R¹ bedeutet ganz besonders bevorzugt 4-Methoxy.

R² bedeutet besonders bevorzugt Methoxy, Ethoxy, Propoxy, F oder Ethyl, weiterhin Difluormethoxy oder Cycloalkoxy, z.B. Cyclopentoxy.
R² bedeutet ganz besonders bevorzugt 3-Ethoxy oder 3-Propoxy.

R³ bedeutet vorzugsweise H, A"R⁷, COA"R⁷, CON(A"R⁷)(A"'R⁷) oder CO-NR¹⁰-Het.
R³ bedeutet ganz besonders bevorzugt z.B. H, COOH, CO-CH₂-NAA', CO-CH₂-CH₂-NAA', CO-CH₂-NHA', CO-CH₂-CH₂-NHA', 1-Methyl-piperazin-4-yl-carbonyl, 1-Methyl-piperidin-4-yl-N-methylamino-carbonyl.

X bedeutet vorzugsweise Methylen, Ethylen, Propylen oder Butylen, ganz besonders bevorzugt Methylen.

B bedeutet vorzugsweise Phenyl, Pyridyl, Pyridyl-N-oxid, Thienyl, Furyl, Pyrrolyl, Pyridazinyl, Pyrimidinyl, Pyrazinyl, Triazinyl, Isoxazolinyl, Oxazolinyl, Thiazolinyl, Pyrazolinyl, Imidazolinyl, Naphthyl, Chinolinyl, Isochinolinyl, Cinnolinyl, Phthalazinyl, Chinazolinyl oder Chinoxalinyl, das unsubstituiert ist oder ein-, zwei- oder dreifach durch R⁴, R⁵ und/oder R⁶ substituiert sein kann.

B bedeutet in einer weiteren bevorzugten Ausführungsform Phenyl, Pyridyl, Pyridyl-N-oxid, Thienyl, Furyl, Pyrrolyl, Pyridazinyl, Pyrimidinyl, Pyrazinyl, Triazinyl, Isoxazolinyl, Oxazolinyl, Thiazolinyl, Pyrazolinyl, Imidazolinyl, Naphthyl, Chinolinyl, Isochinolinyl, Cinnolinyl, Phthalazinyl, Chinazolinyl oder Chinoxalinyl, das unsubstituiert ist oder ein-, zwei- oder dreifach durch OH, OA, NO₂, NH₂, NAA', 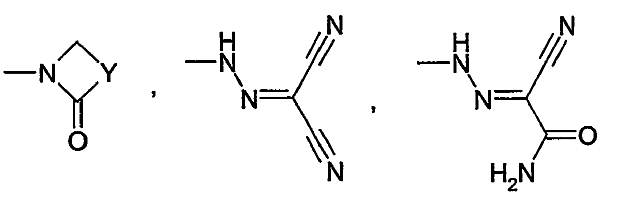 oder 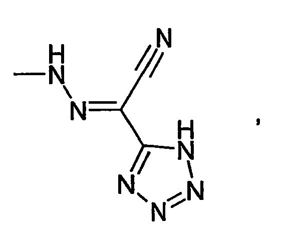 substituiert sein kann.

B bedeutet in einer weiteren bevorzugten Ausführungsform unsubstituiertes oder einfach durch OR¹⁰, NO₂ oder 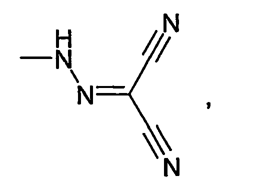 substituiertes Phenyl oder unsubstituiertes Pyridyl oder Pyridyl-N-oxid.

R⁴ bedeutet vorzugsweise H, OR¹⁰, NO₂ oder 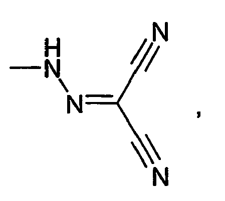 weiterhin bevorzugt NH₂ oder NHCOOA"R⁷.

R⁵ und R⁶ bedeuten vorzugsweise H.

R⁷ bedeutet vorzugsweise H, COOH, NHA oder NAA'.

R⁸ bedeutet vorzugsweise R¹¹, Cycloalkyl mit 3-7 C-Atomen oder Alkylencycloalkyl mit 4-8 C-Atomen.

R⁹ bedeutet vorzugsweise Alkyl mit 1-10 C-Atomen, ganz besonders bevorzugt Alkyl mit 1-6 C-Atomen.

Y bedeutet vorzugsweise Methylen, Ethylen, Propylen oder Butylen.

A, A' bedeuten vorzugsweise jeweils unabhängig voneinander Alkyl mit 1-10 C-Atomen, worin 1-7 H-Atome durch F und/oder Cl ersetzt sein können, oder sie bedeuten jeweils unabhängig voneinander Aryl oder Het.

A", A'" bedeuten vorzugsweise jeweils unabhängig voneinander:
fehlt oder Alkylen mit 1-10 C-Atomen, worin eine CH₂-Gruppe durch NH oder NR⁹ ersetzt sein kann.
A", A'" bedeuten vorzugsweise zusammen auch eine Alkylenkette mit 2-7 C-Atomen, worin eine CH₂-Gruppe durch NH oder NR⁹ ersetzt sein kann.

Aryl bedeutet z.B. unsubstituiertes Phenyl, Naphthyl, Fluorenyl oder Biphenyl, weiterhin vorzugsweise z.B. durch Methyl, Ethyl, Propyl, Butyl, Fluor, Chlor, Hydroxy, Methoxy, Ethoxy, Propoxy, Butoxy, Pentyloxy, Hexyloxy, Nitro, Cyan, Formyl, Acetyl, Propionyl, Trifluormethyl, Amino, Methylamino, Ethylamino, Dimethylamino, Diethylamino, Sulfonamido, Methylsulfonamido, Ethylsulfonamido, Propylsulfonamido, Butylsulfonamido, Dimethylsulfonamido, Carboxy, Methoxycarbonyl, Ethoxycarbonyl oder Aminocarbonyl mono-, di- oder trisubstituiertes Phenyl, Naphthyl, Fluorenyl oder Biphenyl.

Het bedeutet z.B. 2- oder 3-Furyl, 2- oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder-5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 1-,2-,3-,4-,5-,6- oder 7-Indolyl, 4- oder 5-Isoindolyl, 1-,2-,4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7- Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]oxazinyl, weiter bevorzugt 1,3-Benzodioxol-5-yl, 1,4-Benzodioxan-6-yl, 2,1,3-Benzothiadiazol-4- oder - 5-yl oder 2,1,3-Benzoxadiazol-5-yl.
Die heterocyclischen Reste können auch teilweise oder vollständig hydriert sein.
Het kann also z. B. auch bedeuten 2,3-Dihydro-2-, -3-, -4- oder -5-furyl, 2,5-Dihydro-2-, -3-, -4- oder 5-furyl, Tetrahydro-2- oder -3-furyl, 1,3-Dioxolan-4-yl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3- oder-4-pyrazolyl, 1,4-Dihydro-1-, -2-, -3- oder -4-pyridyl, 1,2,3,4-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3- oder - 4-pyranyl, 1,4-Dioxanyl, 1,3-Dioxan-2-, -4-oder-5-yl, Hexahydro-1-, -3- oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder -8-chinolyl, 1,2,3,4-Tetrahydro-1-,-2-,-3-, -4-, -5-, -6-, -7- oder -8-isochinolyl, 2-, 3-, 5-, 6-, 7- oder 8- 3,4-Dihydro-2H-benzo[1,4]oxazinyl, weiter bevorzugt 2,3-Methylendioxyphenyl, 3,4-Methylendioxyphenyl, 2,3-Ethylendioxyphenyl, 3,4-Ethylendioxyphenyl, 3,4-(Difluormethylendioxy)phenyl, 2,3-Dihydrobenzofuran-5- oder 6-yl, 2,3-(2-Oxo-methylendioxy)-phenyl oder auch 3,4-Dihydro-2H-1,5-benzodioxepin-6- oder -7-yl, ferner bevorzugt 2,3-Dihydrobenzofuranyl oder 2,3-Dihydro-2-oxo-furanyl.

In einer weiteren Ausführungsform bedeutet Het besonders bevorzugt unsubstituiertes Pyridyl, Pyridyl-N-oxid, Thienyl, Furyl, Pyrrolyl, Pyridazinyl, Pyrimidinyl, Pyrazinyl, Triazinyl, Isoxazolinyl, Oxazolinyl, Thiazolinyl, Pyrazolinyl, Imidazolinyl, Naphthyl, Chinolinyl, Isochinolinyl, Cinnolinyl, Phthalazinyl, Chinazolinyl oder Chinoxalinyl, ganz besonders bevorzugt ist Pyridyl.

In einer weiteren bevorzugten Ausführungsform bedeutet Het einen einkernigen gesättigten oder ungesättigten Heterocyclus mit 1 bis 2 N- und/oder O- Atomen, der ein- oder zweifach durch Carbonylsauerstoff, OH oder OA substituiert sein kann.
Het bedeutet darin besonders bevorzugt z.B. 2-Oxo-piperidin-1-yl, 2-Oxo-pyrrolidin-1-yl, 2-Oxo-1H-pyridin-1-yl, 3-Oxo-morpholin-4-yl, 4-Oxo-1H-pyridin-1-yl, 2,6-Dioxo-piperidin1-yl, 2-Oxo-piperazin-1-yl, 2,6-Dioxo-piperazin-1-yl, 2,5-Dioxo-pyrrolidin-1-yl, 2-Oxo-1,3-oxazolidin-3-yl, 3-Oxo-2H-pyridazin-2-yl, 2-Caprolactam-1-yl (= 2-Oxo-azepan-1-yl), 2-Hydroxy-6-oxo-piperazin-1-yl, 2-Methoxy-6-oxo-piperazin-1-yl, 2-Aza-bicyclo[2.2.2]-octan-3-on-2-yl, ganz besonders bevorzugt ist 2-Oxo-piperidin-1-yl.

In einer weiteren Ausführungsform bedeutet Het ganz besonders bevorzugt einen einkernigen gesättigten Heterocyclus mit 1 bis 2 N-Atomen, der ein- oder zweifach durch Alkyl mit 1-6 C-Atomen substituiert sein kann.

Dementsprechend betrifft die Verbindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ia bis Ik ausgedrückt werden, die der Formel I entsprechen und worin die nicht näher bezeichneten Reste die bei der Formel I angegebene Bedeutung haben, worin jedoch

| | | |
|---|---|---|
| in Ia | R¹, R² | jeweils unabhängig voneinander Alkoxy mit 1, 2, 3, 4, 5 oder 6 C-Atomen bedeuten; |
| | | |
| in Ib | R¹, R² | jeweils unabhängig voneinander H, Methoxy, Ethoxy, Benzyloxy, Propoxy, Isopropoxy, Difluormethoxy, F, Cl, Cyclopentyloxy, Cyclohexyloxy oder Cycloheptyloxy bedeuten; |
| | | |
| in Ic | R¹, R² | jeweils unabhängig voneinander Methoxy, Ethoxy, Propoxy, Isopropoxy, Cyclopentyloxy oder F, bedeuten; |
| | | |
| in Id | R¹ R² | 4-Methoxy, 3-Ethoxy oder 3-Propoxy bedeuten; |
| | | |
| in Ie | R³ | H, A"R⁷, COA"R⁷, CON(A"R⁷)(A"'R⁷) oder CO-NR¹⁰-Het bedeutet; |
| | | |
| in If | X | Methylen, Ethylen, Propylen oder Butylen bedeutet; |
| | | |
| in Ig. | B | Phenyl, Pyridyl, Pyridyl-N-oxid, Thienyl, Furyl, Pyrrolyl, Pyridazinyl, Pyrimidinyl, Pyrazinyl, Triazinyl, Isoxazolinyl, Oxazolinyl, Thiazolinyl, Pyrazolinyl, Imidazolinyl, Naphthyl, Chinolinyl, Isochinolinyl, Cinnolinyl, Phthalazinyl, Chinazolinyl oder Chinoxalinyl, das unsubstituiert ist oder ein-, zwei- oder dreifach durch R⁴, R⁵ und/oder R⁶ substituiert sein kann, bedeutet; |
| | | |
| in Ih | B | Phenyl, Pyridyl, Pyridyl-N-oxid, Thienyl, Furyl, Pyrrolyl, Pyridazinyl, Pyrimidinyl, Pyrazinyl, Triazinyl, Isoxazolinyl, Oxazolinyl, Thiazolinyl, Pyrazolinyl, Imidazolinyl, Naphthyl, Chinolinyl, Isochinolinyl, Cinnolinyl, Phthalazinyl, Chinazolinyl oder Chinoxalinyl, das unsubstituiert ist oder ein-, zwei- oder dreifach durch OH, OA, NO₂, NH₂, NAA', |
| | | 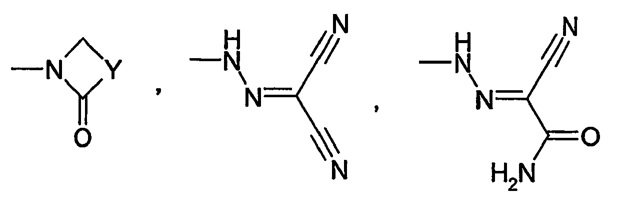 oder 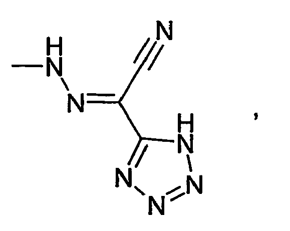 |
| | | substituiert sein kann, bedeutet; |
| | | |
| in Ii | B | unsubstituiertes oder einfach durch OR¹⁰, NO₂ oder |
| | | 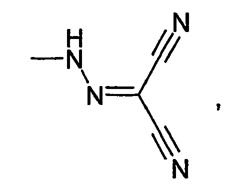 |
| | | substituiertes Phenyl oder unsubstituiertes Pyridyl oder Pyridyl-N-oxid, bedeutet; |
| | | |
| in Ij | R¹, R² | jeweils unabhängig voneinander Alkoxy mit 1, 2, 3, 4, 5 oder 6 C-Atomen, |
| | X | Methylen, Ethylen, Propylen oder Butylen, |
| | R³ | H, A"R⁷, COA"R⁷, CON(A"R⁷)(A"'R⁷) oder CO-NR¹⁰-Het, |
| | A", A"' | jeweils unabhängig voneinander fehlt oder Alkylen mit 1-10 C-Atomen, worin eine CH₂-Gruppe durch NH oder NR⁹ ersetzt sein kann, |
| | A", A'" | zusammen auch eine Alkylenkette mit 2-7 C-Atomen, worin eine CH₂-Gruppe durch NH oder NR⁹ ersetzt sein kann, |
| | B | unsubstituiertes oder einfach durch OR¹⁰, NO₂, |
| | | 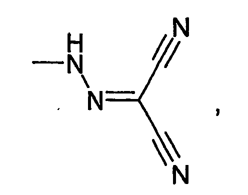 |
| | | NH₂ oder NHCOOA"R⁷ substituiertes Phenyl oder unsubstituiertes Pyridyl oder Pyridyl-N-oxid, |
| | R⁷ | H, COOH, NHA oder NAA', |
| | R⁹ | Alkyl mit 1-6 C-Atomen, |
| | R¹⁰ | H oder Alkyl mit 1-6 C-Atomen, |
| | A, A' | jeweils unabhängig voneinander Alkyl mit 1-10 C-Atomen, worin 1-7 H-Atome durch F und/oder Cl ersetzt sein können, |
| | Het | einen einkernigen gesättigten Heterocyclus mit 1 bis 2 N-Atomen, der ein- oder zweifach durch Alkyl mit 1-6 C-Atomen substituiert sein kann, bedeuten; |
| | | |
| in Ik | R¹, R² | jeweils unabhängig voneinander Alkoxy mit 1, 2, 3, 4, 5 oder 6 C-Atomen, |
| | X | Methylen, Ethylen, Propylen oder Butylen, |
| | R³ | H, A"R⁷, COA"R⁷, CON(A"R⁷)(A"'R⁷) oder CO-NR¹⁰-Het, |
| | A", A"' | jeweils unabhängig voneinander fehlt oder Alkylen mit 1-10 C-Atomen, worin eine CH₂-Gruppe durch NH oder NR⁹ ersetzt sein kann, |
| | A", A"' | zusammen auch eine Alkylenkette mit 2-7 C-Atomen, worin eine CH₂-Gruppe durch NH oder NR⁹ ersetzt sein kann, |
| | B | unsubstituiertes oder einfach durch OR¹⁰, NO₂, |
| | | 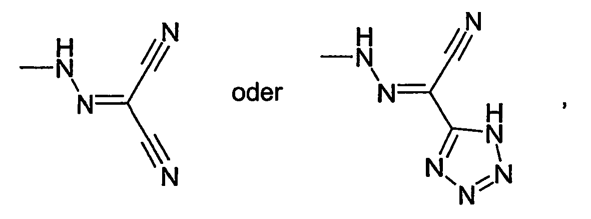 |
| | | NH₂ oder NHCOOA"R⁷ substituiertes Phenyl oder unsubstituiertes Pyridyl oder Pyridyl-N-oxid, |
| | R⁷ | H, COOH, NHA oder NAA', |
| | R⁹ | Alkyl mit 1-6 C-Atomen, |
| | R¹⁰ | H oder Alkyl mit 1-6 C-Atomen, |
| | A, A' | jeweils unabhängig voneinander Alkyl mit 1-10 C-Atomen, worin 1-7 H-Atome durch F und/oder Cl ersetzt sein können, |
| | Het | einen einkernigen gesättigten Heterocyclus mit 1 bis 2 N-Atomen, der ein- oder zweifach durch Alkyl mit 1-6 C-Atomen substituiert sein kann, |

bedeuten;
sowie ihre pharmazeutisch verwendbaren Derivate, Solvate, Stereoisomere und E/Z-Isomeren, einschließlich deren Mischungen in allen Verhältnissen.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart), beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.
In den Verbindungen der Formeln II, III, IV, V, VI und VII haben R¹, R², R³, X und B die angegebenen Bedeutungen, insbesondere die angegebenen bevorzugten Bedeutungen.

Die Ausgangsstoffe der Formel II sind teilweise bekannt. Sofern sie nicht bekannt sind, können sie nach an sich bekannten Methoden hergestellt werden.
Die Ausgangsstoffe der Formel III sind teilweise bekannt. Sofern sie nicht bekannt sind, können sie nach an sich bekannten Methoden hergestellt werden.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.
Andererseits ist es möglich, die Reaktion stufenweise durchzuführen.

Die Verbindungen der Formel I können vorzugsweise erhalten werden, indem man Verbindungen der Formel II mit Verbindungen der Formel III umsetzt.

Verbindungen der Formel I werden vorzugsweise z.B. nach dem folgenden Reaktionsschema hergestellt:

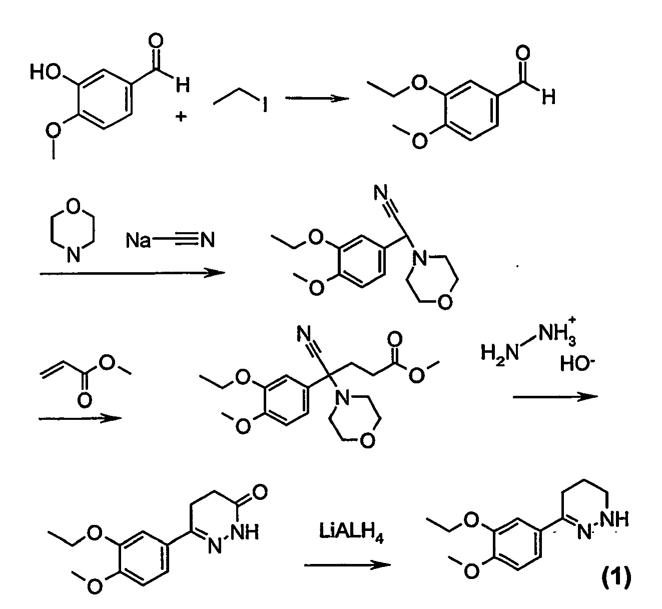

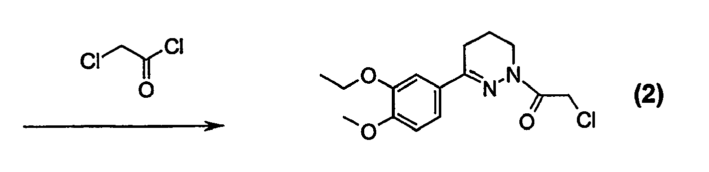

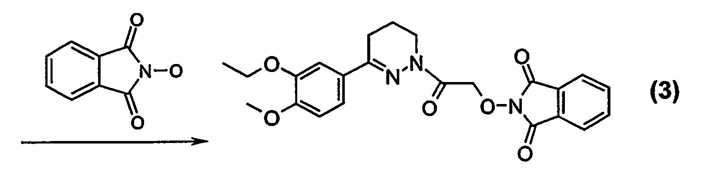

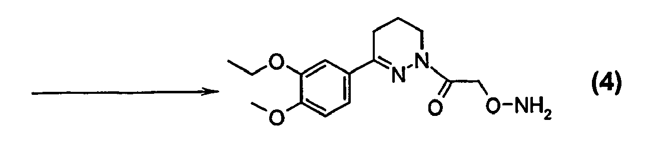

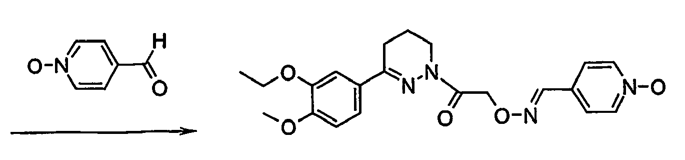

Im einzelnen erfolgt die Umsetzung der Verbindungen der Formel II mit den Verbindungen der Formel III in Gegenwart oder Abwesenheit eines inerten Lösungsmittels bei Temperaturen zwischen etwa -20 und etwa 150°, vorzugsweise zwischen 20 und 100°.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan, Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO);
Schwefelkohlenstoff; Carbonsäuren wie Ameisensäure oder Essigsäure; Nitroverbindungen wie Nitromethan oder Nitrobenzol; Ester wie Ethylacetat, oder Gemische der genannten Lösungsmittel.

Verbindungen der Formel I können weiterhin erhalten werden, indem man Verbindungen der Formel IV mit Verbindungen der Formel V umsetzt. Die Ausgangsstoffe der Formel IV sind teilweise bekannt. Sofern sie nicht bekannt sind, können sie nach an sich bekannten Methoden hergestellt werden.
Die Ausgangsstoffe der Formel V sind teilweise bekannt. Sofern sie nicht bekannt sind, können sie nach an sich bekannten Methoden hergestellt werden.

In den Verbindungen der Formel V bedeutet L vorzugsweise Cl, Br, I oder eine freie oder eine reaktionsfähig abgewandelte OH-Gruppe wie z.B. ein aktivierter Ester, ein Imidazolid oder Alkylsulfonyloxy mit 1-6 C-Atomen (bevorzugt Methylsulfonyloxy oder Trifluormethylsulfonyloxy) oder Arylsulfonyloxy mit 6-10 C-Atomen (bevorzugt Phenyl- oder p-Tolylsulfonyloxy).
Derartige Reste zur Aktivierung der Carboxygruppe in typischen Acylierungsreaktionen sind in der Literatur (z.B. in den Standardwerken wie Houben-Weyl, Methoden der organischen Chemie, Georg-Thieme-Verlag, Stuttgart;) beschrieben.
Aktivierte Ester werden zweckmäßig in situ gebildet, z. B. durch Zusatz von HOBt oder N-Hydroxysuccinimid.

Die Umsetzung erfolgt bei Temperaturen und in Lösungsmitteln wie für die Umsetzung von Verbindungen der Formel II mit solchen der Formel III beschrieben.

Verbindungen der Formel I können weiterhin erhalten werden, indem man Verbindungen der Formel VI mit Verbindungen der Formel VII umsetzt. Die Ausgangsstoffe der Formel VI sind teilweise bekannt. Sofern sie nicht bekannt sind, können sie nach an sich bekannten Methoden hergestellt werden.
Die Ausgangsstoffe der Formel VII sind teilweise bekannt. Sofern sie nicht bekannt sind, können sie nach an sich bekannten Methoden hergestellt werden.

In den Verbindungen der Formel VI bedeutet L vorzugsweise Cl, Br, I oder eine freie oder eine reaktionsfähig abgewandelte OH-Gruppe wie z.B. ein aktivierter Ester, ein Imidazolid oder Alkylsulfonyloxy mit 1-6 C-Atomen (bevorzugt Methylsulfonyloxy oder Trifluormethylsulfonyloxy) oder Arylsulfonyloxy mit 6-10 C-Atomen (bevorzugt Phenyl- oder p-Tolylsulfonyloxy).

Die Umsetzung erfolgt bei Temperaturen und in Lösungsmitteln wie für die Umsetzung von Verbindungen der Formel II mit solchen der Formel III beschrieben.

Verbindungen der Formel I können ferner erhalten werden, indem man Verbindungen der Formel I aus einem ihrer funktionellen Derivate durch Behandeln mit einem solvolysierenden oder hydrogenolysierenden Mittel in Freiheit setzt.

Bevorzugte Ausgangsstoffe für die Solvolyse bzw. Hydrogenolyse sind solche, die sonst der Formel I entsprechen, aber anstelle einer oder mehrerer freier Amino- und/oder Hydroxygruppen entsprechende geschützte Amino- und/oder Hydroxygruppen enthalten, vorzugsweise solche, die anstelle eines H-Atoms, das mit einem N-Atom verbunden ist, eine Aminoschutzgruppe tragen, insbesondere solche, die anstelle einer HN-Gruppe eine R'-N-Gruppe tragen, worin R' eine Aminoschutzgruppe bedeutet, und/oder solche, die anstelle des H-Atoms einer Hydroxygruppe eine Hydroxyschutzgruppe tragen, z.B. solche, die der Formel I entsprechen, jedoch anstelle einer Gruppe -COOH eine Gruppe -COOR" tragen, worin R" eine Hydroxyschutzgruppe bedeutet.
Bevorzugte Ausgangsstoffe sind auch die Oxadiazolderivate, die in die entsprechenden Amidinoverbindungen überführt werden können.

Es können auch mehrere - gleiche oder verschiedene - geschützte Amino- und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an anderen Stellen des Moleküls durchgeführt worden ist. Typisch für solche Gruppen sind insbesondere unsubstituierte oder substituierte Acyl-, Aryl-, Aralkoxymethyl- oder Aralkylgruppen. Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie POA; Alkoxycarbonyl wie Methoxycarbonyl, Ethoxycarbonyl, 2,2,2-Trichlorethoxycarbonyl, BOC (tert.-Butyloxycarbonyl), 2-lodethoxycarbonyl; Aralkyloxycarbonyl wie CBZ ("Carbobenzoxy"), 4-Methoxybenzyloxycarbonyl, FMOC; Arylsulfonyl wie Mtr. Bevorzugte Aminoschutzgruppen sind BOC und Mtr, ferner CBZ, Fmoc, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an anderen Stellen des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. Benzyl, 4-Methoxybenzyl, p-Nitrobenzoyl, p-Toluolsulfonyl, tert.-Butyl und Acetyl, wobei Benzyl und tert.-Butyl besonders bevorzugt sind.

Das In-Freiheit-Setzen der Verbindungen der Formel I aus ihren funktionellen Derivaten gelingt - je nach der benutzten Schutzgruppe - z. B. mit starken Säuren, zweckmäßig mit TFA oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie DMF, halogenierte Kohlenwasserstoffe wie Dichlormethan, ferner auch Alkohole wie Methanol, Ethanol oder Isopropanol, sowie Wasser. Ferner kommen Gemische der vorgenannten Lösungsmittel in Frage. TFA wird vorzugsweise im Überschuß ohne Zusatz eines weiteren Lösungsmittels verwendet, Perchlorsäure in Form eines Gemisches aus Essigsäure und 70 %iger Perchlorsäure im Verhältnis 9:1. Die Reaktionstemperaturen für die Spaltung liegen zweckmäßig zwischen etwa 0 und etwa 50°, vorzugsweise arbeitet man zwischen 15 und 30° (Raumtemperatur).

Die Gruppen BOC, OBut und Mtr können z. B. bevorzugt mit TFA in Dichlormethan oder mit etwa 3 bis 5n HCl in Dioxan bei 15-30° abgespalten werden, die FMOC-Gruppe mit einer etwa 5- bis 50 %igen Lösung von Dimethylamin, Diethylamin oder Piperidin in DMF bei 15-30°.

Hydrogenolytisch entfernbare Schutzgruppen (z. B. CBZ, Benzyl oder die Freisetzung der Amidinogruppe aus ihrem Oxadiazolderivat)) können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z. B. gut an 5 bis 10 %igem Pd/C in Methanol oder mit Ammomiumformiat (anstelle von Wasserstoff) an Pd/C in Methanol/DMF bei 20-30°.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan, Tetrachlorkohlenstoff, Trifluormethylbenzol, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid, N-Methylpyrrolidon (NMP) oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.

Ester können z.B. mit Essigsäure oder mit NaOH oder KOH in Wasser, Wasser-THF oder Wasser-Dioxan bei Temperaturen zwischen 0 und 100° verseift werden.

Ferner kann man freie Amino- und/oder Hydroxygruppen in üblicher Weise mit einem Säurechlorid oder -anhydrid acylieren oder mit einem unsubstituierten oder substituierten Alkylhalogenid alkylieren, oder mit CH₃-C(=NH)-OEt umsetzen, zweckmäßig in einem inerten Lösungsmittel wie Dichlormethan oder THF und /oder in Gegenwart einer Base wie Triethylamin oder Pyridin bei Temperaturen zwischen -60 und +30°.

Es ist ferner möglich, eine Verbindung der Formel I in eine andere Verbindung der Formel I umzuwandeln, indem man einen oder mehrere Rest(e) R¹, R², R³ und/oder B in einen oder mehrere andere Reste R¹, R², R³ und/oder B umwandelt, z.B. indem man
i) einen Ether oder Ester spaltet,
ii) eine OH-Funktion alkyliert oder acyliert,
iii) ein Amin reduktiv alkyliert, wobei mit Aldehyden und komplexen Hydriden umgesetzt wird,
iv) eine Amingruppe mit Malodinitril umsetzt,
v) eine Nitrogruppe (beispielsweise durch Hydrierung an Raney-Nickel oder Pd-Kohle in einem inerten Lösungsmittel wie Methanol oder Ethanol) zu einer Aminogruppe reduziert,
vi) eine Cyangruppe in eine Tetrazolgruppe umwandelt,
vii) eine Carboxygruppe durch Umsetzung mit Alkoholen verestert und/oder
viii) eine Nitrogruppe unter hydrogenolytischen Bedingungen alkyliert, wobei ein alkyliertes Amin erhalten wird.

### Pharmazeutische Salze und andere Formen

Die genannten erfindungsgemäßen Verbindungen lassen sich in ihrer endgültigen Nichtsalzform verwenden. Andererseits umfaßt die vorliegende Erfindung auch die Verwendung dieser Verbindungen in Form ihrer pharmazeutisch unbedenklichen Salze, die von verschiedenen organischen und anorganischen Säuren und Basen nach fachbekannten Vorgehensweisen abgeleitet werden können. Pharmazeutisch unbedenkliche Salzformen der Verbindungen der Formel I werden größtenteils konventionell hergestellt. Sofern die Verbindung der Formel I eine Carbonsäuregruppe enthält, läßt sich eines ihrer geeigneten Salze dadurch bilden, daß man die Verbindung mit einer geeigneten Base zum entsprechenden Basenadditionssalz umsetzt. Solche Basen sind zum Beispiel Alkalimetallhydroxide, darunter Kaliumhydroxid, Natriumhydroxid und Lithiumhydroxid; Erdalkalimetallhydroxide wie Bariumhydroxid und Calciumhydroxid; Alkalimetallalkoholate, z.B. Kaliumethanolat und Natriumpropanolat; sowie verschiedene organische Basen wie Piperidin, Diethanolamin und N-Methylglutamin. Die Aluminiumsalze der Verbindungen der Formel I zählen ebenfalls dazu. Bei bestimmten Verbindungen der Formel I lassen sich Säureadditionssalze dadurch bilden, daß man diese Verbindungen mit pharmazeutisch unbedenklichen organischen und anorganischen Säuren, z.B. Halogenwasserstoffen wie Chlorwasserstoff, Bromwasserstoff oder Jodwasserstoff, anderen Mineralsäuren und ihren entsprechenden Salzen wie Sulfat, Nitrat oder Phosphat und dergleichen sowie Alkyl- und Monoarylsulfonaten wie Ethansulfonat, Toluolsulfonat und Benzolsulfonat, sowie anderen organischen Säuren und ihren entsprechenden Salzen wie Acetat, Tartrat, Maleat, Succinat, Citrat, Benzoat, Salicylat, Ascorbat und dergleichen behandelt. Dementsprechend zählen zu pharmazeutisch unbedenklichen Säureadditionssalzen der Verbindungen der Formel I die folgenden: Acetat, Adipat, Alginat, Arginat, Aspartat, Benzoat, Benzolsulfonat (Besylat), Bisulfat, Bisulfit, Bromid, Butyrat, Kampferat, Kampfersulfonat, Caprylat, Chlorid, Chlorbenzoat, Citrat, Cyclopentanpropionat, Digluconat, Dihydrogenphosphat, Dinitrobenzoat, Dodecylsulfat, Ethansulfonat, Fumarat, Galacterat (aus Schleimsäure), Galacturonat, Glucoheptanoat, Gluconat, Glutamat, Glycerophosphat, Hemisuccinat, Hemisulfat, Heptanoat, Hexanoat, Hippurat, Hydrochlorid, Hydrobromid, Hydroiodid, 2-Hydroxyethansulfonat, Iodid, Isethionat, Isobutyrat, Lactat, Lactobionat, Malat, Maleat, Malonat, Mandelat, Metaphosphat, Methansulfonat, Methylbenzoat, Monohydrogenphosphat, 2-Naphthalinsulfonat, Nicotinat, Nitrat, Oxalat, Oleat, Pamoat, Pectinat, Persulfat, Phenylacetat, 3-Phenylpropionat, Phosphat, Phosphonat, Phthalat, was jedoch keine Einschränkung darstellt.

Weiterhin zählen zu den Basensalzen der erfindungsgemäßen Verbindungen Aluminium-, Ammonium-, Calcium-, Kupfer-, Eisen(III)-, Eisen(II)-, Lithium-, Magnesium-, Mangan(III)-, Mangan(II), Kalium-, Natrium- und Zinksalze, was jedoch keine Einschränkung darstellen soll. Bevorzugt unter den oben genannten Salzen sind Ammonium; die Alkalimetallsalze Natrium und Kalium,sowie die Erdalkalimetalsalze Calcium und Magnesium. Zu Salzen der Verbindungen der Formel I, die sich von pharmazeutisch unbedenklichen organischen nicht-toxischen Basen ableiten, zählen Salze primärer, sekundärer und tertiärer Amine, substituierter Amine, darunter auch natürlich vorkommender substituierter Amine, cyclischer Amine sowie basischer Ionenaustauscherharze, z.B. Arginin, Betain, Koffein, Chlorprocain, Cholin, N,N'-Dibenzylethylendiamin (Benzathin), Dicyclohexylamin, Diethanolamin, Diethylamin, 2-Diethylaminoethanol, 2-Dimethylaminoethanol, Ethanolamin, Ethylendiamin, N-Ethylmorpholin, N-Ethylpiperidin, Glucamin, Glucosamin, Histidin, Hydrabamin, Iso-propylamin, Lidocain, Lysin, Meglumin, N-Methyl-D-glucamin, Morpholin, Piperazin, Piperidin, Polyaminharze, Procain, Purine, Theobromin, Triethanolamin, Triethylamin, Trimethylamin, Tripropylamin sowie Tris-(hydroxymethyl)-methylamin (Tromethamin), was jedoch keine Einschränkung darstellen soll.

Verbindungen der vorliegenden Erfindung, die basische stickstoffhaltige Gruppen enthalten, lassen sich mit Mitteln wie (C₁-C₄) Alkylhalogeniden, z.B. Methyl-, Ethyl-, Isopropyl- und tert.-Butylchlorid, -bromid und -iodid; Di(C₁-C₄)Alkylsulfaten, z.B. Dimethyl-, Diethyl- und Diamylsulfat; (C₁₀-C₁₈)Alkylhalogeniden, z.B. Decyl-, Dodecyl-, Lauryl-, Myristyl- und Stearylchlorid, -bromid und -iodid; sowie Aryl-(C₁-C₄)Alkylhalogeniden, z.B. Benzylchlorid und Phenethylbromid, quartemisieren. Mit solchen Salzen können sowohl wasser- als auch öllösliche erfindungsgemäße Verbindungen hergestellt werden.

Zu den oben genannten pharmazeutischen Salzen, die bevorzugt sind, zählen Acetat, Besylat, Citrat, Fumarat, Gluconat, Hemisuccinat, Hippurat, Hydrochlorid, Hydrobromid, Isethionat, Mandelat, Meglumin, Nitrat, Oleat, Phosphonat, Pivalat, Natriumphosphat, Stearat, Sulfat, Sulfosalicylat, Tartrat, Thiomalat, Tosylat und Tromethamin, was jedoch keine Einschränkung darstellen soll.

Die Säureadditionssalze basischer Verbindungen der Formel I werden dadurch hergestellt, daß man die freie Basenform mit einer ausreichenden Menge der gewünschten Säure in Kontakt bringt, wodurch man auf übliche Weise das Salz darstellt. Die freie Base läßt sich durch In-Kontakt-Bringen der Salzform mit einer Base und Isolieren der freien Base auf übliche Weise regenerieren. Die freien Basenformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Basenformen.

Wie erwähnt werden die pharmazeutisch unbedenklichen Basenadditionssalze der Verbindungen der Formel I mit Metallen oder Aminen wie Alkalimetallen und Erdalkalimetallen oder organischen Aminen gebildet. Bevorzugte Metalle sind Natrium, Kalium, Magnesium und Calcium. Bevorzugte organische Amine sind N,N'-Dibenzylethylendiamin, Chlorprocain, Cholin, Diethanolamin, Ethylendiamin, N-Methyl-D-glucamin und Procain.

Die Basenadditionssalze von erfindungsgemäßen sauren Verbindungen werden dadurch hergestellt, daß man die freie Säureform mit einer ausreichenden Menge der gewünschten Base in Kontakt bringt, wodurch man das Salz auf übliche Weise darstellt. Die freie Säure läßt sich durch In-Kontakt-Bringen der Salzform mit einer Säure und Isolieren der freien Säure auf übliche Weise regenerieren. Die freien Säureformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Säureformen.

Enthält eine erfindungsgemäße Verbindung mehr als eine Gruppe, die solche pharmazeutisch unbedenklichen Salze bilden kann, so umfaßt die Erfindung auch mehrfache Salze. Zu typischen mehrfachen Salzformen zählen zum Beispiel Bitartrat, Diacetat, Difumarat, Dimeglumin, Diphosphat, Dinatrium und Trihydrochlorid, was jedoch keine Einschränkung darstellen soll.

Im Hinblick auf das oben Gesagte sieht man, daß unter dem Ausdruck "pharmazeutisch unbedenkliches Salz" im vorliegenden Zusammenhang ein Wirkstoff zu verstehen ist, der eine Verbindung der Formel I in der Form eines ihrer Salze enthält, insbesondere dann, wenn diese Salzform dem Wirkstoff im Vergleich zu der freien Form des Wirkstoffs oder irgendeiner anderen Salzform des Wirkstoffs, die früher verwendet wurde, verbesserte pharmakokinetische Eigenschaften verleiht. Die pharmazeutisch unbedenkliche Salzform des Wirkstoffs kann auch diesem Wirkstoff erst eine gewünschte pharmakokinetische Eigenschaft verleihen, über die er früher nicht verfügt hat, und kann sogar die Pharmakodynamik dieses Wirkstoffs in bezug auf seine therapeutische Wirksamkeit im Körper positiv beeinflussen.

Zu den pharmakokinetischen Eigenschaften des Wirkstoffs, die günstig beeinflußt werden können, zählen z.B. die Art, wie dieser Wirkstoff durch Zellmembranen hindurch transportiert wird, was wiederum die Absorption, Verteilung, biologische Umwandlung und Exkretion dieses Wirkstoffs direkt und positiv beeinflussen kann. Obwohl der Verabreichungsweg der pharmazeutischen Zusammensetzung wichtig ist und verschiedene anatomische, physiologische und pathologische Aspekte die biologische Verfügbarkeit entscheidend beeinflussen können, hängt die Löslichkeit des Wirkstoffs üblicherweise von der Art seiner jeweiligen Salzform, die verwendet wird, ab. Weiterhin ist dem Fachmann deutlich, daß eine wäßrige Lösung des Wirkstoffs für die rascheste Absorption des Wirkstoffs in den Körper eines behandelten Patienten sorgt, während Lipidlösungen und -suspensionen sowie feste Dosierungsformen zu einer weniger raschen Absorption des Wirkstoffs führen.

Die orale Aufnahme eines Wirkstoffs der Formel I stellt aus Sicherheits-, Bequemlichkeits- und Sparsamkeitsgründen den am stärksten bevorzugten Verabreichungsweg dar, die Absorption einer derartigen oralen Dosierungsform kann jedoch durch physikalische Eigenschaften wie Polarität, durch Reizung der Magen-Darm-Schleimhaut hervorgerufenes Erbrechen, Abbau durch Verdauungsenzyme und niedrigen pH, ungleichmäßige Absorption oder Propulsion in Gegenwart von Nahrungsmitteln oder anderen Arzneistoffen sowie Stoffwechsel durch Enzyme der Schleimhaut, der Darmflora oder der Leber gestört werden. Die Formulierung des Wirkstoffs als unterschiedliche pharmazeutisch unbedenkliche Salzformen kann zur Überwindung oder Verringerung eines oder mehrerer der oben genannten Probleme im Zusammenhang mit der Absorption oraler Dosierungsformen wirksam sein.

Eine gemäß den hier beschriebenen Verfahren hergestellte Verbindung der Formel I läßt sich aus der Reaktionsmischung, in der sie endgültig hergestellt wird, auf jedem beliebigen üblichen Weg, mit dem der Chemiker auf dem Gebiet der organischen Synthese vertraut ist, abtrennen. Die abgetrennten Verbindungen lassen sich nach bekannten Verfahren reinigen. Für die Trennung und Reinigung lassen sich verschiedene Verfahren und Techniken verwenden, darunter z.B. Destillation, Umkristallisieren, Säulenchromatographie, lonenaustauschchromatographie, Gelchromatographie, Affinitätschromatographie, präparative Dünnschichtchromatographie sowie Lösungsmittelextraktion.

### Stereoisomere

Eine Verbindung, die der Formel I entspricht, kann dergestalt sein, daß die Atome, aus denen sie besteht, trotz identischer Verknüpfungen räumlich auf zwei oder mehr Wege angeordnet sein können. Diese Verbindung liegt infolgedessen in Form von Stereoisomeren vor. Cis-trans-Isomerie ist nur eine Art der Stereoisomerie. Sind die Stereoisomere Bild und Spiegelbild, die nicht zur Deckung gebracht werden können, so handelt es sich um Enantiomere mit Chiralität oder Händigkeit, da ein oder mehrere asymmetrische Kohlenstoffatome in der sie bildenden Struktur vorliegen. Enantiomere sind optisch aktiv und daher unterscheidbar, da sie die Ebene von polarisiertem Licht gleich stark, jedoch in entgegengesetzte Richtungen drehen.

Liegen in einer Verbindung der Formel I zwei oder mehr asymmetrische Kohlenstoffatome vor, so existieren an jedem dieser Kohlenstoffatome zwei mögliche Konfigurationen. Liegen zwei asymmetrische Kohlenstoffatome vor, existieren zum Beispiel vier mögliche Stereoisomere. Weiterhin lassen sich diese vier möglichen Stereoisomere in sechs mögliche Stereoisomerenpaare einteilen, die sich voneinander unterscheiden. Ein Molekülpaar mit mehr als einem asymmetrischen Kohlenstoff muß an jedem asymmetrischen Kohlenstoff unterschiedliche Konfigurationen aufweisen, um als Enantiomere zu gelten. Diejenigen Paare, die sich nicht wie Enantiomere verhalten, weisen eine unterschiedliche stereochemische Beziehung auf, die als diastereomere Beziehung bezeichnet wird. Stereoisomere, die keine Enantiomere sind, werden als Diastereoisomere oder häufiger Diastereomere bezeichnet.

Alle diese gut bekannten Aspekte der Stereochemie der Verbindungen der Formel I werden als Teil der vorliegenden Erfindung betrachtet. Die vorliegende Erfindung umfaßt daher Verbindungen der Formel I, die Stereoisomere sind, und, falls es sich bei diesen um Enantiomere handelt, die einzelnen Enantiomere, racemische Mischungen dieser Enantiomere sowie künstliche, d.h. synthetische Mischungen, die Anteile dieser Enantiomere enthalten, die sich von den in einer racemischen Mischung beobachteten Anteilen dieser Enantiomere unterscheiden. Umfaßt eine Verbindung der Formel I Stereoisomere, bei denen es sich um Diastereomere handelt, so umfaßt diese Verbindung die einzelnen Diastereomere sowie Mischungen von zwei oder mehr beliebigen dieser Diastereomeren in beliebigen Anteilen.

Zur Erläuterung soll folgendes dienen: existiert ein einziges asymmetrisches Kohlenstoffatom in einer Verbindung der Formel I, was zu ihren (-)(R)- und (+)(S)-Enantiomeren führt, so umfaßt diese Verbindung alle ihrer pharmazeutisch unbedenklichen Salzformen, Prodrugs und Metaboliten, die therapeutisch wirksam und nützlich zur Behandlung der bzw. Vorbeugung gegen die im weiteren Text beschriebenen Erkrankungen und Leiden sind. Liegt eine Verbindung der Formel I in Form von (-)(R)- und (+)(S)-Enantiomeren vor, so umfaßt diese Verbindung auch das (+)(S)-Enantiomer allein oder das (- )(R)-Enantiomer allein, wenn die therapeutische Wirksamkeit insgesamt, im wesentlichen oder hauptsächlich in nur einem dieser Enantiomere vorliegt bzw. wenn unerwünschte Nebenwirkungen in nur einem dieser Enantiomere vorliegen. Falls zwischen den biologischen Eigenschaften der beiden Enantiomere im wesentlichen kein Unterschied existiert, so umfaßt diese Verbindung der Formel I weiterhin das (+)(S)-Enantiomer und das (-)(R)-Enantiomer gemeinsam als racemische Mischung oder nichtracemische Mischung in einem beliebigen Verhältnis von entsprechenden Anteilen.

Die spezifischen biologischen Wirkungen bzw. physikalischen und chemischen Eigenschaften eines Paares oder Satzes von Enantiomeren einer Verbindung der Formel I - falls vorhanden - können zum Beispiel zur Bildung eines therapeutischen Endprodukts die Verwendung dieser Enantiomere in bestimmten Verhältnissen nahelegen. Zur Veranschaulichung soll folgendes dienen: falls ein Enantiomerenpaar existiert, können die Enantiomere in Verhältnissen wie 90% (R) - 10% (S), 80% (R) - 20% (S), 70% (R) - 30% (S), 60% (R) - 40% (S), 50% (R) - 50% (S), 40% (R) - 60% (S), 30% (R) - 70% (S), 20% (R) - 80% (S) und 10% (R) - 90% (S) verwendet werden. Nach der Auswertung der Eigenschaften der verschiedenen Enantiomeren einer Verbindung der Formel I -falls solche existieren - läßt sich die entsprechende Menge eines oder mehrerer dieser Enantiomere mit bestimmten erwünschten Eigenschaften, die das therapeutische Endprodukt bilden, auf einfache Weise ermittelt werden.

### Isotope

Es ist weiterhin vorgesehen, daß eine Verbindung der Formel I isotopenmarkierte Formen davon umfaßt. Eine isotopenmarkierte Form einer Verbindung der Formel I ist mit dieser Verbindung bis auf die Tatsache, daß eines oder mehrere Atome der Verbindung durch ein Atom bzw. Atome mit einer Atommasse oder Massenzahl ersetzt wurden, die sich von der Atommasse oder Massenzahl des Atoms, das üblicherweise natürlich vorkommt, unterscheidet, identisch. Zu den Isotopen, die leicht im Handel erhältlich sind und in eine Verbindung der Formel I nach gut bekannten Verfahren eingebaut werden können, zählen zum Beispiel Isotope von Wasserstoff, Kohlenstoff, Stickstoff, Sauerstoff, Phosphor, Fluor und Chlor, z.B. ²H, ³H, ¹³C, ¹⁴C, ¹⁵N, ¹⁸O, ¹⁷O, ³¹P, ³²P, ³⁵S, ¹⁸F bzw. ³⁶Cl. Eine Verbindung der Formel I, eines ihrer Prodrugs oder jeweils ein pharmazeutisch unbedenkliches Salz davon, die eines oder mehrere der oben genannten Isotope und/oder andere Isotope von anderen Atomen enthält, ist als Bestandteil der vorliegenden Erfindung vorgesehen. Eine isotopenmarkierte Verbindung der Formel I läßt sich auf vielerlei nützliche Art verwenden. Zum Beispiel eignet sich eine isotopenmarkierte Verbindung der Formel I, in die z.B. ein Radioisotop wie ³H oder ¹⁴C eingebaut worden ist, für Assays zur Verteilung des Arzneistoffs und/oder Substratgewebes. Diese Radioisotope, d.h. Tritium (³H) und Kohlenstoff-14 (¹⁴C), sind aufgrund ihrer einfachen Herstellung und ausgezeichneten Nachweisbarkeit besonders bevorzugt. Der Einbau schwererer Isotope, z.B. Deuterium (²H), in eine Verbindung der Formel I weist therapeutische Vorteile aufgrund der höheren Stabilität dieser isotopenmarkierten Verbindung im Metabolismus auf. Höhere Stabilität in Metabolismus bedeutet unmittelbar eine erhöhte Halbwertszeit in vivo oder niedrigere Dosierungen, was unter den meisten Umständen eine bevorzugte Ausführungsform der vorliegenden Erfindung darstellen würde. Eine isotopenmarkierte Verbindung der Formel I läßt sich üblicherweise durch Durchführung der in den Syntheseschemata und der damit in Zusammenhang stehenden Beschreibung, im Beispielteil und im Herstellungsteil im vorliegenden Text offenbarten Vorgehensweisen herstellen, wobei ein nicht isotopenmarkierter Reaktionspartner durch einen leicht verfügbaren isotopenmarkierten Reaktionspartner ersetzt wird.

Zur Manipulation des oxidativen Metabolismus der Verbindung über den primären kinetischen Isotopeneffekt kann auch Deuterium (²H) in eine Verbindung der Formel I eingebaut werden. Beim primären kinetischen Isotopeneffekt handelt es sich um eine Veränderung der Geschwindigkeit einer chemischen Reaktion aufgrund des Austausches isotopischer Kerne, was wiederum durch die Änderung der für die Bildung kovalenter Bindungen im Anschluß an diesen isotopischen Austausch erforderlichen Grundzustandsenergien verursacht wird. Der Austausch eines schwereren Isotops führt üblicherweise zu einer Erniedrigung der Grundzustandsenergie für eine chemische Bindung und verursacht so eine Verringerung der Geschwindigkeit bei einem geschwindigkeitslimitierenden Bindungsbruch. Findet der Bindungsbruch an bzw. in der Nähe einer Sattelpunktregion entlang der Koordinate einer Reaktion mit mehreren Produkten statt, so können sich die Produktverteilungsverhältnisse stark ändern. Zur Erläuterung: Wird Deuterium an ein Kohlenstoffatom in einer nichtaustauschbaren Position gebunden, so sind Geschwindigkeitsunterschiede von k_{M}/k_{D} = 2-7 typisch. Wird dieser Geschwindigkeitsunterschied erfolgreich auf eine oxidationsanfällige Verbindung der Formel I angewandt, so kann sich dadurch das Profil dieser Verbindung in vivo drastisch ändern und zu verbesserten pharmakokinetischen Eigenschaften führen.

Bei der Entdeckung und Entwicklung von Therapeutika versucht der Fachmann, pharmakokinetische Parameter zu optimieren und gleichzeitig wünschenswerte In-vitro-Eigenschaften beizubehalten. Man kann vernünftig annehmen, daß viele Verbindungen mit schlechten pharmakokinetischen Profilen gegenüber dem oxidativen Metabolismus anfällig sind. Aus derzeitig verfügbaren In-vitro-Assays mit Lebermikrosomen erhält man wertvolle Informationen über den Verlauf dieses oxidativen Metabolismus, aufgrund dessen wiederum deuterierte Verbindungen der Formel I mit einer verbesserten Stabilität durch Resistenz gegenüber einem derartigen oxidativen Metabolismus rational gestaltet werden können. So gelangt man zu wesentlichen Verbesserungen der pharmakokinetischen Profile der Verbindungen der Formel I, die sich quantitativ als erhöhte In-vivo-Halbwertszeit (T/2), Konzentration bei maximaler therapeutischer Wirkung (Cₘₐₓ), Fläche unter der Dosis-Wirkungskurve (AUC) sowie F und als verringerte Clearance, Dosis und Materialkosten ausdrücken lassen.

Zur Veranschaulichung des Obigen soll folgendes dienen: eine Verbindung der Formel I mit mehrfachen potentiellen Angriffsstellen für den oxidativen Metabolismus, z.B. Wasserstoffatome an einem Benzylrest und Wasserstoffatome, die an ein Stickstoffatom gebunden sind, wird als Reihe von Analogen hergestellt, in denen verschiedene Kombinationen von Wasserstoffatomen durch Deuteriumatome ersetzt werden, so daß einige, die meisten oder alle dieser Wasserstoffatome durch Deuteriumatome ersetzt sind. Durch Bestimmungen der Halbwertszeit gelangt man zu einer günstigen und genauen Bestimmung, wie sehr sich die Verbesserung der Widerstandsfähigkeit gegenüber oxidativen Metabolismen verbessert hat. Auf diese Weise wird bestimmt, daß aufgrund eines derartigen Austausches von Wasserstoff gegen Deuterium die Halbwertszeit der Ausgangsverbindung um bis zu 100% verlängert werden kann.

Der Austausch von Wasserstoff gegen Deuterium in einer Verbindung der Formel I läßt sich auch dazu verwenden, um zu einer günstigen Änderung des Stoffwechselproduktspektrums der Ausgangsverbindung zwecks Verringerung oder Ausschluß von unerwünschten toxischen Stoffwechselprodukten zu gelangen. Entsteht zum Beispiel ein toxisches Stoffwechselprodukt aufgrund der Spaltung einer oxidativen Kohlenstoff-Wasserstoff (C-H)-Bindung kann vernünftigerweise angenommen werden, daß das deuterierte Analog die Produktion des unerwünschten Stoffwechselprodukts wesentlich verringert oder ausschließt, sogar dann, wenn es sich bei der jeweiligen Oxidation nicht um einen geschwindigkeitsbestimmenden Schritt handelt. Weitere Informationen zum Stand der Technik in bezug auf den Austausch von Wasserstoff gegen Deuterium finden sich z.B. bei Hanzlik et al., J. Org. Chem. 55, 3992-3997, 1990, Reider et al., J. Org. Chem. 52, 3326-3334, 1987, Foster, Adv. Drug Res. 14, 1-40, 1985, Gillette et al., Biochemistry 33(10), 2927-2937, 1994, und Jarman et al., Carcinogenesis 16(4), 683-688, 1993.

### Therapeutische Anwendungen

Die Erfindung betrifft weiterhin die Verwendung von Verbindungen der Formel I zur Behandlung von Myocarderkrankungen.

Koronare Herzerkrankungen stellen die häufigste Todesursache in der westlichen Welt dar. Bei einem kritisch verengten Herzkranzgefäß kann ein reduzierter Blutstrom zu Myokardischämie führen. Wird mit einer Reperfusion begonnen, so führt dies je nach der Schwere des vorangegangenen Ischämieschubs zu einer reversiblen oder nichtreversiblen Schädigung des Myokards, die durch langandauernde Depression oder den irreversiblen Verlust der Kontraktionsfähigkeit gekennzeichnet ist. Je nach der Größe der betroffenen Myokardregion kann es zu einem akuten oder chronischen Herzversagen kommen.

Ein besonderes klinisches Problem bei dem oben beschriebenen Fall ist die Entstehung einer Restenose nach anfänglich erfolgreicher Reperfusion durch PTKA, auch nach Implantation eines Stents, nach Thrombolyse oder nach Transplantation eines aorto-koronaren Bypasses. Aus experimentellen Tierversuchen wie klinischen Versuchen gibt es Hinweise, daß bei den verschiedenen oben genannten Herzerkrankungen, d.h. koronare Herzerkrankung selbst, reversible oder irreversible Myokardischämie/Reperfusionsschädigung, akutes oder chronisches Herzversagen und Restenose, darunter auch In-Stent-Restenose und Stent-in-Stent-Restenose, Entzündungsvorgänge eine zufällige Rolle spielen. An diesen Entzündungsvorgängen sind existierende sowie einwandernde Makrophagen sowie Neutrophile und TH₁- und TH₂-Helferzellen beteiligt. Diese Leukozytenreaktion führt zu dem charakteristischen Cytokinmuster, an dem TNF-α, IL-1 β, IL-2 und IL-6 sowie IL-10 und IL-13 beteiligt sind (Pulkki KJ: Cytokines and cardiomyocyte death. Ann. Med. 1997 29: 339-343.

Birks EJ, Yacoub MH: The role of nitric oxide and cytokines in heart failure. Coron.Artery.Dis. 1997 8: 389-402).
Die Bildung dieser Spezies wurde an menschlichen Patienten mit Myokardischämie nachgewiesen. Im Tiermodell zeigt sich, daß die Cytokinproduktion der Einwanderung peripherer Makrophagen und Neutrophilen korreliert ist, wodurch die Schädigung in das noch intakte Myokard verschleppt werden kann.

Der wichtigste Faktor bei der Cytokinantwort ist jedoch TNF-α, der entzündliche und pro-apoptotische Reaktionen vereinigt und außerdem eine direkte negativ-ionotrope Wirkung auf die Herzmyozyten ausübt. (Ceconi C, Curello S, Bachetti T, Corti A, Ferrari R: Tumor necrosis factor in congestive heart failure: a mechanism of disease for the new millennium? Prog.Cardiovasc.Dis. 1998 41: 25-30.
Mann DL: The effect of tumor necrosis factor-alpha on cardiac structure and function: a tale of two cytokines. J.Card.Fail. 1996 2: S165-S172. Squadrito F, Altavilla D, Zingarelli B, et al.: Tumor necrosis factor involvement in myocardial ischaemia-reperfusion injury. Eur.J.Pharmacol. 1993 237: 223-230).

An Tiermodellen des Myokardinfarkts wurde gezeigt, daß TNF-α während der Reperfusionsphase rasch freigesetzt wird (Herskowitz A, Choi S, Ansari AA, Wesselingh S: Cytokine mRNA expression in postischemic/reperfused myocardium. Am.J.Pathol. 1995 146: 419-428) und daß die Schutzwirkungen von Arzneistoffen wie Dexamethason (Arras M, Strasser R, Mohri M, et al.: Tumor necrosis factor-alpha is expressed by monocytes/macrophages following cardiac microembolization and is antagonized by cyclosporine. Basic.Res.Cardiol. 1998 93: 97-107), Cyclosporin A (Arras M, Strasser R, Mohri M, et al.: Tumor necrosis factor-alpha is expressed by monocytes/macrophages following cardiac microembolization and is antagonized by cyclosporine. Basic.Res.Cardiol. 1998 93: 97-107.
Squadrito F, Altavilla D, Squadrito G, et al.: Cyclosporin-A reduces leukocyte accumulation and protects against myocardial ischaemia reperfusion injury in rats. Eur.J.Pharmacol. 1999 364: 159-168) oder Clorichromen (Squadrito F, Altavilla D, Zingarelli B, et al.: The effect of cloricromene, a coumarine derivate, on leukocyte accumulation, myocardial necrosis and TNF-alpha production in myocardial ischaemia-reperfusion injury. Life Sci. 1993 53: 341-355) mit einer Verringerung von zirkulierendem TNF- α einhergehen.

PDE IV-Inhibitoren der Formel I sind wirksame Antagonisten der Makrophagen- und T-Zellen-Cytokinproduktion. Sie hemmen außerdem die Proliferation von T-Zellen. Die Hemmung von PDE IV kann daher eine günstige Wirkung bei denjenigen Myokarderkrankungen, bei denen ein kausaler Zusammenhang mit der Cytokinproduktion und mit entzündlichen Vorgängen besteht, ausüben.

Im Vergleich mit PDE III-Inhibitoren und dem frühen PDE IV-Inhibitoren Rolipram fehlen bevorzugten PDE IV-Inhibitoren hämodynamische Nebenwirkungen, die bei der Behandlung der am meisten Herz-Kreislauf-Störungen zu einer Einschränkung der Dosis führen können.

Das Ziel der Erfindung bestand darin, neue Verwendungsmöglichkeiten von Verbindungen mit wertvollen Eigenschaften zu entdecken, insbesondere von denjenigen, die zur Herstellung von Arzneimitteln verwendet werden können.

Es wurde gefunden, daß die Verbindungen der Formel I und ihre Salze äußerst wertvolle pharmakologische Eigenschaften mit guter Verträglichkeit zur Behandlung von Myokarderkrankungen in sich vereinigen.

Die Erfindung sieht vorzugsweise die Verwendung der Verbindungen der Formel I zur Herstellung eines Arzneimittels zur Behandlung von Myokarderkrankungen vor, wobei diese Myokarderkrankungen entzündliche und immunologische Merkmale aufweisen.

Am stärksten bevorzugt sieht die Erfindung die Verwendung der Verbindungen der Formel I zur Herstellung eines Arzneimittels zur Behandlung von koronaren Herzerkrankungen, reversibler oder irreversibler Myokardischämie/Reperfusionsschädigung, akutem oder chronischem Herzversagen und Restenose, darunter auch In-Stent-Restenose und Stent-in-Stent-Restenose, vor.

Bevorzugt sieht die Erfindung die Verwendung der Verbindungen der Formel I zur Herstellung eines Arzneimittels zur Behandlung oder Vorbeugung von einer oder mehreren aus der Gruppe der folgenden Erkrankungen, krankhaften Störungen und Leiden vor:
Asthma jeglicher Art, Ätiologie oder Pathogenese, oder Asthma aus der Gruppe atopisches Asthma, nichtatopisches Asthma, allergisches Asthma, IgE-vermitteltes atopisches Asthma, Bronchialasthma, essentielles Asthma, Primärasthma, durch pathophysiologische Störungen hervorgerufenes endogenes Asthma, durch Umweltfaktoren hervorgerufenes exogenes Asthma, essentielles Asthma unbekannter oder inapparenter Ursache, nichtatopisches Asthma, bronchitisches Asthma, emphysematöses Asthma, durch Belastung induziertes Asthma, Berufsasthma, durch Bakterien-, Pilz-, Protozoen- oder Virusinfektion hervorgerufenes infektallergisches Asthma, nichtallergisches Asthma, inzipientes Asthma, "wheezy infant syndrome";
chronische oder akute Bronchokonstriktion, chronische Bronchitis, Obstruktion der kleinen Atemwege sowie Emphysem;
obstruktive oder entzündliche Atemwegserkrankung jeglicher Art, Ätiologie oder Pathogenese, oder eine obstruktive oder entzündliche Atemwegserkrankung aus der Gruppe Asthma; Staublunge, chronische eosinophile Pneumonie; chronischer obstruktive pulmonaler Krankheit (COPD); COPD inklusive chronische Bronchitis, Lungenemphysem oder damit assoziierte Atemnot, durch irreversible, fortschreitende Obstruktion der Atemwege gekennzeichnete COPD, Schocklunge (adult respiratory distress syndrome, ARDS) sowie Verschärfung der Überempfindlichkeit der Atemwege aufgrund Therapie mit anderen Arzneistoffen;
Staublunge jeglicher Art, Ätiologie oder Pathogenese, oder Staublunge aus der Gruppe Aluminose oder Aluminiumstaublunge, Anthrakose(-Asthma), Asbestose oder Asbeststaublunge, Chalikose oder Kalkstaublunge, durch Einatmen von Straußenfedernstaub verursachte Ptilose, durch Einatmung von Eisenteilchen verursachte Siderose, Silikose oder Steinstaublunge, Byssinose oder Baumwollstaubpneumokoniose sowie Talkpneumokoniose;
Bronchitis jeglicher Art, Ätiologie oder Pathogenese, oder Bronchitis aus der Gruppe akute Bronchitis, akute laryngotracheale Bronchitis, durch Erdnüsse ausgelöste Bronchitis, Bronchialkatarrh, kruppöse Bronchitis, Bronchitis ohne Auswurf, infektiöse Asthmabronchitis, Bronchitis mit Auswurf, Staphylokokken- oder Streptokokkenbronchitis; sowie Vesikulärbronchitis;
Bronchiektasie jeglicher Art, Ätiologie oder Pathogenese, oder Bronchiektasie aus der Gruppe zylindrische Bronchiektasie, sackförmige Bronchiektasie, spindelförmige Bronchiektasie, Bronchiolendilatation, zystische Bronchiektasie, Bronchiektasie ohne Auswurf, sowie follikuläre Bronchiektasie;
jahreszeitlich bedingte allergische Rhinitis, perenniale allergische Rhinitis, oder Sinusitis jeglicher Art, Ätiologie oder Pathogenese, oder Sinusitis aus der Gruppe eitriger oder nichteitriger Sinusitis, akute oder chronische Sinusitis, Ethmoiditis, Stimhöhlenentzündung, Kieferhöhlenentzündung oder Sphenoiditis;
rheumatoide Arthritis jeglicher Art, Ätiologie oder Pathogenese, oder rheumatoide Arthritis aus der Gruppe akute Arthritis, akute Gichtarthritis, primär chronische Polyarthritis, Osteoarthrose, Infektarthritis, Lyme-Arthritis, progrediente Arthritis, Arthritis psoriatica, sowie Spondylarthritis;
Gicht sowie mit Entzündung assoziierte Fieber bzw. mit Entzündung assoziierte Schmerz;
eine mit Eosinophilen in Zusammenhang stehende krankhafte Störung jeglicher Art, Ätiologie oder Pathogenese, oder eine mit Eosinophilen in Zusammenhang stehende krankhafte Störung aus der Gruppe Eosinophilie, eosinophiles Lungeninfiltrat, Löffler-Syndrom, chronische eosinophile Pneumonie, tropische Lungeneosinophilie, bronchopneumonische Aspergillose, Aspergillom, eosinophiles Granulom, allergische granulomatöse Angiitis bzw. Churg-Strauss-Syndrom, Polyarteriitis nodosa (PAN), sowie systemische Vasculitis necroticans;
atopische Dermatitis, allergische Dermatitis, oder allergisches oder atopisches Ekzem;
Nesselsucht jeglicher Art, Ätiologie oder Pathogenese, oder Nesselsucht aus der Gruppe immunbedingte Nesselsucht, Komplementbedingte Nesselsucht, durch Nesselsucht auslösendes Material induzierte Nesselsucht, durch physikalische Reize ausgelöste Nesselsucht, durch Streß ausgelöste Nesselsucht, idiopatische Nesselsucht, akute Nesselsucht, chronische Nesselsucht, angioneurotisches Ödem, Urticaria cholinergica, Kälteurtikaria in ihrer autosomal-dominanten Form oder in ihrer erworbenen Form, Kontakturtikaria, Urticaria gigantean sowie Papelurtikaria;
Konjunktivitis jeglicher Art, Ätiologie oder Pathogenese, oder Konjunktivitis aus der Gruppe Conjunctivitis actinica, akute katarrhalische Konjunktivitis, akute contagiöse Konjunktivitis, allergische Konjunktivitis, atopische Konjunktivitis, chronische katarrhalische Konjunktivitis, eitrige Konjunktivitis sowie Frühjahrskonjunktivitis;
Uveitis jeglicher Art, Ätiologie oder Pathogenese oder Uveitis aus der Gruppe Entzündung der ganzen Uvea oder eines Teils davon, Uveitis anterior, Iritis, Cyclitis, Iridocyclitis, granulomatöse Uveitis, nichtgranulomatöse Uveitis, phakoantigene Uveitis, Uveitis posterior, Choroiditis sowie Choriorethinitis;
Schuppenflechte;
multiple Sklerose jeglicher Art, Ätiologie oder Pathogenese, oder multiple Sklerose aus der Gruppe primär progrediente multiple Sklerose sowie multiple Sklerose mit schubweisem Verlauf und Neigung zu Remissionen;
Autoimmun-/Entzündungserkrankungen jeglicher Art, Ätiologie oder Pathogenese, oder eine Autoimmun-/Entzündungserkrankung aus der Gruppe autoimmunhämatologische Störungen, härnolytische Anämie, aplastische Anämie, aregenerative Anämie, idiopatische thrombozytopene Purpura, systemischer Lupus erythematosus, Polychondritis, Skleroderm, Wegener-Granulomatose, Lichtkrankheit, chronisch-aktive Hepatitis, Myasthenia gravis, Stevens-Johnson-Syndrom, idiopathische Sprue, Autoimmun-Reizkolonerkrankungen, Colitis ulcerosa, Morbus Crohn, endokrine Opthamopathy, Basedow-Krankheit, Sarkoidose, Alveolitis, chronische Hypersensitivitätspneumonitis, primär biliäre Zirrhose, Insulinmangeldiabetes oder Typ 1 Diabetes mellitus, Uveitis anterior, granulomatöse Uveitis oder Uveitis posterior, Keratoconjunctivitis sicca, Keratoconjunctivitis epidemica, (diffuse) interstitielle Lungenfibrose, Lungenzirrhose, Mukoviszidose, Arthritis psoriatica, Glomerulonephritis mit und ohne Nephrose, akute Glomerulonephritis, idiopathische Nephrose, Minimal-Change-Nephropathie, entzündliche/ hyperproliferative Hauterkrankungen, Schuppenflechte, atopische Dermatitis, Kontaktdermatitis, allergische Kontaktdermatitis, familiärer gutartiger Pemphigus, Pemphigus erythematosus, Pemphigus foliaceus sowie Pemphigus vulgaris;
Vorbeugung einer Fremdtransplantatabstoßung nach Organtransplantation,
Reizdarm (inflammatory bowel disease, IBD) jeglicher Art, Ätiologie oder Pathogenese, oder Reizdarm aus der Gruppe ulzerative Kolitis (UC), kollagenöse Kolitis, Colitis polyposa, transmurale Kolitis sowie Morbus Crohn (CD);
septischer Schock jeglicher Art, Ätiologie oder Pathogenese, oder septischer Schock aus der Gruppe Nierenversagen, akutes Nierenversagen, Kachexie, Malariakachexie, hypophysäre Kachexie, uremämische Kachexie, Herzkachexie, Cachexia suprarenalis bzw. Addison-Krankheit, karzinomatöse Kachexie sowie Kachexie auf Grund von Infektion durch Human Immunodeficiency Virus (HIV);
Leberschädigung;
pulmonaler Hochdruck sowie durch Sauerstoffmangel hervorgerufener pulmonaler Hochdruck;
Knochenschwunderkrankungen, primäre Osteoporose und sekundäre Osteoporose;
krankhafte Störungen des Zentralnervensystems jeglicher Art, Ätiologie oder Pathogenese, oder eine krankhafte Störung des Zentralnervensystems aus der Gruppe Depression, Morbus Parkinson, Lern- und Gedächtnisstörungen, tardive Dyskinesie, Drogenabhängigkeit, arteriosklerotische Demenz, sowie Demenz als Begleiterscheinung von Chorea Huntington, Morbus Wilson, Paralysis agitans sowie Thalamusatrophien;
Infektionen, insbesondere Virusinfektionen, wobei diese Viren die Produktion von TNF-α in ihrem Wirt erhöhen oder wobei diese Viren gegenüber Hinaufregulierung von TNF-α, in ihrem Wirt empfindlich sind, so daß ihre Replikation oder andere wichtigen Aktivitäten behindert werden, darunter Viren aus der Gruppe HIV-1, HIV-2 und HIV-3, Zytomegalievirus, CMV; Grippe, Adenoviren und Herpesviren, darunter Herpes zoster und Herpes simplex;
Hefe- und Pilzinfektionen, wobei diese Hefen und Pilze gegenüber Hinaufregulierung durch TNF-α empfindlich sind oder die TNF-α-Produktion in ihrem Wirt auslösen, z.B. Pilzmeningitis, insbesondere bei gemeinsamer Verabreichung mit anderen Arzneistoffen der Wahl zur Behandlung systemischer Hefe- und Pilzinfektionen, darunter den Polymycinen, z.B. Polymycin B, Imidazolen, z.B. Clotrimazol, Econazol, Miconazol und Ketoconazol, den Triazolen, z.B. Fluconazol und Itranazol, sowie den Amphotericinen, z.B. Amphotericin B und liposomales Amphotericin B, was jedoch keine Einschränkung darstellen soll.
Ischämie-Reperfusionsschädigung, Autoimmundiabetes, retinale Autoimmunität, chronische lymphozytische Leukämie, HIV-Infektionen, Lupus erythematosus, Nieren- und Hamleitererkrankungen, krankhafte Urogenital- und Gastrointestinalstörungen, sowie Prostataerkrankungen.

Insbesondere eignen sich Verbindungen der Formel I zur Behandlung von (1) Entzündungserkrankungen und -leiden inklusive Gelenkentzündung, rheumatoide Arthritis, rheumatoide Spondylitis, Osteoarthritis, Reizdarm, ulzerative Kolitis, chronische Glomerulonephritis, Dermatitis sowie Morbus Crohn, (2) Erkrankungen und Leiden der Atemwege, inklusive Asthma, Schocklunge, chronische Pulmonitis, Bronchitits, chronische obstruktive Atemwegerkrankung sowie Silikose, (3) Infektionskrankheiten und -leiden inklusive Sepsis, septischer Schock, endotoxischer Schock, gramnegative Sepsis, toxisches Schocksyndrom, durch Bakterien-, Virus- oder Pilzinfektionen hervorgerufenes Fieber bzw. Myalgie, sowie Grippe; (4) Immunerkrankungen und -leiden inklusive Autoimmundiabetes, systemischer Lupus erythematosis, GvH-Reaktion, Abstoßung von Fremdtransplantaten,multiple Sklerose, Schuppenflechte und allergische Rhinitis, sowie (5) weitere Erkrankungen und Leiden inklusive Knochenresorptionserkrankungen, Reperfusionsschädigung, sekundäre Kachexie aufgrund Infektion oder Malignität, sekundäre Kachexie aufgrund AIDS, Infektion mit Human Immune Deficiency Virus (HIV), oder AIDS-related-Complex (ARC), Keloidbildung, Narbengewebsbildung, Typ 1 Diabetes mellitus sowie Leukämie.

Die vorliegende Erfindung betrifft weiterhin die Kombination einer Verbindung der Formel I zusammen mit einer oder mehreren Mitgliedern der folgenden Gruppe:
(a) Leukotrien-Biosyntheseinhibitoren: 5-Lipoxygenase (5-LO)-Inhibitoren und Antagonisten des 5-Lipoxygenase-aktivierenden Proteins (FLAP) aus der Gruppe Zileuton, ABT-761, Fenleuton, Tepoxalin, Abbott-79175, Abbott-85761, N-(5-substituierte) Thiophen-2-alkylsulfonamide, 2,6-di-tert.-Butylphenolhydrazone, die Klasse der Methoxytetrahydropyrane, darunter Zeneca ZD-2138, die Verbindung SB-210661 und die Klasse, zu der sie gehört, die Klasse der pyridinylsubstituierten 2-Cyannaphthalin-Verbindungen, darunter L 739,010, die Klasse der 2-CyanchinolinVerbindungen, darunter L-746,530, die Klassen der Indol- und Chinolinverbindungen, darunter MK-591, MK-886 und BAY x 1005; (b) Rezeptorantagonisten für die Leukotriene LTB₄, LTC₄, LTD₄ und LTE₄ aus der Gruppe der Klasse der Phenothiazin-3-on-Verbindungen, darunter L-651,392, der Klasse der Amidinoverbindungen, darunter CGS-25019c, der Klasse der Benzoxaolamine, darunter Ontazolast, der Klasse der Benzolcarboximidamide, darunter BIIL 284/260, sowie der Verbindungsklassen, zu denen Zafirlukast, Ablukast, Montelukast, Pranlukast, Verlukast (MK-679), RG-12525, Ro-245913, Iralukast (CGP 45715A) und BAY x 7195 gehören; (c) PDE IV-Inhibitoren; (d) 5-Lipoxygenase-Inhibitoren (5-LO); oder Antagonisten des 5-Lipoxygenase-aktivierenden Proteins (FLAP); (e) Doppelinhibitoren der 5-Lipoxygenase (5-LO) und Antagonisten des blutplättchenaktivierenden Faktors (platelet activating factor PAF); (f) Leukotrienantagonisten (LTRAs) darunter LTB₄, LTC₄, LTD₄ und LTE₄-Antagonisten; (g) Antihistamin-H₁-Rezeptorantagonisten, darunter Cetirizin, Loratadin, Desloratadin, Fexofenadin, Astemizol, Azelastin und Chlorpheniramin; (h) gastroprotektive H₂-Rezeptorantagonisten; (i) oral oder topisch verabreichte α₁- und α₂-Adrenorezeptor-Agonist-Vasokonstriktor-Sympathomimetika zur Schleimhautabschwellung, darunter Propylhexedrin, Phenylephrin, Phenylpropanolamin, Pseudoephedrin, Naphazolinhydrochlorid, Oxymetazolinhydrochlorid, Tetrahydrozolinhydrochlorid, Xylometazolinhydrochlorid sowie Ethylnorepinephrinhydrochlorid; j) α₁- und α₂-Adrenorezeptoragonisten in Kombination mit Inhibitoren der 5-Lipoxygenase (5-LO); (k) Anticholinergika, darunter lpratropiumbromid, Tiotropiumbromid, Oxitropiumbromid, Pirenzepin sowie Telenzepin; (I) β₁- bis β₄-Adrenorezeptoragonisten, darunter Metaproterenol, Isoproterenol, Isoprenalin, Albuterol, Salbutamol, Formoterol, Salmeterol, Terbutalin, Orciprenalin, Bitolterolmesylat und Pirbuterol; (m) Methylxanthanine, darunter Theophyllin und Aminophyllin; (n) Natriumcromoglycat; (o) Muscarinrezeptor (M1, M2 und M3)-Antagonisten; (p) COX-1-Inhibitoren (NSAIDs); COX-2-selektive Inhibitoren, darunter Rofecoxib, sowie Stickoxid-NSAIDs; (q) Mimetika des insulinähnlichen Wachstumsfaktors Typ I (IGF-1); (r) Ciclesonid; (s) Inhalations-Glucokortikoide mit verringerten systemischen Nebenwirkungen, darunter Prednison, Prednisolon, Flunisolid, Triamcinolonacetonid, Beclomethasondipropionat, Budesonid, Fluticasonpropionat sowie Mometasonfuroat; (t) Tryptaseinhibitoren; (u) Antagonisten des blutplättchenaktivierenden Faktors (PAF); (v) monoklonale Antikörper gegen endogene entzüdliche Körper; (w) IPL 576; (x) Anti-Tumor Nekrose Faktor (TNFα)-Mittel, darunter Etanercept, Infliximab und D2E7; (y) DMARDs, darunter Leflunomid; (z) TCR-Peptide; (aa) Inhibitoren des interleukinumwandelnden Enzyms (interleukin converting enzyme, ICE); (bb) IMPDH-Inhibitoren; (cc) Adhäsionsmolekülinhibitoren, darunter VLA-4-Antagonisten; (dd) Kathepsine; (ee) MAP-Kinaseinhibitoren; (ff) Glucose-6-phosphat-dehydrogenase-Inhibitoren; (gg) Kinin-B₁- und -B₂-Rezeptor-Antagonisten; (hh) Gold in Form einer Aurothiogruppe zusammen mit verschiedenen hydrophilen Gruppen; (ii) Immunsuppressiva, z.B. Cyclosporin, Azathioprin und Methotrexat; (jj) Mittel gegen Gicht, z.B. Kolchizin; (kk) Xanthinoxidaseinhibitoren z.B. Allopurinol; (II) Urikosurika z.B. Probenecid, Sulfinpyrazon und Benzbromaron; (mm) Antineoplastica, insbesondere antimitotische Arzneistoffe, darunter die Vinca-Alkaloide wie Vinblastin und Vincristin; (nn) Mittel zur Förderung der Wachstumshormonsekretion; (oo) Inhibitoren der Matrixmetalloproteasen (MMPs), d.h. die Stromelysine, Kollagenasen und Gelatinasen sowie Aggrecanase, insbesondere Kollagenase-1 (MMP-1), Kollagenase-2 (MMP-8), Kollagenase-3 (MMP-13), Stromelysin-1 (MMP-3), Stromelysin-2 (MMP-10) und Stromelysin-3 (MMP-11); (pp) "transforming growth factor" (TGFβ); (qq) platelet-derived growth factor" (PDGF); (rr) Fibroblasten-Wachstumsfaktor, z.B. "basic fibroblast growth factor" (bFGF); (ss) "granulocyte macrophage colony stimulating factor" (GM-CSF); (tt) Capsaicin; (uu) Tachykinin- NK₁- und - NK₃-Rezeptor-Antagonisten aus der Gruppe NKP-608C; SB233412 (Talnetant) und D-4418; sowie (w) Elastaseinhibitoren aus der Gruppe UT-77 und ZD-0892.

Die vorliegende Erfindung betrifft eine Kombination einer Verbindung der Formel I zusammen mit einem oder mehreren zusätzlichen Therapeutika zur gemeinsamen Verabreichung an einen Patienten, um ein besonders erwünschtes therapeutisches Endergebnis zu erzielen. Bei dem zweiten usw. Therapeutikum kann es sich ebenfalls um eine oder mehrere Verbindungen wie oben beschrieben oder eine oder mehrere PDE IV-Inhibitoren handeln, die auf diesem Fachgebiet bekannt und hier genauer beschrieben sind. Insbesondere wird das zweite usw. Therapeutikum aus einer unterschiedlichen Klasse von Therapeutika ausgewählt. Diese gewählten Kombinationen sind unten genauer beschrieben.

Im vorliegenden Zusammenhang sollen, falls sie sich auf die Verbindungen der Formel I und ein oder mehrere andere Therapeutika beziehen, die Ausdrücke "gemeinsame Verabreichung", "gemeinsam verabreicht" und "in Kombination mit" folgendes bedeuten und betreffen und umfassen folgendes:
(a) gleichzeitige Verabreichung einer derartigen Kombination von einer oder mehreren Verbindung(en) und einem Therapeutikum bzw. mehreren Therapeutika an einen behandlungsbedürftigen Patienten, wenn diese Komponenten gemeinsam als eine einzige Dosierungsform formuliert sind, die diese Komponenten im wesentlichen gleichzeitig an den Patienten freigibt,
(b) im wesentlichen gleichzeitige Verabreichung einer solchen Kombination von einer oder mehreren Verbindung(en) und einem Therapeutikum bzw. mehreren Therapeutika an einen behandlungsbedürftigen Patienten, wenn diese Komponenten separat als separate Dosierungsformen formuliert sind, die im wesentlichen gleichzeitig von dem Patienten eingenommen werden, und die Komponenten im wesentlichen gleichzeitig an diesen Patienten freigegeben werden,
(c) Verabreichung einer derartigen Kombination von einer oder mehreren Verbindungen(en) und einem Therapeutikum bzw. mehreren Therapeutika an einen behandlungsbedürftigen Patienten nacheinander, wenn diese Komponenten separat voneinander als separate Dosierungsformen formuliert sind, die von dem Patienten zu aufeinanderfolgenden Zeitpunkten mit einem deutlichen Zeitabstand zwischen jeder Einnahme eingenommen werden, und die Komponenten zu im wesentlichen unterschiedlichen Zeitpunkten an den Patienten freigegeben werden; sowie
(d) Verabreichung einer derartigen Kombination von einer oder mehreren Verbindung(en) und einem Therapeutikum bzw. mehreren Therapeutika an einen behandlungsbedürftigen Patienten nacheinander, wenn diese Komponenten gemeinsam als eine einzige Dosierungsform formuliert sind, die diese Komponenten auf kontrollierte Weise freigibt, und die Komponenten so von dem Patienten gleichzeitig, nacheinander bzw. überlappend zum gleichen Zeitpunkt bzw. zu unterschiedlichen Zeitpunkten eingenommen werden.

### Kombinationen mit Leukoteien-Biosyntheseinhibitorenn: 5-Lioxygenase (5-LO)-Inhibitoren sowie Antagonisten des 5-Lipoxygenase-aktivierenden Proteins (FLAP)

Zur Bildung von erfindungsgemäßen Ausführungsformen wird (werden) eine oder mehrere der Verbindungen der Formel I in Kombination mit Leukotrien-Biosyntheseinhibitorenn, d.h. 5-Lipoxygenaseinhibitorenn bzw. Antagonisten des 5-Lipoxygenase-aktivierenden Proteins verwendet. Die 5-Lipoxygenase (5-LO) stellt eine von zwei Enzymgruppen dar, die Arachidonsäure metabolisieren, wobei es sich bei der anderen Gruppe um die Cyclooxygenasen COX-1 und COX-2 handelt.

Bei dem 5-Lipoxygenase-aktivierenden Protein handelt es sich um ein 18 kDa großes membrangebundenes Arachidonat-bindendes Protein, das die Umwandlung der Arachidonsäure in der Zelle durch 5-Lipoxygenase stimuliert. Die Arachidonsäure wird in 5-Hydroperoxyeicosatetraensäure (5-HPETE) umgewandelt, und dieser Weg führt schließlich zur Bildung von entzündlichen Leukotrienen; die Blockierung des 5-Lipoxygenase-aktivierenden Proteins oder des Enzyms 5-Lipoxygenase selbst stellt daher ein wünschenswertes Ziel dar, um diesen Weg günstig zu beeinflussen. Einer dieser 5-Lipoxygenaseinhibitoren ist Zileuton.
Zu den Klassen der Leuktotriensyntheseinhibitoren, die sich zur Bildung von therapeutischen Kombinationen mit den Verbindungen der Formel I eignen, zählen folgende:
(a) Redox-Mittel, darunter N-Hydroxyhamstoffe, N-Alkylhydroxamidsäuren, Selenit, Hydroxybenzofurane, Hydroxylamine und Katechin, siehe Ford-Hutchinson et al., "5-Lipoxygenase," Ann. Rev. Biochem. 63, 383-417, 1994; Weitzel und Wendel, "Selenoenzymes regulate the activity of leukocyte 5-lipoxygenase via the peroxide tone," J. Biol. Chem. 268, 6288-92, 1993; Bjömstedt et al. "Selenite incubated with NADPH and mammalian thioredoxin reductase yields selenide, which inhibits lipoxygenase and changes the electron spin resonance spectrum of the active site iron," Biochemistry 35, 8511-6, 1996, und Stewart et al., "Structure-activity relationships of N-hydroxyurea 5-lipoxygenase inhibitors," J. Med. Chem. 40, 1955-68, 1997;
(b) Alkylierungsmittel und Verbindungen, die mit SH-Gruppen reagieren, hemmen nachweislich die Leukotriensynthese in vitro, siehe Larsson et al., "Effects of 1-chloro-2,4,6-trinitrobenzene on 5-lipoxygenase activity and cellular leukotriene synthesis," Biochem. Pharmacol. 55, 863-71, 1998 und
(c) kompetitive Inhibitoren der 5-Lipoxygenase auf der Grundlage von Thiopyranoindol- und Methoxyalkylthiazolstrukturen, die als Nichtredox-Inhibitoren der 5-Lipoxygenase wirken; siehe Ford-Hutchinson et al., ibid.; und Hamel et al., "Substituted (pyridylmethoxy)naphthalenes as potent and orally active 5-lipoxygenase inhibitors - synthesis, biological profile und pharmacokinetics of L-739,010," J. Med. Chem. 40, 2866-75, 1997.

Die Beobachtung, daß Arachidonsäurehydroxamat die 5-Lipoxygenase hemmt, führte zu der Entdeckung von klinisch brauchbaren selektiven 5-Lipoxygenaseinhibitorenn, wie den N-Hydroxyhamstoffderivaten Zileuton und ABT-761, die unten dargestellt sind:

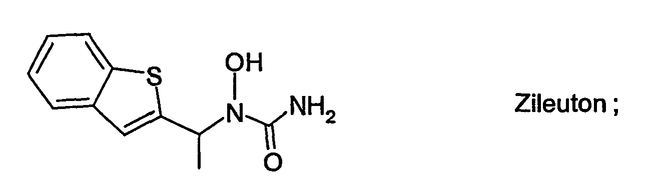

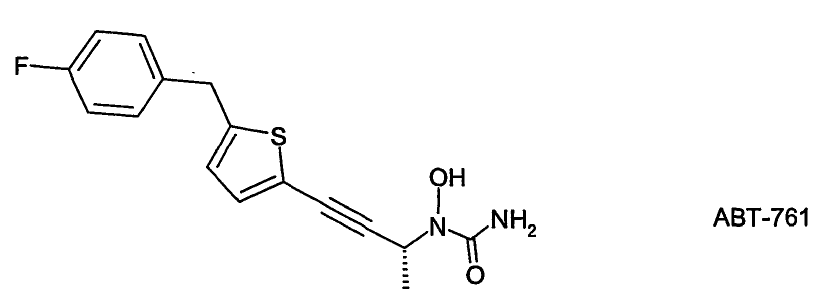

Eine weitere N-Hydroxyhamstoffverbindung ist Fenleuton (Abbott-76745):

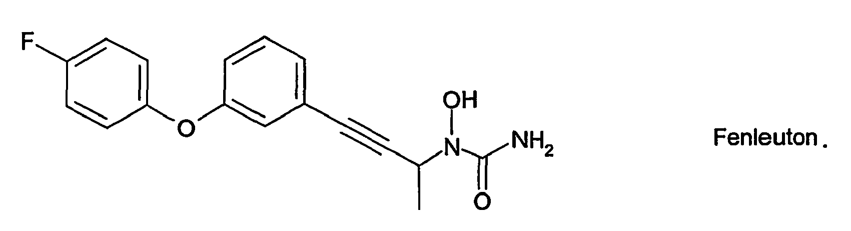

Eine weitere N-Hydroxyhamstoffverbindung ist Abbott-79175 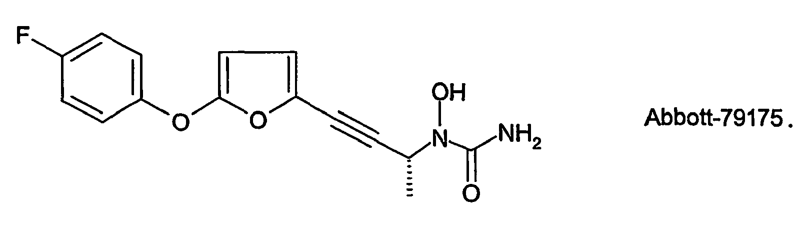 Abbott-79175 weist eine längere Wirkungsdauer als Zileuton auf; Brooks et al., J. Pharm. Exp. Therapeut 272 - 724, 1995.

Noch eine weitere N-Hydroxyhamstoffverbindung ist Abbott-85761 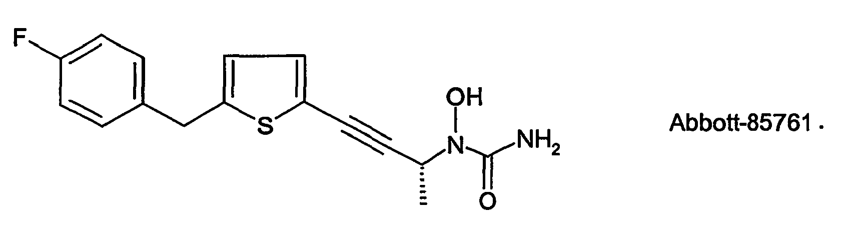 Abbott-85761 wird an die Lunge durch Aerosol-Verabreichung einer homogenen, physikalisch stabilen und beinahe monodispersen Formulierung abgegeben; Gupta et al., "Pulmonary delivery of the 5-lipoxygenase inhibitor, Abbott- 85761, in beagle dogs," International Journal of Pharmaceutics 147, 207-218, 1997.

Zur Bildung von erfindungsgemäßen Ausführungsformen werden Fenleuton, Abbott-79175, Abbott-85761 oder beliebige ihrer oben beschriebenen Derivate oder Tepoxalinderivate mit den Verbindungen der Formel I kombiniert.

Seit der Aufklärung des 5-LO-Biosynthesewegs wird ständig diskutiert, ob es vorteilhafter ist, das Enzym 5-Lipoxygenase zu hemmen oder Antagonisten für die Peptido- oder Nichtpeptidoleukotrienrezeptoren zu verwenden. Man ist der Meinung, daß Inhibitoren der 5-Lipoxygenase den LT-Rezeptorantagonisten überlegen sind, da 5-Lipoxygenaseinhibitoren die Wirkung des gesamten Spektrums der 5-LO-Produkte blockieren, während sich die Wirkung der LT-Antagonisten in einem engeren Spektrum bewegt. Erfindungsgemäße Ausführungsformen beinhalten trotzdem Kombinationen der Verbindungen der Formel 1 nicht nur mit 5-LO-Inhibitoren, sondern auch mit LT-Antagonisten, wie dies unten beschrieben ist. Inhibitoren der 5-Lipoxygenase mit chemischen Strukturen, die sich von den oben beschriebenen Klassen der N-Hydroxyhamstoffe und Hydroxamsäuren unterscheiden, werden ebenfalls mit den Verbindungen der Formel 1 kombiniert und bilden so weitere erfindungsgemäße Ausführungsformen. Ein Beispiel einer derartigen unterschiedlichen Klasse sind die N-(5-substituierten)-Thiophen-2- alkylsulfonamide der Formel 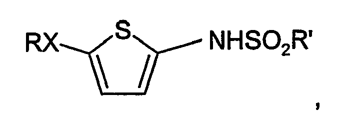 worin X O oder S bedeutet; R' Methyl, Isopropyl, n-Butyl, n-Octyl oder Phenyl bedeutet und R n-Pentyl, Cyclohexyl, Phenyl, Tetrahydro-1-naphthyl, 1- oder 2-Naphthyl oder einfach oder zweifach durch CI, F, Br, CH₃, OCH₃ , SCH₃, SO₂CH₃, CF₃, oder Isopropyl substituiertes Phenyl bedeutet. Eine bevorzugten Verbindung ist

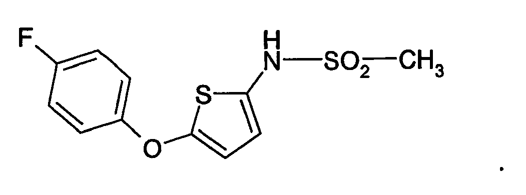

Eine genauere Beschreibung dieser Verbindungen findet sich bei Beers et al., "N-(5-substituted) thiophene-2-alkylsulfonamides as potent inhibitors of 5-lipoxygenase," Bioorganic & Medicinal Chemistry 5(4), 779-786, 1997. Eine weitere unterschiedliche Klasse der 5-Lipoxygenaseinhibitoren ist die bei Cuadro et al., "Synthesis and biological evaluation of 2,6-di-tert.-butylphenol hydrazones as 5-lipoxygenase inhibitors," Bioorganic & Medicinal Chemistry 6, 173-180, 1998 beschriebene Klasse der 2,6-di-tert.-butylphenolhydrazone. Verbindungen dieser Art entsprechen der Formel 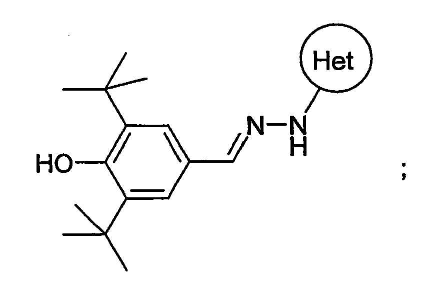 worin "Het" Benzoxazol-2-yl, Benzothiazol-2-yl, Pyridin-2-yl, Pyrazin-2-yl, Pyrimidin-2-yl, 4-Phenylpyrimidin-2-yl, 4,6-Diphenylpyrimidin-2-yl, 4-Methylpyrimidin-2-yl, 4,6-Dimethylpyrimidin-2-yl, 4-Butylpyrimidin-2-yl, 4,6-Dibutylpyrimidin-2-yl und 4-Methyl-6-phenylpyrimidin-2-yl bedeutet.

Die N-(5-substituierten) Thiophen-2-alkylsulfonamide oder die 2,6-Di-tert.-butylphenolhydrazone oder beliebige ihrer oben beschriebenen Derivate werden mit den oben erwähnten Verbindungen der Formel I kombiniert und bilden so erfindungsgemäße Ausführungsformen.

Eine weitere unterschiedliche Klasse von 5-Lipoxygenaseinhibitorenn ist die der Methoxytetrahydropyrane, zu der Zeneca ZD-2138 zählt. 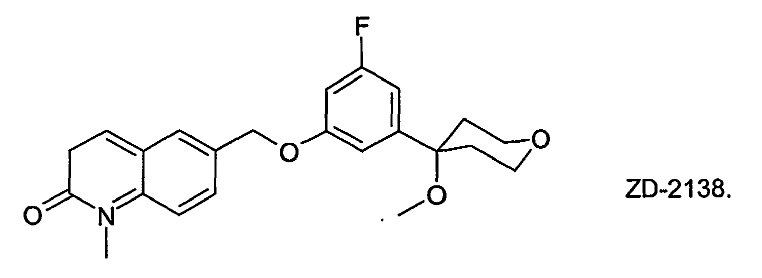 ZD-2138 ist bei oraler Verabreichung in verschiedenen Spezies hoch selektiv und hoch wirksam und wurde bei oraler Verabreichung bei der Behandlung von Asthma und rheumatoider Arthritis ausgewertet. Genaueres über ZD-2138 und seine Derivate findet sich bei Crawley et al., J. Med. Chem., 35, 2600, 1992 und Crawley et al., J. Med. Chem. 36, 295, 1993.

Eine weitere unterschiedliche Klasse von 5-Lipoxygenaseinhibitoren ist diejenige mit der SmithKline Beecham-Verbindung SB-210661

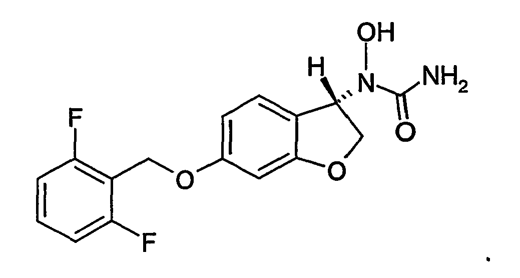

Zwei weitere unterschiedliche verwandte Klassen von 5-Lipoxygenase-inhibitoren umfassen verschiedene pyridinyl-substituierte 2-Cyannaphthalinverbindungen sowie verschiedene 2-Cyanchinolinverbindungen, die von Merck Frosst entdeckt wurden. Diese beiden Klassen von 5-Lipoxygenaseinhibitoren werden durch L-739,010 bzw. L-746,530 veranschaulicht: 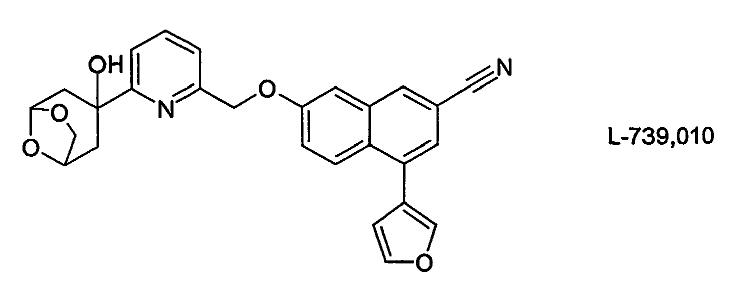 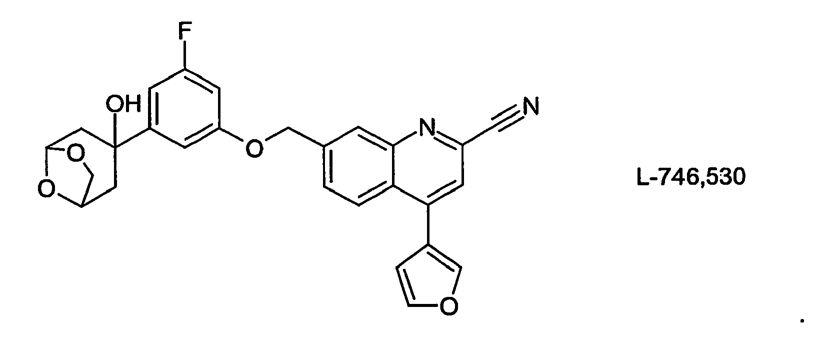 Genaueres über L-739,010 und L-746,530 findet sich bei Dubé et al., "Quinolines as potent 5-lipoxygenase inhibitors: synthesis and biological profile of L-746,530," Bioorganic & Medicinal Chemistry 8, 1255-1260, 1998 sowie in WO 95/03309 (Friesen et al.).

Die Klasse der Methoxytetrahydropyrane, darunter Zeneca ZD-2138, oder die Leitverbindung SB-210661 und die Klasse, zu der sie gehört, oder die Reihe der pyridinyl-substituierten 2-Cyannaphthalinverbindungen, darunter L-739,010, oder die Reihe der 2-Cyanchinolinverbindungen, darunter L-746,530, oder beliebige der oben beschriebenen Derivate von beliebigen der oben genannten Klassen werden mit den Verbindungen der Formel I kombiniert und bilden so erfindungsgemäße Ausführungsformen.

Die andere endogene Substanz, die außer dem Enzym 5-Lipoxygenase eine wesentliche Rolle bei der Leukotrien-Biosynthese spielt, ist das 5-Lipoxygenase-aktivierende Protein (FLAP). Im Gegensatz zur direkten Rolle des Enzyms 5-Lipoxygenase kommt diesem Protein eine indirekte Rolle zu. Trotzdem verwendet man Antagonisten des 5-Lipoxygenase-aktivierenden Proteins zur Hemmung der Leukotriensynthese in der Zelle und als solche werden auch sie in Kombination mit den Verbindungen der Formel I verwendet und bilden so erfindungsgemäße Ausführungsformen.

Verbindungen, die an das 5-Lipoxygenase-aktivierende Protein binden und so die Verwertung des endogenen Arachidonsäure-Pools, der vorliegt, blockieren, wurden aus Indol- und Chinolinstrukturen dargestellt; siehe Ford-Hutchinson et al., ibid., Rouzer et al. "WK-886, a potent and specific leukotriene biosynthesis inhibitor blocks and reverses the membrane association of 5-lipoxygenase in ionophore-challenged leukocytes," J. Biol. Chem. 265, 1436- 42, 1990 und Gorenne et al., "{(R)-2-quinolin-2-yl-methoxy)phenyl)-2-cyclopentyl acetic acid} (BAY x1005), a potent leukotriene synthesis inhibitor: effects on anti-IgE challenge in human airways," J. Pharmacol. Exp. Ther. 268, 868-72, 1994.

MK-591, mit der Bezeichnung Quiflipon-Natrium, gehorcht der Formel

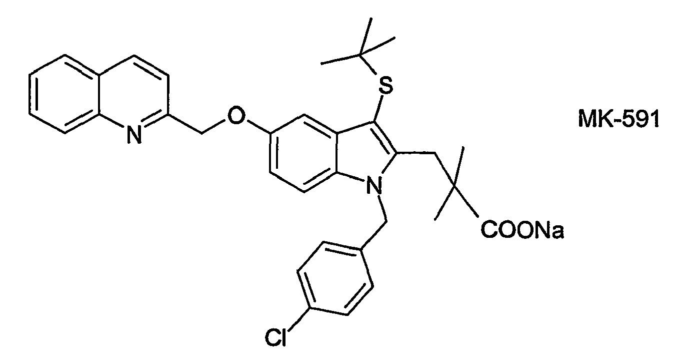

Die oben genannten Verbindungsklassen der Indole und Chinoline, darunter die Einzelverbindungen MK-591, MK-886 und BAY x 1005 oder beliebige der oben beschriebenen Derivate von beliebigen der oben genannten Klassen werden mit den Verbindungen der Formel I kombiniert und bilden so erfindungsgemäße Ausführungsformen.

### Kombinationen mit Rezeptorantaaonisten für die Leukotriene LTB₄, LTC₄, LTD₄ und LTE₄

Eine Verbindung der Formel bzw. mehrere Verbindungen der Formel 1 wird bzw. werden in Kombination mit Rezeptorantagonisten für die Leukotriene LTB₄, LTC₄, LTD₄ und LTE₄ verwendet. Die wichtigsten dieser Leukotriene in bezug auf die Vermittlung einer Entzündungsreaktion sind LTB₄ und LTD₄. Klassen von Antagonisten für die Rezeptoren dieser Leukotriene sind in den folgenden Absätzen beschrieben.

4-Brom-2,7-dimethoxy-3H-phenothiazin-3-one, darunter L-651,392, sind wirksame LTB₄-Antagonisten, die in US 4,939,145 (Guindon et al.) und US 4,845,083 (Lau et al.) beschrieben sind

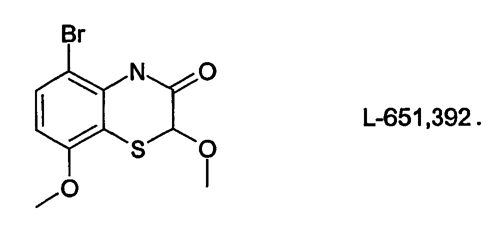

Eine Klasse von Amidinoverbindungen, zu der CGS-25019c zählt, ist in US 5,451,700 (Morrissey und Suh); US 5,488,160 (Morrissey) und US 5,639,768 (Morrissey und Suh) beschrieben. Ein typischer Vertreter dieser LTB₄-Antagonisten ist das im folgenden dargestellte CGS-25019c:

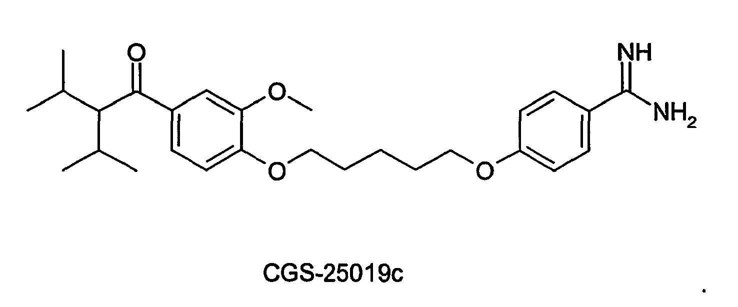

Ontazolast, ein Mitglied einer Klasse von Benzoxazolaminen, die LTB₄-Antagonisten sind, ist in der EP 535 521 (Anderskewitz et al.) beschrieben:

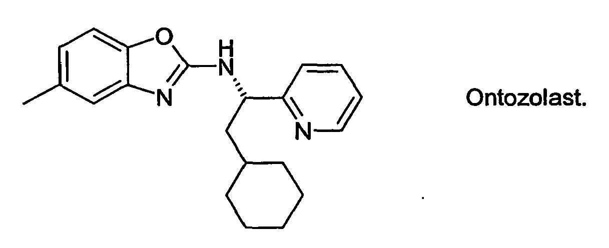

Die gleiche Arbeitsgruppe entdeckte auch eine Klasse von Benzolcarboximidamiden, die LTB₄-Antagonisten darstellen, die in der WO 97/21670 (Anderskewitz et al.) und WO 98/11119 (Anderskewitz et al.) beschrieben sind und von denen BIIL 284/260 ein typischer Vertreter ist:

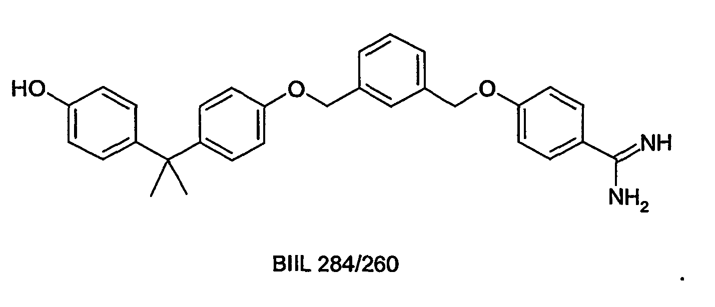

Zafirlukast ist ein LTC₄-, LTD₄- und LTE₄-Rezeptorantagonist, der unter der Bezeichnung Accolate^{®} im Handel erhältlich ist. Er gehört zu einer Klasse von heterocyclischen Amidderivaten, die in der US 4,859,692 (Bernstein et al.), US 5,319,097 (Holohan und Edwards), US 5,294,636 (Edwards und Sherwood), US 5,482,963; US 5,583,152 (Bernstein et al.) und US 5,612,367 (Timko et al.) beschrieben ist:

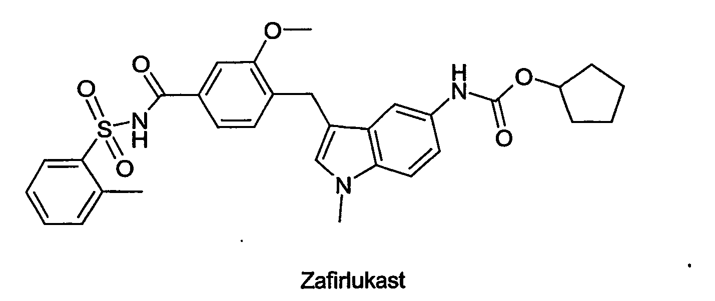

Ablukast ist ein LTD₄-Rezeptorantagonist, der die Bezeichnung Ro 23-3544/001 trägt:

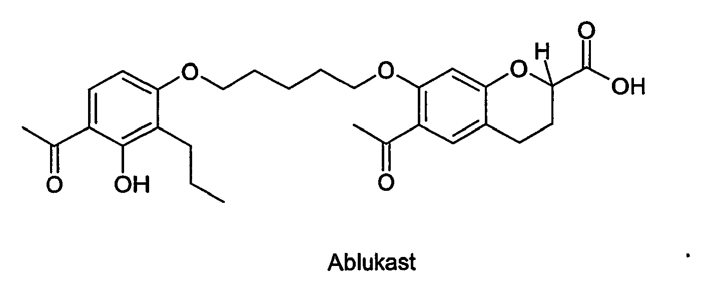

Montelukast ist ein LTD₄-Rezeptorantagonist, der unter der Bezeichnung Singulair^{®} im Handel erhältlich und in der US 5,565,473 beschrieben ist:

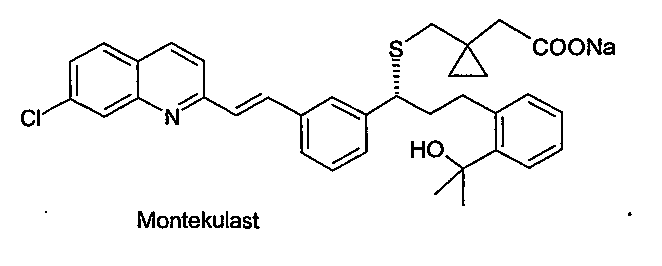

Zu weiteren LTD₄-Rezeptorantagonisten zählen Pranlukast, Verlukast (MK-679), RG-12525, Ro-245913, Iralukast (CGP 45715A) und BAY x 7195.

Die oben genannte Verbindungsklasse der Phenothiazin-3-one, darunter L-651,392, die Klasse der Amidinoverbindungen, zu denen CGS-25019c zählt, die Klasse der Benzoxazolamine mit Ontazolast; die Klasse der Benzolcarboximidamide, von denen BIIL 284/260 ein typischer Vertreter ist; die heterocyclischen Amidderivate mit Zafirlukast, Ablukast und Montelukast und die Verbindungsklassen, zu denen sie zählen, oder beliebige der oben beschriebenen Derivate von beliebigen der oben genannten Klassen werden mit den Verbindungen der Formel I kombiniert und bilden so erfindungsgemäße Ausführungsformen.

### Kombinationen mit anderen Therapeutika

Eine oder mehrere Verbindungen der Formel I werden zusammen mit anderen Therapeutika sowie Nichttherapeutika verwendet und es werden so Kombinationen gebildet, die weitere erfindungsgemäße Ausführungsformen darstellen und die sich zur Behandlung einer ganzen Reihe von unterschiedlichen, hierin beschriebenen Erkrankungen, krankhaften Störungen und Leiden eignen. Diese Ausführungsformen umfassen eine oder mehrere Verbindungen der Formel I zusammen mit einer oder mehreren der folgenden Substanzen:
(a) PDE IV-Inhibitoren;
(b) 5-Lipoxygenase (5-LO)-Inhibitoren oder Antagonisten des 5-lipoxygenase-aktivierenden Proteins (FLAP);
(c) Doppelinhibitoren der 5-Lipoxygenase (5-LO) und Antagonisten des blutplättchenaktivierenden Faktors (PAF);
(d) Leukotrienantagonisten (LTRAs), darunter LTB₄-, LTC4-; LTD₄- und LTE₄-Antagonisten;
(e) Antihistamin-H₁-Rezeptorantagonisten, darunter Cetirizin, loratadin, Desloratadin, Fexofenadin, Astemizol, Azelastin, und Chlorpheniramin;
(f) gastroprotektive H₂-Rezeptorantagonisten;
(g) oral oder topisch verabreichte α₁- und α₂-Adrenorezeptor-Agonist-Vasokonstriktor-Sympathomimetika zur Schleimhautabschwellung, darunter Propylhexedrin, Phenylephrin, Phenylpropanolamin, Pseudoephedrin, Naphazolinhydrochlorid, Oxymetazolinhydrochlorid, Tetrahydrozolinhydrochlorid, Xylometazolinhydrochlorid sowie Ethylnorepinephrinhydrochlorid;
(h) α₁- und α₂-Adrenorezeptoragonisten in Kombination mit Inhibitoren der 5-Lipoxygenase (5-LO);
(i) Anticholinergika, darunter Ipratropiumbromid, Tiotropiumbromid, Oxitropiumbromid, Pirenzepin sowie Telenzepin;
(j) β₁- bis β₄-Adrenorezeptoragonisten, darunter Metaproterenol, Isoproterenol, Isoprenalin, Albuterol, Salbutamol, Formoterol, Salmeterol, Terbutalin, Orciprenalin, Bitolterolmesylat und Pirbuterol;
(k) Theophyllin und Aminophyllin;
(l) Natriumcromoglycat;
(m) Muscarinreeptor (M1, M2 und M3)-Aantagonisten;
(n) COX-1-Inhibitoren (NSAIDs); COX-2-selektive Inhibitoren, darunter Rofecoxib, sowie Stickoxid-NSAIDs;
(o) Mimetika des insulinähnlichen Wachstumsfaktors Typ I (IGF-1);
(p) Ciclesonid;
(q) Inhalations-Glucokortikoide mit verringerten systemischen Nebenwirkungen, darunter Prednison, Prednisolon, Flunisolid, Triamcinolonacetonid, Beclomethasondipropionat, Budesonid, Fluticasonpropionat sowie Mometasonfuroat;
(r) Tryptaseinhibitoren;
(s) Antagonisten des blutplättchenaktivierenden Faktors (PAF);
(t) monoklonale Antikörper gegen endogene entzündliche Körper;
(u) IPL 576;
(v) Anti-Tumor Nekrose Faktor (TNFα)-Mittei, darunter Etanercept, Infliximab und D2E7;
(w) DMARDs, darunter Leflunomid;
(x) TCR-Peptide;
(y) Inhibitoren des interleukinumwandelnden Enzyms (interleukin converting enzyme, ICE);
(z) IMPDH-Inhibitoren;
(aa) Adhäsionsmolekülinhibitoren, darunter VLA-4-Antagonisten;
(bb) Kathepsine;
(cc) MAP-Kinaseinhibitoren;
(dd) Glucose-6-phosphat-dehydrogenase-Inhibitoren;
(ee) Kinin-B₁- und B₂-Rezeptor-Antagonisten;
(ff) Gold in Form einer Aurothiogruppe zusammen mit verschiedenen hydrophilen Gruppen;
(gg) lmmunosuppressiva, z.B. Cyclosporin, Azathioprin und Methotrexat;
(hh) Mittel gegen Gicht, z.B. Kolchizin;
(ii) Xanthinoxidaseinhibitoren z.B. Allopurinol;
(jj) Urikosurika z.B. Probenecid, Sulfinpyrazon und Benzbromaron;
(kk) Antineoplastica, insbesondere antimitotische Arzneistoffe, darunter die Vinca-Alkaloide wie Vinblastin und Vincristin;
(ll) Mittel zur Förderung der Wachstumshormonsekretion;
(mm) Inhibitoren der Matrixmetalloproteasen (MMPs), d.h. die Stromelysine, Kollagenasen und Gelatinasen sowie Aggrecanase, insbesondere Kollagenase-1 (MMP-1), Kollagenase-2 (MMP-8), Kollagenase-3 (MMP-13), Stromelysin-1 (MMP-3), Stromelysin-2 (MMP-10) und Stromelysin-3 (MMP-11);
(nn) "transforming growth factor" (TGFβ);
(oo) "platelet-derived growth factor" (PDGF);
(pp) Fibroblasten-Wachstumsfaktor, z.B. "basic fibroblast growth factor" (bFGF);
(qq) "granulocyte macrophage colony stimulating factor" (GM-CSF);
(rr) Capsaicin;
(ss) Tachykinin-NK₁- und -NK₃-Rezeptor-Antagonisten aus der Gruppe NKP-608C; SB233412 (Talnetant) und D-4418;
(tt) Elastaseinhibitoren aus der Gruppe UT-77 und ZD-0892, sowie
(uu) Adenosin-A2a-Rezeptoragonisten.

### Pharmazeutische Zusammensetzungen und Formulierungen

Die folgende Beschreibung betrifft die Art und Weise, auf die die Verbindungen der Formel I, gewünschtenfalls zusammen mit anderen Therapeutika oder Nichttherpeutika, mit überwiegend üblichen pharmazeutisch unbedenklichen Trägem kombiniert werden, wodurch man zu Dosierungsformen gelangt, die sich für die unterschiedlichen Verabreichungswege, die für einen gegebenen Patienten verwendet werden, und die Erkrankung, krankhafte Störung oder das Leiden, für die bzw. das ein gegebener Patient behandelt wird, eignen.

Die erfindungsgemäßen pharmazeutischen Zusammensetzungen umfassen eine oder mehrere beliebige der oben beschriebenen erfindungsgemäßen Inhibitorverbindungen oder eines ihrer pharmazeutisch unbedenklichen Salze wie ebenfalls oben beschrieben zusammen mit einem pharmazeutisch unbedenklichen Träger gemäß den Eigenschaften und dem erwarteten Verhalten solcher Träger, die dem Fachmann gut bekannt sind.

Die Menge an Wirkstoff, die mit den Trägermaterialien kombiniert werden kann, um so eine einzelne Dosierungsform zu bilden, hängt von dem behandelten Patienten und dem jeweiligen Verabreichungsweg ab. Es ist jedoch klar, daß ein bestimmtes Dosierungs- und Behandlungsschema für einen bestimmten Patienten von verschiedensten Faktoren, darunter der Wirksamkeit der jeweils verwendeten Verbindung, dem Alter, Körpergewicht, allgemeinem Gesundheitszustand, Geschlecht, der Ernährung, dem Verabreichungszeitpunkt, der Ausscheidungsgeschwindigkeit, der Arzneistoffkombination und dem Ermessen des behandelnden Arztes sowie der Schwere der jeweils behandelten Krankheit abhängt. Die Wirkstoffmenge kann auch von dem Therapeutikum oder Prophylaktikum, das gegebenenfalls mit dem Wirkstoff gemeinsam verabreicht wird, abhängen.

Die Verbindungen der Formel I lassen sich in der Form von Säuren, Estern oder anderen chemischen Verbindungsklassen, zu denen die beschriebenen Verbindungen zählen, verwenden. Die vorliegende Erfindung umfaßt auch die Verwendung dieser Verbindungen in der Form von pharmazeutisch unbedenklichen Salzen, die sich von verschiedenen organischen und anorganischen Säuren und Basen ableiten. Ein Wirkstoff mit einer bevorzugten Verbindung wird häufig in der Form eines ihrer Salze verwendet, insbesondere wenn diese Salzform dem Wirkstoff im Vergleich zu der freien Form des Wirkstoffs oder einer zuvor verwendeten anderen Salzform des Wirkstoffs verbesserte pharmakokinetische Eigenschaften verleiht. Die pharmazeutisch unbedenkliche Salzform des Wirkstoffs kann auch diesem Wirkstoff erst eine erwünschte pharmakokinetische Eigenschaft verleihen, über die er früher nicht verfügt hat, und kann sogar die Pharmakodynamik dieses Wirkstoffs in bezug auf seine therapeutische Wirksamkeit im Körper positiv beeinflussen.

Zu den pharmakokinetischen Eigenschaften des Wirkstoffs, die günstig beeinflußt werden können, zählen z.B. die Art, wie dieser Wirkstoff durch Zellmembranen hindurch transportiert wird, was wiederum die Absorption, Verteilung, biologische Umwandlung und Exkretion dieses Wirkstoffs direkt und positiv beeinflussen kann. Obwohl der Verabreichungsweg der pharmazeutischen Zusammensetzung wichtig ist und verschiedene anatomische, physiologische und pathologische Aspekte die biologische Verfügbarkeit entscheidend beeinflussen können, hängt die Löslichkeit des Wirkstoffs üblicherweise von der Art seiner jeweiligen Salzform, die verwendet wird, ab. Weiterhin ist dem Fachmann deutlich, daß eine wäßrige Lösung des Wirkstoffs für die rascheste Absorption des Wirkstoffs in den Körper eines behandelten Patienten sorgt, während Lipidlösungen und -suspensionen sowie feste Dosierungsformen zu einer weniger raschen Absorption des Wirkstoffs führen. Die orale Aufnahme eines Wirkstoffs der Formel I stellt aus Sicherheits-, Bequemlichkeits- und Sparsamkeitsgründen den am stärksten bevorzugten Verabreichungsweg dar, die Absorption einer derartigen oralen Dosierungsform kann jedoch durch physikalische Eigenschaften wie Polarität, durch Reizung der Magen-Darm-Schleimhaut hervorgerufenes Erbrechen, Abbau durch Verdauungsenzyme und niedrigen pH, ungleichmäßige Absorption oder Propulsion in Gegenwart von Nahrungsmitteln oder anderen Arzneistoffen sowie Stoffwechsel durch Enzyme der Schleimhaut, der Darmflora oder der Leber gestört werden. Die Formulierung des Wirkstoffs als unterschiedliche pharmazeutisch unbedenkliche Salzformen kann zur Überwindung oder Verringerung eines oder mehrerer der oben genannten Probleme im Zusammenhang mit der Absorption oraler Dosierungsformen wirksam sein.

Zu den oben genannten pharmazeutischen Salzen, die bevorzugt sind, zählen Azetat, Besylat, Citrat, Fumarat, Gluconat, Hemisuccinat, Hippurat, Hydrochlorid, Hydrobromid, Isethionat, Mandelat, Meglumin, Nitrat, Oleat, Phosphonat, Pivalat, Natriumphosphat, Stearat, Sulfat, Sulfosalicylat, Tartrat, Thiomalat, Tosylat und Tromethamin, was jedoch keine Einschränkung darstellen soll.

Enthält eine Verbindung der Formel I mehr als eine Gruppe, die solche pharmazeutisch unbedenklichen Salze bilden kann, so umfaßt die Erfindung auch mehrfache Salze. Zu typischen mehrfachen Salzformen zählen zum Beispiel Bitartrat, Diacetat, Difumarat, Dimeglumin, Diphosphat, Dinatrium und Trihydrochlorid, was jedoch keine Einschränkung darstellen soll.

Die erfindungsgemäßen pharmazeutischen Zusammensetzungen umfassen eine oder mehrere der oben beschriebenen Inhibitorverbindungen oder eines ihrer ebenfalls wie oben beschriebenen pharmazeutisch unbedenklichen Salze zusammen mit einem pharmazeutisch unbedenklichen Träger entsprechend den Eigenschaften und dem erwarteten Verhalten solcher dem Fachmann gut bekannten Träger.

Der Begriff "Träger" umfaßt im vorliegenden Zusammenhang unbedenkliche Streckmittel, Exzipientien, Hilfsstoffe, Konstituentien, Lösungsvermittler, viskositätsmodifizierende Mittel, Konservierungsmittel und andere Mittel, die dem Fachmann gut bekannt sind, um der endgültigen pharmazeutischen Zusammensetzung günstige Eigenschaften zu verleihen. Zur Veranschaulichung dieser Träger folgt nun eine kurze Übersicht pharmazeutisch unbedenklicher Träger, die sich bei den erfindungsgemäßen pharmazeutischen Zusammensetzungen verwenden lassen, und im Anschluß daran eine genauere Beschreibung der unterschiedlichen Arten von Bestandteilen. Zu typischen Trägern zählen die folgenden, was jedoch keinesfalls eine Einschränkung darstellen soll: lonenaustauscherzusammensetzungen, Aluminiumoxid, Aluminiumstearat, Lezitin, Serumproteine, z.B. Humanserumalbumin, Phosphate, Glycin, Sorbinsäure, Kaliumsorbat, Partialglyceridmischungen gesättigter Pflanzenfettsäuren, hydrierte Palmöle, Wasser, Salze oder Elektrolyte, z.B. Prolaminsulfat, Dinatriumhydrogenphosphat, Kaliumhydrogenphosphat, Natriumchlorid und Zinksalze, kolloidale Silica, Magnesiumtrisilicat, Polyvinylpyrrolidon, Substanzen auf Zellulosegrundlage, z.B. Natriumcarboxymethylcellulose, Polyethylenglycol, Polyacrylate, Wachse, Polyethylen-Polyoxypropylen-Blockpolymere sowie Wollfett.

Insbesondere umfaßt die bei den erfindungsgemäßen pharmazeutischen Zusammensetzungen verwendeten Träger unterschiedliche Klassen und Arten von Zusatzstoffen, die unabhängig aus den im wesentlichen in den folgenden Absätzen genannten Gruppen ausgewählt werden.

Ansäuernde und alkalisierende Mittel werden zugesetzt, um zu einem gewünschten oder vorbestimmten pH zu gelangen; sie umfassen Säuerungsmittel, z.B. Essigsäure, Eisessig, Äpfelsäure und Propionsäure. Stärkere Säuren wie Salzsäure, Salpetersäure und Schwefelsäure können verwendet werden, sind jedoch weniger bevorzugt. Zu den alkalisierenden Mitteln zählen zum Beispiel Edetol, Kaliumcarbonat, Kaliumhydroxid, Natriumborat, Natriumcarbonat und Natriumhydroxid. Alkalisierende Mittel, die aktive Aminogruppen enthalten, wie Diethanolamin und Trolamin, können ebenfalls verwendet werden.

Soll die pharmazeutische Zusammensetzung als Aerosol unter beträchtlichem Druck abgegeben werden, so sind Aerosoltreibmittel erforderlich. Zu diesen Treibmittein zählen zum Beispiel unbedenkliche Fluorchlorkohlenwasserstoffe wie Dichlordifluormethan, Dichlortetrafluorethan und Trichlormonofluormethan, Stickstoff, ein flüchtiger Kohlenwasserstoff wie Butan, Propan oder Isobutan, oder deren Mischungen.

Antimikrobielle Mittel, darunter Mittel gegen Bakterien, Pilze und Protozoen, werden zugegeben, wenn die pharmazeutische Zusammensetzung topisch auf Hautflächen aufgetragen wird, die wahrscheinlich einer schädigenden Umgebung ausgesetzt waren, oder Abschürfungen oder Schnitte erlitten haben, die die Haut für eine Infektion durch Bakterien, Pilzen oder Protozoen anfällig macht. Zu den antimikrobiellen Mitteln zählen Verbindungen wie Benzylalkohol, Chlorbutanol, Phenylethylalkohol, Phenylquecksilberacetat, Kaliumsorbat und Sorbinsäure. Zu den antifungalen Mitteln zählen Verbindungen wie Benzoesäure, Butylparaben, Ethylparaben, Methylparaben, Propylparaben und Natriumbenzoat. Antimikrobielle Konservierungsstoffe werden den erfindungsgemäßen pharmazeutischen Zusammensetzungen zugegeben, um diese gegen das Wachstum von möglicherweise schädlichen Mikroorganismen zu schützen, die üblicherweise in die wäßrige Phase einwandern, in manchen Fällen jedoch auch in der Ölphase einer Zusammensetzung wachsen können. Es sind daher Konservierungsstoffe erwünscht, die sowohl in wäßrigen Medien als auch in Lipiden löslich sind. Zu geeigneten antimikrobiellen Konservierungsstoffen zählen z.B. p-Hydroxybenzoesäurealkylester, Propionatsalze, Phenoxyethanol, Methylparaben-Natrium, Propylparaben-Natrium, Natriumdehydroacetat, Benzalkoniumchlorid, Benzethoniumchlorid, Benzylalkohol, Hydantoinderivate, quartäre Ammoniumverbindungen und kationische Polymere, Imidazolidinylharnstoff, Diazolidinylhamstoff und Trinatriumethylendiamintetraacetat (EDTA).
Konservierungsstoffe werden vorzugsweise in Mengen von ungefähr 0,01 Gew.-% bis ungefähr 2,0 Gew.-% der Gesamtzusammensetzung eingesetzt.

Antioxidantien werden zugesetzt, um alle Bestandteile der pharmazeutischen Zusammensetzung gegen Schädigung oder Abbau durch Oxidationsmittel, die in der Zusammensetzung selbst oder in der Umgebung, in der sie verwendet werden, vorliegen, zu schützen, z.B. Anoxomer, Ascorbylpalmitat, Butylhydroxyanisol, Butylhydroxytoluol, hypophosphorige Säure, Kaliummetabisulfit, Gallensäurepropyl-, -octyl- und -dodecylester, Natriummetabisulfit, Schwefeldioxid und Tocopherole.

Puffersubstanzen werden verwendet, um einen erwünschten pH-Wert einer Zusammensetzung nach der Einstellung gegenüber den Auswirkungen externer Einflüsse und Gleichgewichtsverschiebungen von Bestandteilen der Zusammensetzungen aufrechtzuerhalten. Die Puffersubstanz kann unter den dem Fachmann auf dem Gebiet der Galenik bekannten ausgewählt werden, z.B. Calciumacetat, Kaliummetaphosphat, Kaliumdihydrogenphosphat und Weinsäure.

Cheliermittel dienen zur Erhaltung der Ionenstärke der pharmazeutischen Zusammensetzung; sie binden an schädigende Verbindungen und Metalle und entfernen diese dadurch wirksam. Dazu zählen z.B. Dikaliumedetat, Dinatriumedetat und EDTA.

Dermatologische Wirkstoffe werden den erfindungsgemäßen pharmazeutischen Zusammensetzungen dort zugegeben, wo diese topisch anzuwenden sind; dazu zählen z.B. Wundheilungsmittel wie Peptidderivate, Hefe, Panthenol, Hexylresorcin, Phenol, Tetracyclinhydrochlorid, Lamin und Kinetin; Retinoide zur Behandlung von Hautkrebs, z.B. Retinol, Tretinoin, Isotretinoin, Etretinat, Acitretin und Arotinoid, milde antibakterielle Mittel zur Behandlung von Hautinfektionen, z.B. Resorcin, Salicylsäure, Benzoylperoxid, Erythromycin-Benzoylperoxid, Erythromycin und Clindamycin; antifungale Mittel zur Behandlung von Tinea corporis, Tinea pedis, Candida-Infektionen und Tinea versicolor, z.B. Griseofulvin, Azole wie Miconazol, Econazol, Itraconazol, Fluconazol und Ketoconazol, sowie Allylamine wie Naftifin und Terfinafin; antivirale Mittel zur Behandlung von Herpes simplex der Haut, Gürtelrose und Windpocken, z.B. Acyclovir, Famciclovir und Valacyclovir, Antihistamine zur Behandlung von Juckreiz, atopischer Dermatitis und Kontaktdermatitis, z.B. Diphenhydramin, Terfenadin, Astemizol, Loratadin, Cetirizin, Acrivastin und Temelastin, Lokalanästhetika zur Linderung von Schmerzen, Reizung und Jucken, z.B. Benzocain, Lidocain, Dibucain und Pramoxinhydrochlorid, Lokalanalgetika zur Linderung von Schmerzen und Entzündungen, z.B. Salicylsäuremethylester, Kampfer, Menthol und Resorcin, topische Antiseptika zur Verhinderung von Infektionen, z.B. Benzalkoniumchlorid und Povidon-Iod, sowie Vitamine und ihre Derivate, wie Tocopherol, Tocopherolacetat, Retinsäure und Retinol.

Dispergier- und Suspendiermittel werden als Hilfsstoffe bei der Herstellung stabiler Formulierungen eingesetzt, dazu zählen z.B. Poligeenan, Povidon und Siliziumdioxid.

Emollientia sind vorzugsweise nichtölige, wasserlösliche Stoffe, die die Haut erweichen und beruhigen, insbesondere Haut, die durch übermäßigen Wasserverlust trocken geworden ist. Solche Stoffe werden bei erfindungsgemäßen pharmazeutischen Zusammensetzungen verwendet, die zur topischen Anwendung bestimmt sind; dazu zählen z.B. Kohlenwasserstofföle und -wachse, Triglyceridester, acetylierte Monoglyceride, Methylester und andere Alkylester von C₁₀-C₂₀-Fettsäuren, C₁₀-C₂₀-Fettsäuren, C₁₀-C₂₀-Fettalkohole, Lanolin und Derivate, Ester von mehrwertigen Alkoholen, wie Polyethylenglykol (200-600), Polyoxyethylensorbitanfettsäureester, Wachsester, Phospholipide und Sterole; Emulgatoren zur Herstellung von ÖI-in-Wasser-Emulsionen; Exzipientien, z.B. Laurocapram und Polyethylenglykolmonomethylether, Feuchthaltemittel, z.B. Sorbit, Glycerin und Hyaluronsäure, Salbengrundlagen, z.B. Vaselin, Polyethylenglykol, Lanolin und Poloxamer, Penetrationsförderer, z.B. Dimethylisosorbid, Diethylglykolmonoethylether, 1-Dodecylazacycloheptan-2-on und Dimethylsulfoxid (DMSO), Konservierungsstoffe, z.B. Benzalkoniumchlorid, Benzethoniumchlorid, p-Hydroxybenzoesäurealkylester, Hydantoinderivate, Cetylpyridiniumchlorid, Propylparaben, Quartärammoniumverbindungen, wie Kaliumbenzoat sowie Thimerosal; Sequestriermittel, darunter Cyclodextrine, Lösungsmittel, z.B. Aceton, Alkohol, Amylenhydrat, Butylalkohol, Maiskeimöl, Baumwollsamenöl, Essigester, Glycerin, Hexylenglykol, Isopropylalkohol, Isostearylalkohol, Methylalkohol, Methylenchlorid, Mineralöl, Erdnußöl, Phosphorsäure, Polyethylenglykol, Polyoxypropylen-15-stearylether, Propylenglykol, Propylenglykoldiacetat, Sesamöl sowie gereinigtes Wasser, Stabilisatoren, z.B. Calciumsaccharat und Thymol, Tenside, z.B. Lapyriumchlorid; Laureth-4, d.h. α-Dodecyl-ω-hydroxy-poly(oxy-1,2-ethandiyl)- oder Polyethylenglykolmonododecylether.

Emulgatoren, darunter emulgierende und verdickende Mittel und Emulsionshilfsstoffe, werden zur Herstellung von Öl-in-Wasser-Emulsionen verwendet, wenn diese die Grundlage der erfindungsgemäßen pharmazeutischen Zusammensetzungen bilden. Zu diesen Emulgatoren zählen z.B. nichtionogene Emulgatoren, wie C₁₀-C₂₀-Fettalkohole und die Kondensationsprodukte dieser Fettalkohole mit 2 bis 20 Mol Ethylenoxid oder Propylenoxid, das Kondensationsprodukt von (C₆-C₁₂)Alkylphenolen und 2 bis 20 Mol Ethylenoxid, Ethylenglykolmono- und -di-C₁₀-C₂₀-Fettsäureester, C₁₀-C₂₀-Fettsäuremonoglycerid, Diethylenglykol, Polyethylenglykole mit einem MG von 200-6000, Polypropylenglykole mit einem MG von 200-3000 und insbesondere Sorbit, Sorbitan, Polyoxyethylensorbit, Polyoxyethylensorbitan, hydrophile Wachsester, Cetostearylalkohol, Oleylalkohol, Lanolinalkohole, Cholesterin, Mono- und Diglyceride, Glycerylmonostearat, Polyethylenglykolmonostearat, Ethylenglykol- und Ppolyoxyethylenglykol-mono- und - distearinsäuremischester, Propylenglykolmonostearat, sowie Hydroxypropylcellulose. Emulgatoren mit aktiven Aminogruppen können ebenfalls verwendet werden; dazu zählen typischerweise anionenaktive Emulgatoren wie Fettsäureseifen, z.B. Natrium-, Kalium- und Triethanolaminseifen der C₁₀-C₂₀-Fettsäuren, Alkalimetall-, Ammonium- oder substituierte Ammoniumsalze von (C₁₀-C₃₀)Alkylsulfat, (C₁₀-C₃₀)Alkylsulfonate und (C₁₀-C₅₀)alkylethoxyethersulfonate. Zu weiteren geeigneten Emulgatoren zählen Rizinusöl und hydriertes Rizinusöl, Lecithin; sowie Polymere der 2-Propensäure zusammen mit Acrylsäurepolymeren, die beide mit Saccharose- und/oder Pentaerythrit-Allylethern vernetzt sind und unterschiedliche Viskositäten aufweisen, diese sind durch die Produktbezeichnungen Carbomer 910, 934, 934P, 940, 941 und 1342 gekennzeichnet. Kationenaktive Emulgatoren mit aktiven Aminogruppen können ebenfalls verwendet werden, darunter diejenigen auf Grundlage der quarteren Ammonium-, Morpholinium- und Pyridiniumverbindungen. Ähnlich können amphotere Emulgatoren mit aktiven Aminogruppen wie Cocobetaine, Lauryldimethylaminoxid und Cocoylimidazolin verwendet werden. Zu den Emulgatoren und Verdickungsmitteln, die verwendet werden können, zählen auch Cetylalkohol und Natriumstearat sowie Emulsionshilfstoffe wie Ölsäure, Stearinsäure und Stearylalkohol.

Zu den Exzipientien zählen z.B. Laurocapram und Polyethylenglykolmonomethylether.
Soll die erfindungsgemäße pharmazeutische Zusammensetzung topisch angewndet werden, so können Penetrationsförderer verwendet werden, darunter z.B. Dimethylisosorbid, Diethylglykolmonoethylether, 1-Dodecylazacycloheptan-2-on und Dimethylsulfoxid (DMSO). Solche Zusammensetzungen enthalten typischerweise auch Salbengrundlagen, z.B. Vaselin, Polyethylenglykol, Lanolin und Poloxamer, bei dem es sich um ein Polyoxyethylen-Polyoxypropylen-Blockcopolymer handelt, das auch als Tensid oder Emulgator dienen kann.

Konservierungsstoffe werden verwendet, um erfindungsgemäße pharmazeutische Zusammensetzungen gegen Abbau durch Mikroorganismen der Umgebung zu schützen, dazu zählen z.B. Benzalkoniumchlorid, Benzethoniumchlorid, p-Hydroxybenzoesäurealkylester, Hydantoinderivate, Cetylpyridiniumchlorid, Monothioglycerin, Phenol, Phenoxyethanol, Methylparaben, Imidazolidinylharnstoff, Natriumdehydroacetat, Propylparaben, quartäre Ammoniumverbindungen, insbesondere Polymere wie Polixetoniumchlorid, Kaliumbenzoat, Natriumformaldehydsulfoxylat, Natriumpropionat sowie Thimerosal.

Sequestriermittel werden verwendet, um die Stabilität der erfindungsgemäßen pharmazeutischen Zusammensetzung zu verbessern; dazu zählen z.B. die Cyclodextrine, bei denen es um eine Familie natürlicher cyclischer Oligosaccharide handelt, die mit unterschiedlichen Stoffen Einschlußkomplexe bilden können und unterschiedliche Ringgrößen aufweisen, wobei man diejenigen mit 6, 7 und 8 Glucoseresten pro Ring üblicherweise als α-Cyclodextrine, β-Cyclodextrine bzw. γ-Cyclodextrine bezeichnet. Zu geeigneten Cyclodextrinen zählen z.B. α-Cyclodextrin, β-Cyclodextrin, γ-Cyclodextrin, γ-Cyclodextrin sowie kationisierte Cyclodextrine.

Zu den Lösungsmitteln, die bei der Herstellung der erfindungsgemäßen pharmazeutischen Zusammensetzungen verwendet werden können, zählen z.B. Aceton, Alkohol, Amylenhydrat, Butylalkohol, Maiskeimöl, Baumwollsamenöl, Essigester, Glycerin, Hexylenglykol, Isopropylalkohol, lsostearylalkohol, Methylalkohol, Methylenchlorid, Mineralöl, Erdnußöl, Phosphorsäure, Polyethylenglykol, Polyoxypropylen-15-stearylether, Propylenglykol, Propylenglykoldiacetat, Sesamöl und gereinigtes Wasser.

Zu den Stabilisatoren, die sich geeigneterweise verwenden lassen, zählen z.B. Calciumsaccharat und Thymol.
Verdickungsmittel werden typischerweise bei Formulierungen zur topischen Anwendung verwendet, um diesen die gewünschte Viskosität bzw. die gewünschten Handhabungseigenschaften zu verleihen; dazu zählen z.B. Cethylesterwachs, Myristylalkohol, Paraffin, synthetisches Paraffin, Emulgierwachs, mikrokristallines Wachs, gebleichtes Wachs und gelbes Wachs.

Zucker werden häufig verwendet, um den erfindungsgemäßen pharmazeutischen Zusammensetzungen verschiedene erwünschte Eigenschaften zu verleihen und um die erzielten Ergebnisse zu verbessern; dazu zählen z.B. Monosaccharide, Disaccharide und Polysaccharide wie Glucose, Xylose, Fruktose, Reose, Ribose, Pentose, Arabinose, Allose, Tallose, Altrose, Mannose, Galaktose, Laktose, Saccharose, Erythrose, Glyceraldehyd oder deren Kombinationen.

Tenside werden verwendet, um erfindungsgemäßen pharmazeutischen Zusammensetzungen mit mehreren Bestandteilen Stabilität zu verleihen, bereits vorhandene Eigenschaften dieser Zusammensetzungen zu verstärken und den Zusammensetzungen neue erwünschte Eigenschaften zu verleihen. Tenside werden als Netzmittel, Antischaummittel, zur Verringerung der Oberflächenspannung von Wasser sowie als Emulgatoren, Dispergatoren und Penetrationsförderer verwendet; dazu zählen z.B. Lapyriumchlorid; Laureth 4, d.h. α-Dodecyl-ω-hydroxy-poly(oxy-1,2-ethandiyl)- oder Polyethylenglykolmonododecylether, Laureth-9, d.h. ein Gemisch aus Polyethylenglykolmonododecylethem mit einem Durchschnitt von 9 Ethylenoxidgruppen pro Molekül, Monoethanolamin; Nonoxynol-4,-9 und -10, d.h. Polyethylenglykolmono(p-nonylphenyl)ether; Nonoxynol-15, d.h. α-(p-Nonylphenyl)-ω-hydroxypenta-deca(oxyethylen); Nonoxynol 30,d.h. α-(p-Nonylphenyl)-ω-hydroxtriaconta(oxyethylen), Poloxalene, d.h. nichtionogenes Polymer des Typs Polyethylenpolypropylenglykol, MG = ca. 3000, Poloxamer, das oben den Salbengrundlagen diskutiert wurde, Polyoxyl(8)-, -(40)- und -(50)-Stearat, d.h. Poly(oxy-1,2-ethanediyl)-a-hydro-cu-hydroxy-octadecanoat; Polyoxyl-10-oleylether, d.h. Poly(oxy-1,2-ethandiyl)-, α-[(Z)-9-Octadecenyl-ω-hydroxy-[Laküne], Polysorbat 20, d.h. Poly(oxy-1,2-ethandiyl)sorbitanmonododecanoat, Polysorbat 40, d.h. Poly(oxy-1,2-ethandiyl)sorbitanmonohexadecanoat, Polysorbat 60, d.h. Poly(oxy-1,2-ethandiyl)sorbitanmonooctadecanoat, Polysorbat 65, d.h. Poly(oxy-1,2-ethandiyl)sorbitantrioctadecanoat, Polysorbat 80, d.h. Poly(oxy-1,2-ethandiyl)sorbitanmono-9-octadecenoat, Polysorbat 85, d.h. Poly(oxy-1,2-ethandiyl)sorbitantri-9-octadecenoat, Natriumlaurylsulfat; Sorbitanmonolaurat, Sorbitanmonooleat, Sorbitanmonopalmitat, Sorbitanmonostearat, Sorbitansesquioleat, Sorbitantrioleat und Sorbitantristearat.

Die Herstellung der erfindungsgemäßen pharmazeutischen Zusammensetzungen erfolgt auf äußerst einfache Art, wie dies dem Durchschnittsfachmann gut bekannt ist. Handelt es sich bei den erfindungsgemäßen pharmazeutischen Zusammensetzungen um einfache wäßrige Lösungen bzw. Lösungen in anderen Lösungsmitteln, so werden die verschiedenen Bestandteile der Gesamtzusammensetzung in einer beliebigen praktischen Reihenfolge zusammengegeben, die hauptsächlich von Gründen der Bequemlichkeit bestimmt wird. Diejenigen Bestandteile, die eine geringere Löslichkeit in Wasser, jedoch eine ausreichende Löslichkeit in dem gleichen Hilfslösungsmittel mit Wasser aufweisen, lassen sich alle in diesem Hilfslösungsmittel auflösen, wonach der Wasseranteil des Trägers mit der Hilfslösung versetzt wird, wodurch sich die darin gelösten Stoffe im Wasser lösen. Zur Unterstützung dieses Dispergiervorgangs bzw. Lösungsvorgangs kann ein Tensid eingesetzt werden.

Sollen die erfindungsgemäßen pharmazeutischen Zusammensetzungen in Form von Emulsionen vorliegen, so werden die Bestandteile der pharmazeutischen Zusammensetzung gemäß den folgenden allgemeinen Vorgehensweisen zusammengegeben. Die geschlossene Wasserphase wird erst auf eine Temperatur im Bereich von ungefähr 60°C bis ungefähr 95°C, vorzugsweise ungefähr 70°C bis ungefähr 95°C, erhitzt, wobei die Wahl der verwendeten Temperatur von den physikalischen und chemischen Eigenschaften der Bestandteile, die die ÖI-in-Wasser-Emulsion bilden, abhängt. Sobald die geschlossene Wasserphase die gewählte Temperatur erreicht hat, werden die Bestandteile der endgültigen Zusammensetzung, die in diesem Stadium zuzugeben sind, unter starkem Rühren mit dem Wasser vermischt und darin dispergiert. Als nächstes wird die Temperatur des Wassers auf ungefähr das Ausgangsniveau gebracht, wonach die Bestandteile der Zusammensetzung, die den nächsten Schritt bilden, zu der Zusammensetzungsmischung unter mäßigem Rühren zugegeben werden, und es wird ungefähr 5 bis ungefähr 60 Minuten, vorzugsweise ungefähr 10 bis ungefähr 30 Minuten, je nach den Bestandteilen der ersten zwei Stufen, weiter gemischt. Hiernach wird die Zusammensetzungsmischung passiv oder aktiv auf ungefähr 20°C bis ungefähr 55°C gekühlt, so daß in den verbleibenden Stufen weitere Komponenten zugegeben werden können, wonach so viel Wasser zugegeben wird, daß die ursprünglich bestimmte Konzentration in der Gesamtzusammensetzung erzielt wird.

Erfindungsgemäß können die pharmazeutischen Zusammensetzungen in Form eines sterilen Injektionspräparats, zum Beispiel einer sterilen wäßrigen oder öligen Suspension zur Injektion vorliegen. Diese Suspension läßt sich nach fachbekannten Techniken mit geeigneten Dispergier-, Netz- und Suspendiermitteln formulieren. Bei dem sterilen Injektionspräparat kann es sich auch um eine sterile Lösung oder Suspension zur Injektion in einem nichttoxischen parenteral unbedenklichen Verdünnungsmittel oder Lösungsmittel handeln, zum Beispiel in Form einer Lösung in 1,3-Butandiol. Zu den unbedenklichen Konstituentien und Lösungsmitteln, die verwendet werden können, zählen Wasser, Ringersche Lösung sowie isotonische Kochsalzlösung. Außerdem werden sterile stabilisierte Öle üblicherweise als Lösungs- oder Suspendiermittel verwendet. Jedes milde stabilisierte Öl, darunter auch synthetische Mono- oder Diglyceride, kann für diesen Zweck verwendet werden. Fettsäuren wie Ölsäure und seine Glyceridderivate eignen sich zur Herstellung von Iniectabilia, ebenso natürliche pharmazeutisch unbedenkliche Öle, wie Olivenöl oder Rizinusöl, insbesondere in Form ihrer Polyethoxylate. Diese Öllösungen oder -suspensionen können auch als Verdünnungs- oder Dispergiermittel einen langkettigen Alkohol wie RH, HCIX oder einen ähnlichen Alkohol enthalten.

Die erfindungsgemäßen pharmazeutischen Zusammensetzungen lassen sich oral in einer beliebigen oral unbedenklichen Dosierungsform verabreichen, darunter Kapseln, Tabletten, wäßrige Suspensionen oder Lösungen, was jedoch keine Einschränkung darstellen soll. Bei den Oraltabletten zählen Laktose und Maisstärke zu häufig verwendeten Trägern. Typischerweise werden auch Gleitmittel wie Magnesiumstearat zugegeben. Bei der oralen Verabreichung in Kapselform zählen Laktose und getrocknete Maisstärke zu nützlichen Streckmitteln. Sollen wäßrige Lösungen oral verwendet werden, so wird der Wirkstoff mit Emulgatoren und Suspendiermitteln vereinigt. Falls gewünscht können auch bestimmte Süßstoffe, Geschmacksstoffe oder Farbstoffe zugegeben werden. Die erfindungsgemäßen pharmazeutischen Zusammensetzungen können jedoch auch in Form von Suppositorien zur rektalen Verabreichung verabreicht werden. Solche Suppositorien können dadurch hergestellt werden, daß man das Mittel mit einem geeigneten nicht reizenden Exzipiens vermischt, welches bei Raumtemperatur fest, bei der Rektaltemperatur jedoch flüssig ist und deshalb im Rektum schmilzt und so den Arzneistoff freigibt. Zu diesen Substanzen zählen Kakaobutter, Bienenwachs sowie Polyethylenglykole.

Die erfindungsgemäßen pharmazeutischen Zusammensetzungen können auch topisch verabreicht werden, insbesondere dann, wenn Flächen oder Organe, die einer topischen Anwendung leicht zugänglich sind, das Ziel der Behandlung bilden, darunter Augenerkrankungen, Hauterkrankungen oder Erkrankungen des unteren Verdauungstrakts. Geeignete topische Formulierungen lassen sich leicht für diese Flächen oder Organe herstellen

Die topische Anwendung für den unteren Verdauungstrakt kann als Rektalsuppositorienformulierung wie oben beschrieben oder in Form eines geeigneten Darmeinlaufpformulierung erfolgen. Topisch wirksame Transdermalpflaster können ebenfalls verwendet werden.

Für die topischen Anwendung können die pharmazeutischen Zusammensetzungen als geeignete Salbe formuliert werden, die den wirksamen Bestandteil in einem oder mehreren Trägem suspendiert oder gelöst enthält. Zu Trägem für die topische Verabreichung der erfindungsgemäßen Verbindungen zählen Mineralöl, Paraffinöl, weißes Vaselin, Propylenglykol, Polyoxyethylen-Polyoxypropylen-Verbindung, emulgierendes Wachs und Wasser, was jedoch keine Einschränkung darstellen soll. Die pharmazeutischen Zusammensetzungen können jedoch auch als geeignete Lotion oder Creme, die die wirksamen Bestandteile in einem oder mehreren pharmazeutisch unbedenklichen Trägern suspendiert oder gelöst enthalten, formuliert werden. Zu geeigneten Trägern zählen Mineralöl, Sorbitanmonostearat, Polysorbat, Cetylesterwachs, Cetearylalkohol, 2-Octyldodecanol, Benzylalkohol und Wasser, was jedoch keine Einschränkung darstellen soll.

Zu pharmazeutischen Zusammensetzungen, auf die sich die vorliegende Verbindung erstreckt, zählen auch diejenigen, bei denen die therapeutisch wirksame Menge eines Wirkstoffs mit einer Verbindung der Formel I, die zur Behandlung oder Vorbeugung von durch Modulation der PDE IV-Aktivität wie hierin beschrieben vermittelten bzw. damit assoziierten Erkrankungen, krankhaften Störungen und Leiden erforderlich ist, in einer zur systemischen Verabreichung geeigneten Dosierungsform bereitgestellt wird. Eine derartige pharmazeutische Zusammensetzung enthält den Wirkstoff in einer geeigneten flüssigen Form zur Abgabe durch: (1) Injektion oder Infusion, sei es intraarteriell, intra- oder transdermal, subkutan, intramuskulär, intraspinal, intrathecal oder intravenös, wobei der Wirkstoff (a) als gelöster Stoff in Lösung vorliegt, (b) in der offenen Phase einer Emulsion oder in der offenen Phase einer Emulsion mit Phasenumkehr, bei der sich die Phase bei Injektion oder Infusion umkehrt, wobei solche Emulsionen geeignete Emulgatoren enthalten, vorliegt, oder (c) in einer Suspension als suspendierter Feststoff in kolloidaler Form oder in Form feinster Teilchen vorliegt, wobei diese Suspension geeignete Suspendiermittel enthält, (2) Injektion oder Infusion in geeignete Körpergewebe oder -höhlen als Depot, wobei die Zusammensetzung den Wirkstoff lagert und anschließend zur systemischen Verteilung in Form einer Delayed-Release, Sustained-Release bzw. Controlled-Release freigibt, (3) Instillation, Inhalation oder Insufflation der pharmazeutischen Zusammensetzung in einer geeigneten festen Form in geeignete Körpergewebe oder -höhlen, wobei der Wirkstoff (a) in einem festen Implantat der Zusammensetzung vorliegt, das für die Freisetzung des Wirkstoffs in Form einer Delayed-Release, Sustained-Release bzw. Controlled-Release sorgt, (b) in einer teilchenförmigen Zusammensetzung, die in die Lunge eingeatmet wird, vorliegt, bzw. (c) in einer teilchenförmigen Zusammensetzung vorliegt, die in geeignete Körpergewebe oder -höhlen eingeblasen wird, wo die Zusammensetzung gewünschtenfalls für die Freisetzung des Wirkstoffs in Form einer Delayed-Release, Sustained-Release bzw. Controlled-Release bereitsteht, oder (4) Einnahme der pharmazeutischen Zusammensetzung in einer geeigneten festen oder flüssigen Form zur peroralen Abgabe des Wirkstoffs, wobei der Wirkstoff in einer festen Dosierungsform enthalten ist, oder (b) in einer flüssigen Dosierungsform enthalten ist.

Zu einzelnen Dosierungsformen der oben beschriebenen pharmazeutischen Zusammensetzungen zählen (1) Suppositorien als Spezialtyp eines Implantats, welche Grundlagen umfassen, die bei Raumtemperatur fest sind, jedoch bei Körpertemperatur schmelzen und so den Wirkstoff, den sie umfassen, langsam in das umgebende Körpergewebe abgeben, wo der Wirkstoff absorbiert wird und ein Transport erfolgt, so daß er systemisch verabreicht wird, (2) feste perorale Dosierungsformen der Gruppe (a) Oraltabletten, Kapseln, Caplets, Pastillen, Trochisken und mehrteilige Formen mit Delayed-Release-Freisetzung, (b) magensaftresistente Tabletten und Kapseln, die die Freisetzung und Absorption im Magen verhindern und so die Abgabe jenseits des Magens des behandelten Patienten ermöglichen, (c) Oraltabletten, Kapseln und feinteilige Formen mit Sustained-Release-Freigabe zur systemischen gesteuerten Ffreisetzung des Wirkstoffs über einen Zeitraum bis zu 24 Stunden, (d) rasch zerfallende Tabletten, (e) eingekapselte Lösungen, (f) Oral pasten, (g) ein Granulat, das in die bzw. in das Nahrungsmittel eines behandelten Patienten eingebracht wird, sowie (h) flüssige perorale Dosierungsformen aus der Gruppe der Lösungen, Suspensionen, Emulsionen, Emulsionen mit Phasenumkehr, Elexiere, Extrakte, Tinkturen und Konzentrate.

Zu pharmazeutischen Zusammensetzungen, auf die sich die vorliegende Verbindung erstreckt, zählen auch diejenigen, bei denen die therapeutisch wirksame Menge eines Wirkstoffs mit einer erfindungsgemäßen Verbindung, die zur Behandlung oder Vorbeugung von durch Modulation der PDE IV-Aktivität wie hierin beschrieben vermittelten bzw. damit assoziierten Erkrankungen, krankhaften Störungen und Leiden erforderlich ist, in einer Dosierungsform bereitgestellt wird, die sich für die lokale Verabreichung an einen behandelten Patienten eignet, wobei eine derartige pharmazeutische Zusammensetzung den Wirkstoff in einer geeigneten flüssigen Form enthält, um den Wirkstoff abzugeben durch (1) lokale Injektion oder Infusion, sei es intraarteriell, intraartikulär, intrachondrial, intrakostal, intrazystisch, intra- oder transdermal, intrafasciculär, intraligamentös, intramedullär, intramuskulär, intranasal, intraneural, intraoculär, d.h. opthalmische Verabreichung, intraossär, intrapelvin, intrapericardial, intraspinal, intrastemal, intrasynovial, intratarsal oder intrathecal, darunter auch Bestandteile, die für eine Delayed-Release-, Controlled-Release- bzw. Sustained-Release-Freisetzung des Wirkstoffs an diesem Lokus sorgen, wobei der Wirkstoff (a) als gelöster Stoff in Lösung vorliegt, (b) in der offenen Phase einer Emulsion oder in der offenen Phase einer Emulsion mit Phasenumkehr, bei der sich die Phase bei Injektion oder Infusion umkehrt, wobei solche Emulsionen geeignete Emulgatoren enthalten, vorliegt, oder (c) in einer Suspension als suspendierter Feststoff in kolloidaler Form oder in Form feinster Teilchen vorliegt, wobei diese Suspension geeignete Suspendiermittel enthält, (2) in einer Injektion oder Infusion als Depot enthalten ist zur Freisetzung des Wirkstoffs an den Lokus, wobei die Zusammensetzung den Wirkstoff lagert und anschließend an den Lokus in Form einer Delayed-Release, Sustained-Release bzw. Controlled-Release freigibt, wobei die Zusammensetzung auch Bestandteile beinhaltet, die sicherstellen, daß der Wirkstoff in erster Linie lokal wirkt und wenig systemisches Carry-over verursacht, oder wobei die pharmazeutische Zusammensetzung den Wirkstoff in einer geeigneten festen Form zur Abgabe des Inhibitors auf folgenden Weg enthält: (3) Instillation, Inhalation oder Insufflation an diesen Lokus, wobei der Wirkstoff enthalten ist in: (a) einem festen Implantat der Zusammensetzung, das an diesem Lokus implantiert wird, wobei die Zusammensetzung den Wirkstoff gegebenenfalls in Form einer Delayed-Release-, Sustained-Release- bzw. Controlled-Release-Freisetzung an den Lokus freigibt, (b) in einer teilchenförmigen Zusammensetzung, die in einen Lokus, darunter auch die Lunge, eingeatmet wird, bzw. (c) in einer teilchenförmigen Zusammensetzung, die in einen Lokus eingeblasen wird, wobei die Zusammensetzung Bestandteile beinhaltet, die sicherstellen, daß der Wirkstoff in erster Linie lokal wirkt und unwesentlich einem systemischen Carry-over unterliegt, sowie gegebenenfalls den Wirkstoff lokal in Form einer Delayed-Release-, Sustained-Release- bzw. Controlled-Relaise-Freigabe freisetzt. Zur ophthalmischen Verwendung lassen sich die pharmazeutischen Zusammensetzungen als mikronisierte Suspension in einer isotonischen sterilen Kochsalzlösung mit eingestelltem pH oder vorzugsweise als Lösungen in einer isotonischen sterilen Kochsalzlösung mit eingestelltem pH, mit oder ohne Konservierungsmittel wie Benzylalkoniumchlorid formulieren. Zur ophtalmischen Verwendung lassen sich die pharmazeutischen Zusammensetzungen auch in einer Salbe wie Vaselin formulieren.

Die erfindungsgemäßen pharmazeutischen Zusammensetzungen lassen auch mit einem Nasen-Aerosol oder durch Inhalation mit Verwendung eines Nebulisators, Trockenpulverinhalators oder Dosierinhalators verabreichen. Solche Zusammensetzungen werden nach Techniken, die in der Galenik gut bekannt sind, hergestellt, und können in Form von Lösungen in Kochsalzlösung mit Benzylalkohol oder anderen geeigneten Konservierungsmitteln, Resorptionsförderern zur Verbesserung der biologischen Verfügbarkeit, Fluorkohlenwasserstoffen und/oder anderen üblichen Solubilisierungsmitteln oder Dispergatoren hergestellt werden.

Wie bereits erwähnt können die erfindungsgemäßen Verbindungen der Formel I einen zu behandelnden Patienten systemisch in Form einer pharmazeutischen Zusammensetzung in einer geeigneten flüssigen Form mittels Injektion oder Infusion verabreicht werden. Im Körper des Patienten befinden sich verschiedene Stellen und Organsysteme, die es der korrekt formulierten pharmazeutischen Zusammensetzung, sobald sie injiziert oder infundiert ist, gestatten, den gesamten Körper und alle Organsysteme des behandelten Patienten zu durchdringen. Bei einer Injektion handelt es sich um eine Einzeldosis der pharmazeutischen Zusammensetzung, die üblicherweise mittels einer Spritze in das betreffende Gewebe eingepreßt wird. Die häufigsten Arten der Injektion sind intramuskulär, intravenös und subkutan. Im Gegensatz dazu handelt es sich bei der Infusion um die langsame Einbringung der pharmazeutischen Zusammensetzung in das betroffene Gewebe. Die häufigste Art der Infusion ist die intravenöse Infusion. Zu weiteren Arten der Injektion oder Infusion zählen die intraarterielle, intra- oder transdermale (darunter auch subkutane) oder intraspinale, insbesondere intratekale, Injektion oder Infusion. In diesen flüssigen pharmazeutischen Zusammensetzungen kann der Wirkstoff als gelöster Stoff in Lösung vorliegen. Dies stellt den häufigsten und am stärksten bevorzugten Typ einer solchen Zusammensetzung dar, es ist jedoch ein Wirkstoff in einer Salzform erforderlich, die eine einigermaßen gute Löslichkeit in Wasser aufweist. Das mit Abstand am stärksten bevorzugte Lösungsmittel für solche Zusammensetzungen ist Wasser (oder Kochsalzlösung). Gelegentlich können übersättigte Lösungen verwendet werden, diese sind jedoch problematisch in bezug auf ihre Stabilität und daher für den alltäglichen Gebrauch unpraktisch.

Falls es nicht möglich ist, eine bevorzugte Verbindung in einer Form zu erhalten, die die erforderliche Löslichkeit in Wasser aufweist, wie dies manchmal der Fall ist, kann der Durchschnittsfachmann mit seinen Fähigkeiten eine Emulsion herstellen, wobei es sich um eine Dispersion von kleinen Tröpfchen einer Flüssigkeit, der offenen oder inneren Phase, in einer zweiten Flüssigkeit, der geschlossenen oder äußeren Phase, mit der diese unmischbar ist, handelt. Die beiden Flüssigkeiten werden durch pharmazeutisch unbedenkliche Emulgatoren in emulgiertem Zustand gehalten. Handelt es sich bei dem Wirkstoff um ein wasserunlösliches Öl, kann er daher in einer Emulsion, bei der er die offene Phase bildet, verabreicht werden. Ist der Wirkstoff wasserunlöslich, jedoch in einem mit Wasser unmischbaren Lösungsmittel löslich, kann ebenfalls eine Emulsion verwendet werden. Obwohl der Wirkstoff am häufigsten als offene oder innere Phase einer sogenannten Öl-in-Wasser-Emulsion verwendet würde, könnte er auch als offene oder innere Phase einer Emulsion mit Phasenumkehr, die üblicherweise als Wasser in-ÖI-Emulsion bezeichnet wird, verwendet werden. Hier ist der Wirkstoff wasserlöslich und könnte als einfache wäßrige Lösung verabreicht werden. Solche Emulsionen mit Phasenumkehr invertieren jedoch bei Injektion oder Infusion in ein wäßriges Medium, wie das Blut, und bieten den Vorteil eines rascheren und wirksameren Dispergierens des Wirkstoffs in dieses wäßrige Medium als bei Verwendung einer wäßrigen Lösung. Emulsionen mit Phasenumkehr werden mit fachbekannten geeigneten pharmazeutisch unbedenklichen Emulgatoren hergestellt.

Ist der Wirkstoff beschränkt wasserlöslich, so kann er auch als suspendierter Feststoff in kolloidaler oder feinteiliger Form in einer Suspension, die unter Verwendung geeigneter pharmazeutisch unbedenklicher Suspendiermittel hergestellt wird, verabreicht werden. Die den Wirkstoff enthaltenden suspendierten Feststoffe können auch als Zusammensetzungen mit Delayed-Release-, Sustained-Release-, bzw. Controlled-Release-Freigabe formuliert werden.

Obwohl die systemische Verabreichung am häufigsten durch Injektion oder Infusion einer Flüssigkeit erfolgt, existieren viele Situationen, in denen es vorteilhaft oder sogar erforderlich ist, den Wirkstoff als Feststoff abzugeben. Die systemische Verabreichung von Feststoffen wird durch Instillation, Inhalation oder Insufflation einer pharmazeutischen Zusammensetzung in geeigneter fester Form, die den Wirkstoff enthält, durchgeführt. Bei der Instillation des Wirkstoffs kann ein festes Implantat der Zusammensetzung in geeignete Körpergewebe oder -höhlen eingesetzt werden. Das Implantat kann eine Matrix aus biologisch kompatiblen und biologisch abbaubaren Substanzen enthalten, in der Teilchen eines festen Wirkstoffs dispergiert sind, oder in der möglicherweise Tröpfchen oder isolierte Zellen eines flüssigen Wirkstoffs eingeschlossen sind. Die Matrix soll vom Körper möglichst abgebaut und vollständig resorbiert werden. Die Zusammensetzung der Matrix wird auch bevorzugt so ausgewählt, daß der Wirkstoff über längere Zeiträume, sogar mehrere Monate, in Form einer Controlled-Release-, Sustained-Release- bzw. Delayed-Release-Freisetzung abgegeben wird.

Der Ausdruck "Implantat" bezieht sich meistens auf eine feste wirkstoffhaltige pharmazeutische Zusammensetzung, während der Ausdruck "Depot" üblicherweise eine flüssige wirkstoffhaltige pharmazeutische Zusammensetzung bedeutet, die in einem beliebigen geeigneten Körpergewebe oder einer beliebigen geeigneten Körperhöhle abgelegt wird und so ein Reservoir oder einen Pool bildet, der langsam in die umgebenden Gewebe und Organe wandert und schließlich und endlich systemisch verteilt wird. Diese Unterscheidungen werden in der Fachwelt jedoch nicht immer streng gehandhabt und es wird daher vorgesehen, daß sich der Umfang der vorliegenden Erfindung auf flüssige Implantate und feste Depots sowie sogar jeweils feste und flüssige Mischformen erstreckt. Suppositorien können als eine Art Implantat aufgefaßt werden, da sie Grundlagen enthalten, die bei Raumtemperatur fest sind, jedoch bei der Körpertemperatur eines Patienten schmelzen und so den Wirkstoff, mit dem sie ausgestattet sind, langsam in das umgebende Gewebe des Körpers des Patienten freigeben, wo der Wirkstoff resorbiert und abtransportiert wird, und so systemisch verabreicht wird.

Die systemische Verabreichung läßt sich auch mittels Inhalation oder Insufflation eines Pulvers durchführen, d.h. einer teilchenförmigen wirkstoffhaltigen Zusammensetzung. Zum Beispiel kann der Wirkstoff in Pulverform mit üblichen Geräten zur Aerosolbildung teilchenförmiger Formulierungen in die Lunge eingeatmet werden. Der Wirkstoff kann als teilchenförmige Formulierung auch durch Insufflation verabreicht werden, d.h. durch einfaches Stäuben oder mit üblichen Geräten zur Aerosolbildung von teilchenförmigen Formulierungen in geeignete Körpergewebe oder - höhlen geblasen oder anderweitig dispergiert werden. Diese teilchenförmigen Zusammensetzungen können ebenfalls nach gut bekannten Prinzipien und mit bekannten Materialien so formuliert werden, daß man zu einem Wirkstoff mit Delayed-Release-, Sustained-Release- bzw. Controlled-Release-Freigabe gelangt.

Zu weiteren Möglichkeiten der systemischen Verabreichung, bei denen die erfindungsgemäßen Wirkstoffe entweder in flüssiger oder in fester Form verwendet werden, zählen der transdermale, intranasale und ophthalmische Verabreichungsweg. Insbesondere können Transdermalpflaster nach in der Arzneistoffabgabe bekannten Techniken hergestellt und auf die Haut des zu behandelnden Patienten aufgebracht werden, wonach der Wirkstoff aufgrund seiner formulierten Löslichkeitseigenschaften durch die Epidermis und in die Dermisschichten der Haut des Patienten wandert, wo er als Teil der allgemeinen Zirkulation des Patienten aufgenommen wird und schließlich und endlich zu einer systemischen Verteilung des Wirkstoffs über eine gewünschte, längere Zeitdauer führt. Dazu zählen auch Implantate, die unter die Epidermisschicht der Haut gegeben werden, d.h. zwischen die Epidermis und die Dermis der Haut des behandelten Patienten. Ein derartiges Implantat wird gemäß gut bekannter Prinzipien und Materialien, die häufig bei dieser Abgabetechnik verwendet werden, formuliert, und kann auf solche Art und Weise hergestellt werden, daß der Wirkstoff nach dem Prinzip der Controlled-Release-, Sustained-Release- bzw. Delayed-Release-Freisetzung in die systemische Zirkulation des Patienten abgegeben wird. Derartige subepidermalen (subkutikulären) Implantate sind genauso leicht wie Transdermalpflaster einzusetzen und bieten die gleiche wirksame Abgabe, jedoch ohne dem Abbau, der Schädigung oder der zufälligen Entfernung ausgesetzt zu sein, die davon herrührt, daß das Pflaster auf der äußersten Schicht der Haut des Patienten exponiert ist.

In der obigen Beschreibung der pharmazeutischen Zusammensetzungen, die eine bevorzugte Verbindung enthalten, wurden die gleichwertigen Ausdrücke "Verabreichung", "Verabreichung von", "Verabreichen" und "ein (e)...verabreichen" in bezug auf diese pharmazeutischen Zusammensetzungen verwendet. Diese Ausdrücke sollen im vorliegenden Zusammenhang bedeuten, daß einen behandlungsbedürftigen Patienten eine erfindungsgemäße pharmazeutische Zusammensetzung auf einem beliebigen der hier beschriebenen Verabreichungswege zur Verfügung gestellt wird, wobei es sich bei dem Wirkstoff um eine bevorzugte Verbindung oder ein Prodrug, ein Derivat oder einen Metaboliten hiervon handelt, das bzw. der sich zur Behandlung einer durch Modulation der PDE IV-Aküvität vermittelten oder hiermit assoziierten Erkrankung, krankhaften Störung oder eines derartigen Leiden bei diesem Patienten eignet. Die vorliegende Erfindung erstreckt sich daher auf eine beliebige andere Verbindung, die bei Verabreichung an einen Patienten fähig ist, eine bevorzugte Verbindung direkt oder indirekt zur Verfügung zu stellen. Solche Verbindungen sind als Prodrugs bekannt, und es existieren viele etablierte Vorgehensweisen zur Herstellung solcher Prodrug-Formen der bevorzugten Verbindungen.

Die Dosis bzw. Dosierung der bei der Behandlung oder Vorbeugung einer durch Modulation der PDE IV-Aktivität vermittelten bzw. damit assoziierten Erkrankung, krankhaften Störung bzw. eines derartigen Leidens hängt von verschiedenen Faktoren wie der Art des Inhibitors, der Größe des Patienten, dem Behandlungsziel, der Art der zu behandelnden Pathologie, der jeweils verwendeten pharmazeutischen Zusammensetzung sowie den Beobachtungen und Schlußfolgerungen des behandelnden Arztes ab.

Bei einer oralen Dosierungsform, z.B. einer Tablette oder Kapsel, liegen geeignete Dosismengen der Verbindungen der Formel I zwischen ungefähr 0,1 µg Wirkstoff/kg und ungefähr 50,0 mg Wirkstoff/kg Körpergewicht und Tag, vorzugsweise zwischen ungefähr 5,0 µg Wirkstoff/kg und ungefähr 5,0 mg Wirkstoff/kg Körpergewicht und Tag, stärker bevorzugt zwischen ungefähr 10,0 µg Wirkstoff/kg und ungefähr 1,0 mg Wirkstoff/kg Körpergewicht und Tag, am stärksten bevorzugt zwischen ungefähr 20,0 µg Wirkstoff/kg und ungefähr 0,5 mg Wirkstoff/kg Körpergewicht und Tag.

Wird die Dosierungsform topisch an die Bronchien und die Lunge verabreicht, z.B. mittels Pulverinhalator oder Nebulisator, so liegen geeignete Dosismengen der Verbindungen zwischen ungefähr 0,001 µg Wirkstoff/kg und ungefähr 10,0 mg Wirkstoff/kg Körpergewicht und Tag, vorzugsweise zwischen ungefähr 0,5 µg Wirkstoff/kg und ungefähr 0,5 mg Wirkstoff/kg Körpergewicht und Tag, stärker bevorzugt zwischen 1,0 µg Wirkstoff/kg und ungefähr 0,1 mg Wirkstoff/kg Körpergewicht und Tag, am stärksten bevorzugt zwischen ungefähr 2,0 µg Wirkstoff/kg und ungefähr 0,05 mg Wirkstoff/kg Körpergewicht und Tag.

Um den Bereich der oralen Tagesdosis, die wie oben beschrieben verwendet werden könnte, zu erläutern und unter Zuhilfenahme eines typischen Körpergewichts von 10 kg und 100 kg liegen geeignete Dosismengen der Verbindungen der Formel I zwischen ungefähr 1,0 -10,0 µg und 500,0 - 5000,0 mg des Wirkstoffs mit einer bevorzugten Verbindung pro Tag, vorzugsweise zwischen ungefähr 50,0 und 500,0 µg und 50,0 - 500,0 mg des Wirkstoffs mit einer bevorzugten Verbindung pro Tag, stärker bevorzugt zwischen ungefähr 100,0 -1000,0 µg und 10,0 - 100,0 mg eines Wirkstoffs mit einer bevorzugten Verbindung pro Tag, am stärksten bevorzugt zwischen ungefähr 200,0 - 20000 µg und ungefähr 5,0 - 500 mg des Wirkstoffs mit einer bevorzugten Verbindung pro Tag. Diese Dosierbereiche stellen Gesamtdosismengen des Wirkstoffs pro Tag für einen bestimmten Patienten dar. Wie oft eine Dosis pro Tag verabreicht wird, hängt von pharmakologischen und pharmakokinetischen Faktoren wie der Halbwertszeit des Wirkstoffs, welche seine Katabolisierungsgeschwindigkeit und Clearance widerspiegelt, sowie dem minimalen und optimalen Blutplasmaspiegel bzw. anderen Körperflüssigkeitsspiegeln des Wirkstoffs in einem Patienten, die für eine therapeutische Wirksamkeit erforderlich sind, ab.

Bei der Festsetzung der Anzahl Dosen pro Tag und der Wirkstoffmenge pro Dosis, die verabreicht werden, müssen auch zahlreiche andere Faktoren in Betracht gezogen werden. Ein weiterer derartiger Faktor ist nicht zuletzt die jeweilige Reaktion des behandelten Patienten. So werden zum Beispiel bei Verwendung des Wirkstoffs zur Behandlung oder Vorbeugung von Asthma bei topischer Verabreichung über Aerosol-Inhalation in die Lungen ein bis vier Dosen, die aus Betätigungen eines Abgabegeräts bestehen, d.h. "Sprühstößen" eines Inhalators, pro Tag verabreicht, wobei jede Dosis ungefähr 50,0 µg bis ungefähr 10,0 mg Wirkstoff enthält.

Die Erfindung betrifft weiterhin auch Arzneimittel mit mindestens einer Verbindung der Formel I und/oder ihren pharmazeutisch verwendbaren Derivaten, Solvaten und Stereoisomeren, deren Mischungen in allen Verhältnissen, sowie gegebenenfalls Exzipientien und/oder Hilfsstoffen. Die Erfindung betrifft weiterhin auch Arzneimittel mit mindestens einer Verbindung der Formel I und/oder ihren pharmazeutisch verwendbaren Derivaten, Solvaten und Stereoisomeren, einschließlich deren Mischungen in allen Verhältnissen sowie mindestens einem weiteren Arzneimittelwirkstoff.

Die Erfindung betrifft auch einen Satz (ein Kit) bestehend aus getrennten Packungen
(a) einer wirksamen Menge einer Verbindung der Formel und/oder ihren pharmazeutisch verwendbaren Derivaten, Solvaten und Stereoisomeren, sowie deren Mischungen in allen Verhältnissen
   und
(b) einer wirksamen Menge eines weiteren Arzneimittelwirkstoffs.

Der Satz beinhaltet geeignete Behältnisse wie Schachteln, einzelne Flaschen, Beutel oder Ampullen. Der Satz kann zum Beispiel einzelne Ampullen umfassen, die jeweils eine wirksame Menge einer Verbindung der Formel I und/oder ihrer pharmazeutisch verwendbaren Derivate, Solvate und Stereoisomeren, einschließlich deren Mischungen in allen Verhältnissen, sowie eine wirksame Menge eines weiteren Arzneimittelwirkstoffs in gelöster oder lyophilisierter Form enthalten.

Vor und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und/oder durch Kristallisation

| | |
|---|---|
| Massenspektrometrie | (MS) (electron impact ionization) M⁺ |
| | FAB (fast atom bombardment)(M+H)⁺ |

### Beispiel 1

1.1 Zu einer Lösung von 9,8 g (1) und 3,8 ml Pyridin in 100 ml Dichlormethan (DCM) tropft man unter Rühren und Kühlung 3,5 ml Chloracetylchlorid und rührt 16 Stunden bei Raumtemperatur nach. Man arbeitet wie üblich auf und erhält 6,1 g

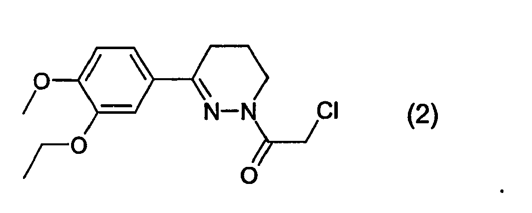

1.2 Zu einer Lösung von 3,045 g N-Hydroxyphthalimid und 3,3 g Kaliumcarbonat in 35 ml 1-Methyl-2-pyrrolidon (NMP) gibt man 5,8 g (2) und rührt 4 Stunden bei 50° nach. Man arbeitet wie üblich auf und erhält 4,8 g (3).

1.3 Eine Lösung von 4,8 g (3) in 200 ml Essigester wird mit 700 mg Ethanolamin versetzt und 2 Stunden bei 60° gerührt.
Man arbeitet wie üblich auf und erhält 2,8 g (4).

1.4 Eine Lösung von 0,1 g (4) und 0,02 ml Eisessig in 10 ml Ethanol wird mit 44,3 mg 4-Methoxybenzaldehyd versetzt und 16 Stunden bei Raumtemperatur gerührt.
Das Lösungsmittel wird entfernt, der Rückstand wird mit Ether aufgerührt, abgesaugt und mit Ether gewaschen.
Man erhält 110 mg der Verbindung 4-Methoxy-benzaldehyd O-{2-[3-(3-ethoxy-4-methoxy-phenyl)-5,6-dihydro-4H-pyridazin-1-yl]-2-oxo-ethyl}-oxim (I-A-1; siehe Tabelle 1).

### Verbindungen der Formeln I-A, I-B und I-C

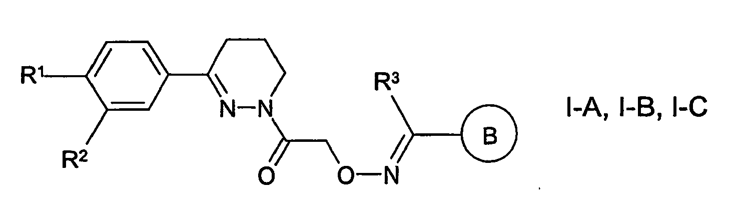

**Tabelle 1**

| Nr. | R¹ | R² | R³ | B | Bemerkungen |
|---|---|---|---|---|---|
| I-A-1 | OMe | OEt | H | 4-Methoxy-phenyl | |
| I-A-2 | OMe | OEt | H | Phenyl | |
| I-A-3 | OMe | OEt | H | 4-Hydroxy-phenyl | |
| I-A-4 | OMe | OEt | H | Pyridin-4-yl | |
| I-A-5 | OMe | OEt | H | Pyridin-4-yl-N-oxid | |
| | | | | | |
| I-B-1 | OMe | OPr | H | 4-Methoxyphenyl | |
| I-B-2 | OMe | OPr | H | Phenyl | |
| I-B-3 | OMe | OPr | H | Pyridn-4-yl | |
| I-B-4 | OMe | OPr | H | Pyridin-4-yl-N-oxid | |
| I-B-5 | OMe | OPr | H | 4-Nitrophenyl | |
| | | | | | |
| I-C-1 | OMe | OEt | H | 4-BOC-amino-phenyl | |
| I-C-2 | OMe | OEt | H | 4-Amino-phenyl | |
| I-C-3 | OMe | OEt | H | 4-[(CN)₂C=N-NH]-phenyl | |
| I-C-4 | OMe | OEt | H | 3-[(CN)₂C=N-NH]-phenyl | |
| I-C-5 | OMe | OPr | H | 4-[(CN)₂C=N-NH]-phenyl | |

| | | | | | |
|---|---|---|---|---|---|
| Me = Methyl; Et = Ethyl; Pr = Propyl | | | | | |

Analog erhält man durch Umsetzung von (4) mit
Benzaldehyd,
4-Hydroxybenzaldehyd,
Pyridin-4-carbaldehyd,
Pyridin-N-oxid-4-carbaldehyd,
die nachstehenden Verbindungen
I-A-2,
I-A-3,
I-A-4,
I-A-5.

### Beispiel 2

Analog Beispiel 1 erhält man durch Umsetzung von 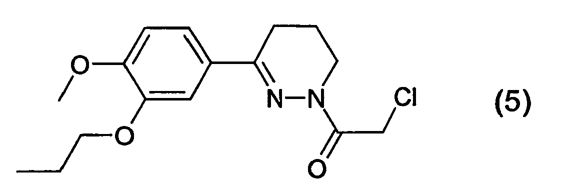 mit
4-Methoxybenzaldehyd,
Benzaldehyd,
Pyridin-4-carbaldehyd,
Pyridin-N-oxid-4-carbaldehyd, 4-Nitrobenzaldehyd
die nachstehenden Verbindungen der Formel I-B (Tabelle 1)
I-B-1,
I-B-2,
I-B-3,
I-B-4,
I-B-5.

### Beispiel 3

3.1 Analog Beispiel 1 erhält man durch Umsetzung von (4) mit 4-BOC-amino-benzaldehyd die Verbindung I-C-1 (Tabelle 1).

3.2 Durch Abspaltung der BOC-Schutzgruppe mit HCI in Dioxan erhält man I-C-2.

3.3 Zu einer Lösung von 0,6 25 %iger HCI in 10 ml Wasser gibt man 270 mg 1-C-2. Man kühlt auf -2°, gibt 48,3 mg NaNO₂ zu und rührt 1 Stunde nach. Dann werden 43,5 mg Malodinitril zugegeben und 1 Stunde bei Raumtemperatur nachgerührt. Man stellt mit gesättigter Natriumacetatlösung auf pH 5, filtriert das ausgefallene Material ab, wäscht mit Wasser und trocknet 16 Stunden bei 50°.
Mit der äquimolaren Menge einer 0,5 N KOH in Methanol wird das Filtrat versetzt und eingeengt. Der Rest wird aus Ethanol umkristallisiert. Ausbeute: 230 mg 2-{[4-({2-[3-(3-Ethoxy-4-methoxy-phenyl)-5,6-dihydro-4H-pyridazin-1-yl]- 2-oxo-ethoxyimino}-methyl)-phenyl]-hydrazono}-malonsäurenitril, Kaliumsalz (I-C-3)

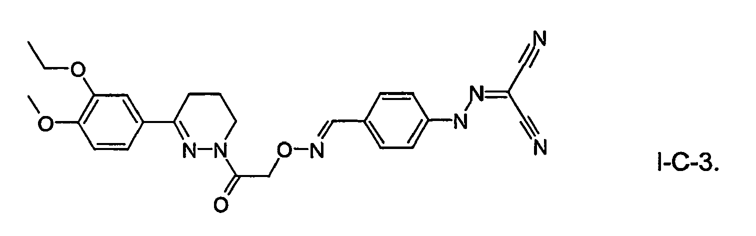

### Analog erhält man die Verbindungen

2-{[3-({2-[3-(3-Ethoxy-4-methoxy-phenyl)-5,6-dihydro-4*H*-pyridazin-1-yl]- 2-oxo-ethoxyimino}-methyl)-phenyl]-hydrazono}-malonsäurenitril, Kaliumsalz (I-C-4) und
2-{[3-({2-[3-(3-Propoxy-4-methoxy-phenyl)-5,6-dihydro-4*H*-pyridazin-1-yl]- 2-oxo-ethoxyimino}-methyl)-phenyl]-hydrazono}-malonsäurenitril, Kaliumsalz (I-C-5).

### Beispiel 4

4.1 Zu einer Lösung von 600 mg (4) in Ethanol/Eisessig gibt man 293 mg Benzoylameisensäure und rührt 16 Stunden bei Raumtemperatur. Das Lösungsmittel wird abdestilliert, der Rückstand wird mit Ether aufgerührt, abgesaugt und mit Ether gewaschen.
Man erhält 800 mg der Verbindung {2-[3-(3-Ethoxy-4-methoxy-phenyl)-5,6-dihydro-4H-pyridazin-1-yl]-2-oxo-ethoxyimino}-2-phenyl-essigsäure (I-D-1; Tabelle 2).

### Verbindungen der Formeln I-D

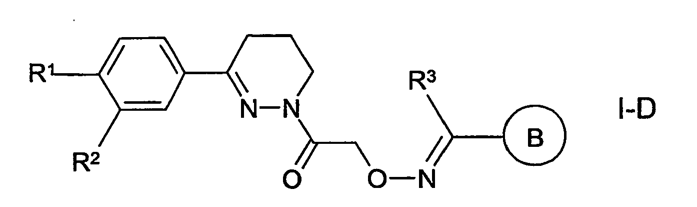

**Tabelle 2**

| Nr. | R¹ | R² | R³ | B | Bemerkungen |
|---|---|---|---|---|---|
| I-D-1 | OMe | OEt | COOH | Phenyl | |
| I-D-2 | OMe | OEt | N-(1-Methyl-piperidin-4-yl)-N-methylaminocarbonyl- | Phenyl | |
| I-D-3 | OMe | OEt | 4-Methyl-piperazinyl-carbonyl | Phenyl | |
| I-D-4 | OMe | OEt | N,N-Dimethylaminoethylaminocarbonyl | Phenyl | |
| | | | | | |

| | | | | | |
|---|---|---|---|---|---|
| Me = Methyl; Et = Ethyl; Pr = Propyl | | | | | |

4.2 Durch weitere Umsetzung von I-D-1 mit den Aminen
1-Methyl-4-(methylamino)-piperidin,
4-Methyl-piperazin,
2-Dimethylamino-ethylamin
erhält man die nachstehenden Verbindungen
2-{2-[3-(3-Ethoxy-4-methoxy-phenyl)-5,6-dihydro-4H-pyridazin-1-yl]-2-oxo-ethoxyimino}-N-methyl-N-(1-methyl-piperidin-4-yl)-2-phenyl-acetamid (I-D-2) 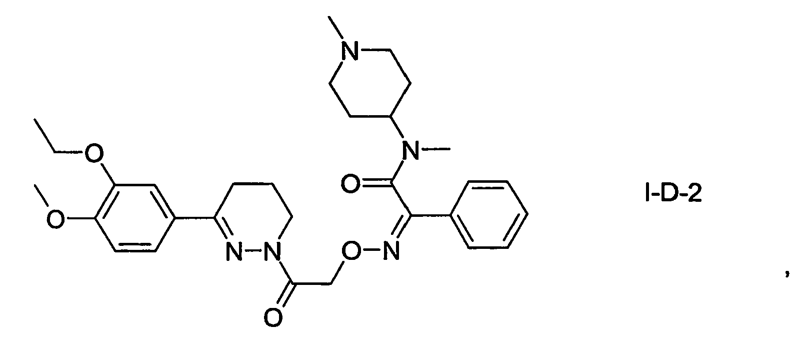
1-(4-Methyl-piperazin-1-yl)-2-phenyl-ethane-1,2-dione 2-(0-{2-[3-(3-ethoxy-4-methoxy-phenyl)-5,6-dihydro-4H-pyridazin-1-yl]-2-oxo-ethyl}-oxim (I-D-3) 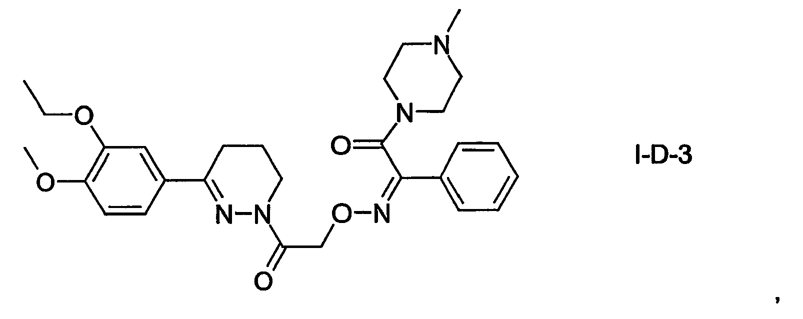
N-(2-Dimethylamino-ethyl)-2-{2-[3-(3-ethoxy-4-methoxy-phenyl)-5,6-dihydro-4H-pyridazin-1-yl]-2-oxo-ethoxyimino}-2-phenyl-acetamid (I-D-4)

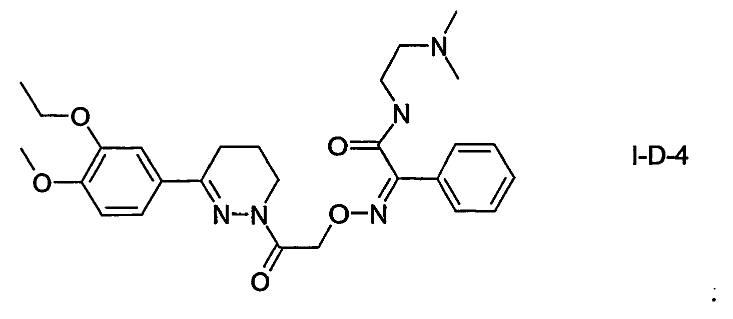

### Beispiel 5

Eine Lösung von 500 mg I-C-3, 66 mg Ammoniumchlorid, 81 mg Natriumazid und einer katalytischen Menge Lithiumchlorid in 4 ml DMF wird 24 Stunden bei 120° gerührt. Die Aufarbeitung erfolgt auf übliche Weise, wonach die getrockneten Kristalle wieder in Methanol gelöst werden. Man versetzt mit einer äquivalenten Menge KOH in Methanol. Das Lösungsmittel wird entfernt und der Rückstand wird mit Essigester/Diethylether versetzt.
Ausbeute: 460 mg der Verbindung 2-{[4-({2-[3-(3-Ethoxy-4-methoxy-phenyl)-5,6-dihydro-4*H*-pyridazin-1-yl]-2-oxo-ethoxyimino}-methyl)-phenyl]-hydrazono}-2-(1*H*-tetrazol-5-yl)essigsäurenitril, Kaliumsalz (I-E-1)

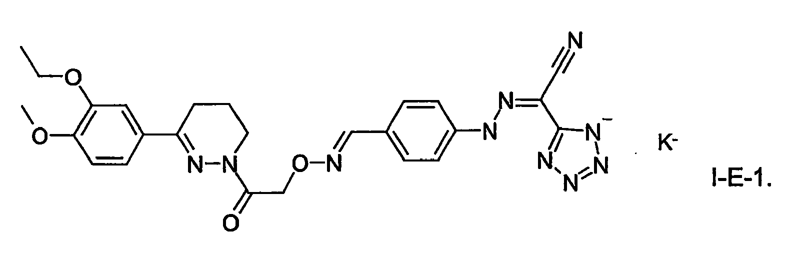

### Beispiel I: Auswirkung der Verbindungen der Formel I auf die Proliferation von T-Zellen

Periphere Blutmonozyten (PBMC) werden aus dem Blut gesunder Spender nach dem Lymphoprep-Gradientenverfahren isoliert. Pro Well werden 200000 PBMC in RPMI1640-Kulturmedium mit 5% hitzeinaktiviertem Humanserum (AB-Pool) 5 Tage bei 37°C und 10% CO₂ in 96-Well-Flachbodenmikrtotiterplatten kultiviert. Die T-Zellen der PBMC-Probe werden selektiv mit einem monoklonalen Antikörper gegen CD3 stimuliert.
Die Kulturen werden in dreifacher Wiederholung, darunter eine Kontrollgruppe ohne Behandlung, angesetzt.
Die Verbindungen der Formel I werden in einer Konzentration von 10⁻² M in DMSO gelöst und mit Kulturmedium verdünnt. Die Kontrollkulturen werden mit DMSO entsprechend der Inhibitorkonzentration behandelt. 18 Stunden vor dem Ende des Assays werden die Kulturen mit ³H-Thymidin versetzt.
Der Einbau der Radioaktivität in die Zellen wird dann in einem Beta-Zählgerät gemessen.
Die Werte von mindestens drei unabhängigen Versuchen werden als Prozenthemmung der Kontrolle (Mittel ± SFN) ohne Inhibitor berechnet.
Aus diesen Werten wird der IC₅₀-Wert bestimmt.

### Beispiel II: Auswirkung der Verbindungen der Formel I auf die Cytokinproduktion in menschlichen peripheren Blutmonocyten

Periphere Blutmonozyten (PBMC) werden aus dem Blut gesunder Spender nach dem Lymphoprep-Gradientenverfahren isoliert. Pro Well werden 200000 PBMC in RPMI1640-Kulturmedium mit 5% hitzeinaktiviertem Humanserum (AB-Pool) bei 37°C und 10% CO₂ in 96-Well-Flachbodenmikrtotiterplatten kultiviert. Die Kulturen werden in dreifacher Wiederholung, darunter eine Kontrollgruppe, angesetzt. Lösungen der Verbindungen der Formel I in DMSO werden in einer Konzentration von 10⁻² M hergestellt und mit Kulturmedium verdünnt. Die Kontrollkulturen werden mit DMSO-Konzentrationen entsprechend den Inhibitorkonzentrationen behandelt.
Die Kulturüberstände von drei unabhängigen Versuchen werden gepoolt und die Cytokinaktivität im Überstand wird mit kommerziell erhältlichen ELISA-Test-Kits gemessen.
Die Daten werden als Prozenthemmung/Stimulation der Kontrolle ohne die Verbindung berechnet und der IC₅₀-Wert bzw. EC₅₀-Wert bei der Stimulation wird daraus bestimmt.

Die folgenden Beispiel betreffen pharmazeutische Zubereitungen:

### Beispiel A: Injektionsgläser

Eine Lösung von 100 g eines Wirkstoffes der Formel I und 5 g Dinatriumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaselin unter aseptischen Bedingungen.

### Beispiel E: Tabletten

Ein Gemisch von 1 kg Wirkstoff der Formel I, 4 kg Laktose, 1,2 kg Kartoffelstärke, 0,2 kg Talk und 0,1 kg Magnesiumstearat wird in üblicher Weise so zu Tabletten verpreßt, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen der Formel I 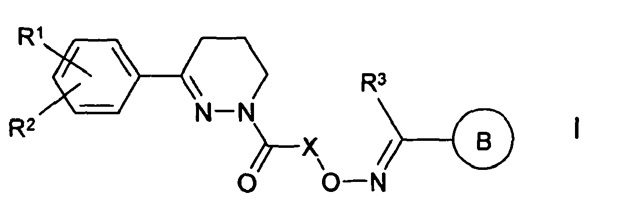 worin
R¹, R² jeweils unabhängig voneinander H, OH, OR⁸, -SR⁸, -SOR⁸, -SO₂R⁸ oder Hal bedeuten,
R¹ und R² zusammen auch -OCH₂O- oder -OCH₂CH₂O- bedeuten,
R³ H, A"R⁷, COA"R⁷, COOA"R⁷, CONH₂, CONHA"R⁷, CON(A"R⁷)(A'''R⁷), CONR¹⁰Het, NH₂, NHA"R⁷, N(A"R⁷)(A'''R⁷), NCOA"R⁷ oder NCOOA"R⁷,
B Phenyl, Pyridyl, Pyridyl-N-oxid, Thienyl, Furyl, Pyrrolyl, Pyridazinyl, Pyrimidinyl, Pyrazinyl, Triazinyl, Isoxazolinyl, Oxazolinyl, Thiazolinyl, Pyrazolinyl, Imidazolinyl, Naphthyl, Chinolinyl, Isochinolinyl, Cinnolinyl, Phthalazinyl, Chinazolinyl oder Chinoxalinyl, das unsubstituiert ist oder ein-, zwei- oder dreifach durch R⁴, R⁵ und/oder R⁶ substituiert sein kann,
X Alkylen mit 1-10 C-Atomen oder Alkenylen mit 2-8 C-Atomen,
worin eine, zwei oder drei CH₂-Gruppen durch O, S, SO, SO₂, NH oder NA"R⁷ ersetzt sein können,
1-7 H-Atome durch F und/oder Cl ersetzt sein können,
R⁴, R⁵, R⁶ jeweils unabhängig voneinander H, A"R⁷, OH, OA"R⁷, NO₂, NH₂, NHA"R⁷, N(A"R⁷)(A'''R⁷), NHCOA"R⁷, NHCOOA"R⁷, NHCONH₂, NHCONHA"R⁷, NHCON(A"R⁷)(A"'R⁷), Hal, COOH, COOA"R⁷, CONH₂, CONHA"R⁷, CON(A"R⁷)(A"'R⁷), 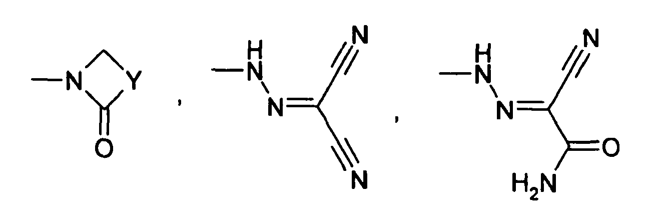 oder 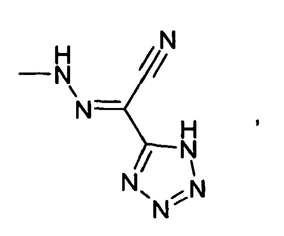
R⁷ H, COOH, COOA, CONH₂, CONHA, CONAA', NH₂, NHA, NAA', NCOA, NCOOA, OH oder OA,
R⁸ A, Cycloalkyl mit 3-7 C-Atomen, Alkylencycloalkyl mit 4-8 C- Atomen oder Alkenyl mit 2-8 C-Atomen,
R⁹ Alkyl mit 1-10 C-Atomen, Cycloalkyl mit 3-7 C-Atomen, Alkylencycloalkyl mit 4-8 C-Atomen oder Alkenyl mit 2-8 C- Atomen,
worin eine, zwei oder drei CH₂-Gruppen durch O, S, SO, SO₂, NH, NMe, NEt und/oder durch -CH=CH-Gruppen ersetzt sein können,
1-7 H-Atome durch F und/oder Cl ersetzt sein können
und/oder 1 H-Atom durch R⁷ ersetzt sein kann,
Y Alkylen mit 1-10 C-Atomen oder Alkenylen mit 2-8 C-Atomen, worin eine, zwei oder drei CH₂-Gruppen durch O, S, SO, SO₂, NH oder NR⁹ und/oder
1-7 H-Atome durch F und/oder Cl ersetzt sein können,
A, A' jeweils unabhängig voneinander Alkyl mit 1-10 C-Atomen oder Alkenyl mit 2-8 C-Atomen,
worin eine, zwei oder drei CH₂-Gruppen durch O, S, SO, SO₂, NH oder NR⁹ und/oder
1-7 H-Atome durch F und/oder Cl ersetzt sein können,
oder
Aryl oder Het,
A und A' zusammen auch eine Alkylenkette mit 2-7 C-Atomen, worin eine, zwei oder drei CH₂-Gruppen durch O, S, SO, SO₂, NH, NR⁹, NCOR⁹ oder NCOOR⁹ ersetzt sein können,
A", A"' jeweils unabhängig voneinander
fehlt, Alkylen mit 1-10 C-Atomen, Alkenylen mit 2-8 C-Atomen oder Cycloalkylen mit 3-7 C-Atomen,
worin eine, zwei oder drei CH₂-Gruppen durch O, S, SO, SO₂, NH oder NR⁹ und/oder
1-7 H-Atome durch F und/oder Cl ersetzt sein können,
A" und A"' zusammen auch eine Alkylenkette mit 2-7 C-Atomen, worin eine, zwei oder drei CH₂-Gruppen durch O, S, SO, SO₂, NH, NR⁹, NCOR⁹ oder NCOOR⁹ ersetzt sein können,
Aryl unsubstituiertes oder ein-, zwei- oder dreifach durch Hal, R¹¹, OR¹⁰, N(R¹⁰)_{2,} NO₂, CN, COOR¹⁰, CON(R¹⁰)₂, NR¹⁰COR¹⁰, NR¹⁰CON(R¹⁰)₂, NR¹⁰SO₂A, COR¹⁰, SO₂N(R¹⁰)₂, S(O)ₘR¹¹ substituiertes Phenyl, Naphthyl, Fluorenyl oder Biphenyl,
R¹⁰ H oder Alkyl mit 1-6 C-Atomen,
R¹¹ Alkyl mit 1-6 C-Atomen,
Het einen ein- oder zweikernigen gesättigten, ungesättigten oder aromatischen Heterocyclus mit 1 bis 2 N-, O- und/oder S- Atomen, der unsubstituiert oder ein- oder zweifach durch Carbonylsauerstoff, Hal, R¹¹, OR¹⁰, N(R¹⁰)₂, NO₂, CN, COOR¹⁰, CON(R¹⁰)₂, NR¹⁰COR¹⁰, NR¹⁰CON(R¹⁰)₂, NR¹⁰SO₂R¹¹, COR¹⁰, SO₂NR¹⁰ und/oder S(O)ₘR¹¹ substituiert sein kann,
Hal F, Cl, Br oder I,
m 0, 1 oder 2,
bedeuten,
sowie ihre pharmazeutisch verwendbaren Salze, Solvate, Stereoisomere und E/Z-Isomeren, einschließlich deren Mischungen in allen Verhältnissen.

2. Verbindungen nach Anspruch 1, worin
R¹, R² jeweils unabhängig voneinander Alkoxy mit 1, 2, 3, 4, 5 oder 6 C- Atomen
bedeuten,
sowie ihre pharmazeutisch verwendbaren Salze, Solvate, Stereoisomere und E/Z-Isomeren, einschließlich deren Mischungen in allen Verhältnissen.

3. Verbindungen nach Anspruch 1, worin
R¹, R² jeweils unabhängig voneinander H, Methoxy, Ethoxy, Benzyloxy, Propoxy, Isopropoxy, Difluormethoxy, F, Cl, Cyclopentyloxy, Cyclohexyloxy oder Cycloheptyloxy
bedeuten,
sowie ihre pharmazeutisch verwendbaren Salze, Solvate, Stereoisomere und E/Z-Isomeren, einschließlich deren Mischungen in allen Verhältnissen.

4. Verbindungen nach Anspruch 1, worin
R¹, R² jeweils unabhängig voneinander Methoxy, Ethoxy, Propoxy, Isopropoxy, Cyclopentyloxy oder F,
bedeuten,
sowie ihre pharmazeutisch verwendbaren Salze, Solvate, Stereoisomere und E/Z-Isomeren, einschließlich deren Mischungen in allen Verhältnissen.

5. Verbindungen gemäß einem oder mehreren der Ansprüche 1-4, worin
R¹ 4-Methoxy,
R² 3-Ethoxy oder 3-Propoxy,
bedeuten,
sowie ihre pharmazeutisch verwendbaren Salze, Solvate, Stereoisomere und E/Z-Isomeren, einschließlich deren Mischungen in allen Verhältnissen.

6. Verbindungen gemäß einem oder mehreren der Ansprüche 1-5,
worin
R³ H, A"R⁷, COA"R⁷, CON(A"R⁷)(A"'R⁷) oder CO-NR¹⁰-Het bedeutet,
sowie ihre pharmazeutisch verwendbaren Salze, Solvate, Stereoisomere und E/Z-Isomeren, einschließlich deren Mischungen in allen Verhältnissen.

7. Verbindungen gemäß einem oder mehreren der Ansprüche 1-6, worin
X Methylen, Ethylen, Propylen oder Butylen bedeutet,
sowie ihre pharmazeutisch verwendbaren Salze, Solvate, Stereoisomere und E/Z-Isomeren, einschließlich deren Mischungen in allen Verhältnissen.

8. Verbindungen gemäß einem oder mehreren der Ansprüche 1-7, worin
B Phenyl, Pyridyl, Pyridyl-N-oxid, Thienyl, Furyl, Pyrrolyl, Pyridazinyl, Pyrimidinyl, Pyrazinyl, Triazinyl, Isoxazolinyl, Oxazolinyl, Thiazolinyl, Pyrazolinyl, Imidazolinyl, Naphthyl, Chinolinyl, Isochinolinyl, Cinnolinyl, Phthalazinyl, Chinazolinyl oder Chinoxalinyl, das unsubstituiert ist oder ein-, zwei- oder dreifach durch OH, OA, NO₂, NH₂, NAA', 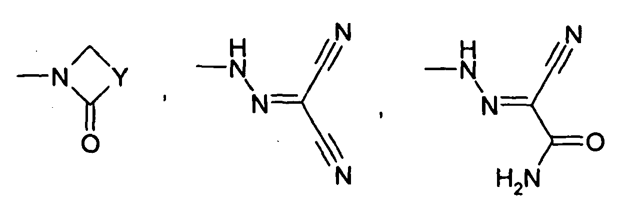 oder 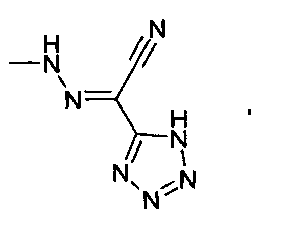 substituiert sein kann,
bedeutet,
sowie ihre pharmazeutisch verwendbaren Salze, Solvate, Stereoisomere und E/Z-Isomeren, einschließlich deren Mischungen in allen Verhältnissen.

9. Verbindungen gemäß einem oder mehreren der Ansprüche 1-8, worin
B unsubstituiertes oder einfach durch OR¹⁰, NO₂ oder 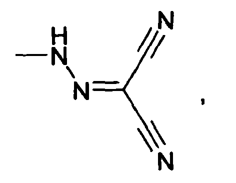 substituiertes Phenyl oder unsubstituiertes Pyridyl oder Pyridyl-N-oxid,
bedeutet,
sowie ihre pharmazeutisch verwendbaren Salze, Solvate, Stereoisomere und E/Z-Isomeren, einschließlich deren Mischungen in allen Verhältnissen.

10. Verbindungen gemäß einem oder mehreren der Ansprüche 1-9, worin
R¹, R² jeweils unabhängig voneinander Alkoxy mit 1, 2, 3, 4, 5 oder C- Atomen,
X Methylen, Ethylen, Propylen oder Butylen,
R³ H, A"R⁷, COA"R⁷, CON(A"R⁷)(A"'R⁷) oder CO-NR¹⁰-Het,
A", A"' jeweils unabhängig voneinander fehlt oder Alkylen mit 1-10 C- Atomen, worin eine CH₂-Gruppe durch NH oder NR⁹ ersetzt sein kann,
A", A"' zusammen auch eine Alkylenkette mit 2-7 C-Atomen, worin eine CH₂-Gruppe durch NH oder NR⁹ ersetzt sein kann,
B unsubstituiertes oder einfach durch OR¹⁰, NO₂, 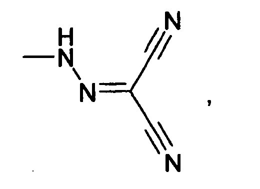 NH₂ oder NHCOOA"R⁷ substituiertes Phenyl oder unsubstituiertes Pyridyl oder Pyridyl-N-oxid,
R⁷ H, COOH, NHA oder NAA',
R⁹ Alkyl mit 1-6 C-Atomen,
R¹⁰ H oder Alkyl mit 1-6 C-Atomen,
A, A' jeweils unabhängig voneinander Alkyl mit 1-10 C-Atomen, worin 1- 7 H-Atome durch F und/oder Cl ersetzt sein können,
Het einen einkernigen gesättigten Heterocyclus mit 1 bis 2 N-Atomen, der ein- oder zweifach durch Alkyl mit 1-6 C-Atomen substituiert sein kann,
bedeuten,
sowie ihre pharmazeutisch verwendbaren Salze, Solvate, Stereoisomere und E/Z-Isomeren, einschließlich deren Mischungen in allen Verhältnissen.

11. Verbindungen gemäß einem oder mehreren der Ansprüche 1-10, worin
R¹, R² jeweils unabhängig voneinander Alkoxy mit 1, 2, 3, 4, 5 oder 6 C- Atomen,
X Methylen, Ethylen, Propylen oder Butylen,
R³ H, A"R⁷, COA"R⁷, CON(A"R⁷)(A"'R⁷) oder CO-NR¹⁰-Het,
A", A"' jeweils unabhängig voneinander fehlt oder Alkylen mit 1-10 C- Atomen, worin eine CH₂-Gruppe durch NH oder NR⁹ ersetzt sein kann,
A", A"' zusammen auch eine Alkylenkette mit 2-7 C-Atomen, worin eine CH₂-Gruppe durch NH oder NR⁹ ersetzt sein kann,
B unsubstituiertes oder einfach durch OR¹⁰, NO₂, 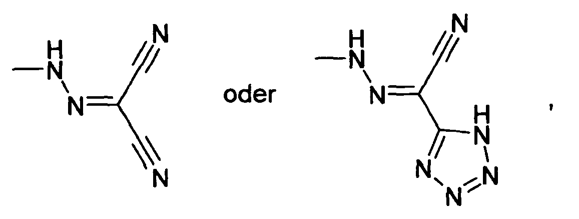 NH₂ oder NHCOOA"R⁷ substituiertes Phenyl oder unsubstituiertes Pyridyl oder Pyridyl-N-oxid,
R⁷ H, COOH, NHA oder NAA',
R⁹ Alkyl mit 1-6 C-Atomen,
R¹⁰ H oder Alkyl mit 1-6 C-Atomen,
A, A' jeweils unabhängig voneinander Alkyl mit 1-10 C-Atomen, worin 1- 7 H-Atome durch F und/oder Cl ersetzt sein können,
Het einen einkernigen gesättigten Heterocyclus mit 1 bis 2 N-Atomen, der ein- oder zweifach durch Alkyl mit 1-6 C-Atomen substituiert sein kann
bedeuten,
sowie ihre pharmazeutisch verwendbaren Salze, Solvate, Stereoisomere und E/Z-Isomeren, einschließlich deren Mischungen in allen Verhältnissen.

12. Verbindungen der Formel I nach Anspruch 1 aus der Gruppe
a) 4-Methoxy-benzaldehyd O-{2-[3-(3-ethoxy-4-methoxy-phenyl)-5,6-dihydro-4H-pyridazin-1-yl]-2-oxo-ethyl}-oxim,
b) Benzaldehyd O-{2-[3-(3-ethoxy-4-methoxy-phenyl)-5,6-dihydro-4H-pyridazin-1-yl]-2-oxo-ethyl}-oxim,
c) 4-Hydroxy-benzaldehyd O-{2-[3-(3-ethoxy-4-methoxy-phenyl)-5,6-dihydro-4H-pyridazin-1-yl]-2-oxo-ethyl}-oxim,
d) Pyridin-4-carbaldehyd O-{2-[3-(3-ethoxy-4-methoxy-phenyl)-5,6-dihydro-4H-pyridazin-1-yl]-2-oxo-ethyl}-oxim,
e) 1-Oxy-pyridin-4-carbaldehyd O-{2-[3-(3-ethoxy-4-methoxy-phenyl)-5,6-dihydro-4H-pyridazin-1-yl]-2-oxo-ethyl}-oxim,
f) 4-Methoxy-benzaldehyd O-{2-[3-(4-methoxy-3-propoxy-phenyl)-5,6-dihydro-4H-pyridazin-1-yl]-2-oxo-ethyl}-oxim,
g) Benzaldehyd O-{2-[3-(4-methoxy-3-propoxy-phenyl)-5,6-dihydro-4H-pyridazin-1-yl]-2-oxo-ethyl}-oxim,
h) Pyridin-4-carbaldehyd O-{2-[3-(4-methoxy-3-propoxy-phenyl)-5,6-dihydro-4H-pyridazin-1-yl]-2-oxo-ethyl}-oxim,
i) 1-Oxy-pyridin-4-carbaldehyd O-{2-[3-(4-methoxy-3-propoxy-phenyl)-5,6-dihydro-4H-pyridazin-1-yl]-2-oxo-ethyl}-oxim,
j) 4-Nitro-benzaldehyd O-{2-[3-(4-methoxy-3-propoxy-phenyl)-5,6-dihydro-4H-pyridazin-1-yl]-2-oxo-ethyl}-oxim,
k) 4-Amino-benzaldehyd O-{2-[3-(4-methoxy-3-propoxy-phenyl)-5,6-dihydro-4H-pyridazin-1-yl]-2-oxo-ethyl}-oxim,
l) 4-tert.-Butyloxycarbonyl-amino-benzaldehyd O-{2-[3-(4-methoxy-3-propoxy-phenyl)-5,6-dihydro-4H-pyridazin-1-yl]-2-oxo-ethyl}-oxim,
m) 2-{[4-({2-[3-(3-Ethoxy-4-methoxy-phenyl)-5,6-dihydro-4H-pyridazin-1-yl]-2-oxo-ethoxyimino}-methyl)-phenyl]-hydrazono}-malonsäurenitril,
n) 2-{[3-({2-[3-(3-Ethoxy-4-methoxy-phenyl)-5,6-dihydro-4H-pyridazin-1-yl]-2-oxo-ethoxyimino}-methyl)-phenyl]-hydrazono}-malonsäurenitril,
o) 2-{[4-({2-[3-(4-Methoxy-3-propoxy-phenyl)-5,6-dihydro-4H-pyridazin-1-yl]-2-oxo-ethoxyimino}-methyl)-phenyl]-hydrazono}-malonsäurenitril,
p) {2-[3-(3-Ethoxy-4-methoxy-phenyl)-5,6-dihydro-4H-pyridazin-1-yl]-2-oxo-ethoxyimino}-2-phenyl-essigsäure,
q) 2-{2-[3-(3-Ethoxy-4-methoxy-phenyl)-5,6-dihydro-4H-pyridazin-1-yl]-2-oxo-ethoxyimino}-N-methyl-N-(1-methyl-piperidin-4-yl)-2-phenyl-acetamid,
r) 1-(4-Methyl-piperazin-1-yl)-2-phenyl-ethane-1,2-dione 2-(O-{2-[3-(3-ethoxy-4-methoxy-phenyl)-5,6-dihydro-4H-pyridazin-1-yl]-2-oxo-ethyl}-oxim,
s) N-(2-Dimethylamino-ethyl)-2-{2-[3-(3-ethoxy-4-methoxy-phenyl)-5,6-dihydro-4H-pyridazin-1-yl]-2-oxo-ethoxyimino}-2-phenyl-acetamid,
t) 2-{[4-({2-[3-(3-Ethoxy-4-methoxy-phenyl)-5,6-dihydro-4*H*-pyridazin-1-yl]- 2-oxo-ethoxyimino}-methyl)-phenyl]-hydrazono}-2-(1*H-*tetrazol-5-yl)essigsäurenitril,
sowie ihre pharmazeutisch verwendbaren Salze, Solvate, Stereoisomere und E/Z-Isomeren, einschließlich deren Mischungen in allen Verhältnissen.

13. Verbindungen der Formel I nach einem oder mehreren der Ansprüche 1 bis 12 als Phosphodiesterase IV-Inhibitoren.

14. Verfahren zur Herstellung von Verbindungen der Formel I sowie deren Salzen und Solvaten, **dadurch gekennzeichnet, daß** man
a) eine Verbindung der Formel II 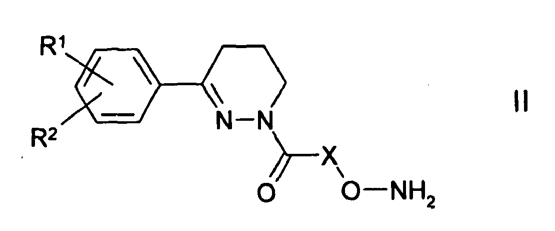 worin
X, R¹ und R² die in Anspruch 1 angegebenen Bedeutungen haben, mit einer Verbindung der Formel III 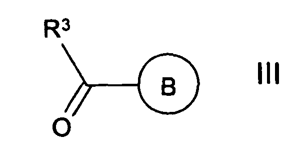 worin
R³ und B die in Anspruch 1 angegebenen Bedeutungen haben,
mit der Maßgabe, daß falls eine weitere OH- und/oder Aminogruppe vorliegt, diese geschützt ist,
umsetzt,
und anschließend gegebenenfalls eine Schutzgruppe abspaltet,
oder
b) eine Verbindung der Formel IV 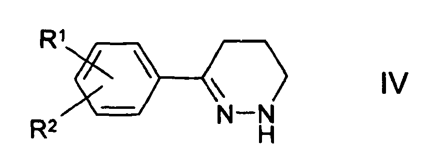 worin
R¹ und R² die in Anspruch 1 angegebenen Bedeutungen haben, mit einer Verbindung der Formel V 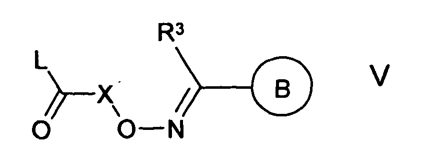 worin
L Cl, Br, I oder eine freie oder reaktionsfähig funktionell abgewandelte OH-Gruppe bedeutet,
und R³, X und B die in Anspruch 1 angegebenen Bedeutungen haben, mit der Maßgabe, daß falls eine weitere OH- und/oder Aminogruppe vorliegt, diese geschützt ist,
umsetzt,
und anschließend gegebenenfalls eine Schutzgruppe abspaltet,
oder
c) eine Verbindung der Formel VI 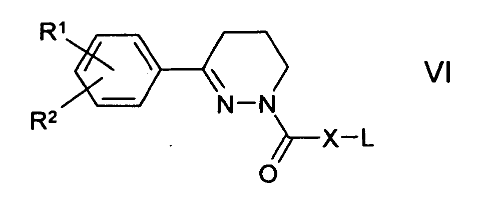 worin
X, R¹ und R² die in Anspruch 1 angegebenen Bedeutungen haben und L Cl, Br, I oder eine freie oder reaktionsfähig funktionell abgewandelte OH-Gruppe bedeutet,
mit einer Verbindung der Formel VII 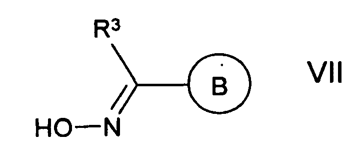 worin R³ und B die in Anspruch 1 angegebenen Bedeutungen haben, mit der Maßgabe, daß falls eine weitere OH- und/oder Aminogruppe vorliegt, diese geschützt ist,
umsetzt,
und anschließend gegebenenfalls eine Schutzgruppe abspaltet,
oder
d) in einer Verbindung der Formel I einen oder mehrere Reste R¹, R² R³ und/oder B in einen oder mehrere andere Reste R¹, R², R³ und/oder B umwandelt, indem man
i) einen Ether oder Ester spaltet,
ii) eine OH-Funktion alkyliert oder acyliert,
iii) eine Aminogruppe reduktiv alkyliert,
iv) eine Aminogruppe mit Malodinitril umsetzt,
v) eine Cyangruppe in eine Tetrazolgruppe überführt,
und/oder daß man eine basische Verbindung der Formel I durch Behandeln mit einer Säure in eines ihrer Salze überführt.

15. Arzneimittel, enthaltend mindestens eine Verbindung der Formel I nach einem oder mehreren der Ansprüche 1 bis 12 und/oder ihre pharmazeutisch verwendbaren Salze, Solvate, Stereoisomere und E/Z-Isomeren, einschließlich deren Mischungen in allen Verhältnissen, sowie gegebenenfalls Träger- und/oder Hilfsstoffe.

16. Verwendung von Verbindungen der Formel I nach einem oder mehreren der Ansprüche 1 bis 12 und/oder ihren physiologisch unbedenklichen Salzen oder Solvaten zur Herstellung eines Arzneimittels zur Behandlung eines Patienten, der an einer durch das PDE IV-Isozym in seiner Rolle bei der Regulierung der Aktivierung und Degranulation von menschlichen Eosinophilen vermittelten Erkrankung oder einem solchen Leiden leidet.

17. Verwendung nach Anspruch 16 von Verbindungen der Formel I nach einem oder mehreren der Ansprüche 1 bis 12 und/oder ihren physiologisch unbedenklichen Salzen oder Solvaten zur Herstellung eines Arzneimittels zur Bekämpfung von allergischen Krankheiten, Asthma, chronischer Bronchitis, atopischer Dermatitis, Psoriasis und anderer Hautkrankheiten, entzündlichen Erkrankungen, Autoimmunerkrankungen, wie z.B. rheumatoide Arthritis, multiple Sklerose, Morbus Crohn, Diabetes mellitus oder ulzerative Kolitis, Osteoporose, Transplantatabstoßungsreaktionen, Kachexie, Tumorwachstum oder Tumor-metastasen, Sepsis, Gedächtnisstörungen, Atherosklerose und AIDS.

18. Verwendung nach Anspruch 16 oder 17 einer Verbindung der Formel I gemäß Ansprüchen 1 bis 12 zur Herstellung eines Arzneimittels zur Behandlung oder Vorbeugung von einer oder mehreren aus der Gruppe der folgenden Erkrankungen, krankhaften Störungen und Leiden:
Asthma jeglicher Art, Ätiologie oder Pathogenese, oder Asthma aus der Gruppe atopisches Asthma, nichtatopisches Asthma, allergisches Asthma, IgE-vermitteltes atopisches Asthma, Bronchialasthma, essentielles Asthma, Primärasthma, durch pathophysiologische Störungen hervorgerufenes endogenes Asthma, durch Umweltfaktoren hervorgerufenes exogenes Asthma, essentielles Asthma unbekannter oder inapparenter Ursache, nichtatopisches Asthma, bronchitisches Asthma, emphysematöses Asthma, durch Belastung induziertes Asthma, Berufsasthma, durch Bakterien-, Pilz-, Protozoen- oder Virusinfektion hervorgerufenes infektallergisches Asthma, nichtallergisches Asthma, inzipientes Asthma, "wheezy infant syndrome";
chronische oder akute Bronchokonstriktion, chronische Bronchitis, Obstruktion der kleinen Atemwege sowie Emphysem;
obstruktive oder entzündliche Atemwegserkrankung jeglicher Art, Ätiologie oder Pathogenese, oder eine obstruktive oder entzündliche Atemwegserkrankung aus der Gruppe Asthma; Staublunge, chronische eosinophile Pneumonie; chronischer obstruktive pulmonaler Krankheit (COPD); COPD inklusive chronische Bronchitis, Lungenemphysem oder damit assoziierte Atemnot, durch irreversible, fortschreitende Obstruktion der Atemwege **gekennzeichnet**e COPD, Schocklunge (adult respiratory distress syndrome, ARDS) sowie Verschärfung der Überempfindlichkeit der Atemwege aufgrund Therapie mit anderen Arzneistoffen;
Staublunge jeglicher Art, Ätiologie oder Pathogenese, oder Staublunge aus der Gruppe Aluminose oder Aluminiumstaublunge, Anthrakose(-Asthma), Asbestose oder Asbeststaublunge, Chalikose oder Kalkstaublunge, durch Einatmen von Straußenfedernstaub verursachte Ptilose, durch Einatmung von Eisenteilchen verursachte Siderose, Silikose oder Steinstaublunge, Byssinose oder Baumwollstaubpneumokoniose sowie Talkpneumokoniose;
Bronchitis jeglicher Art, Ätiologie oder Pathogenese, oder Bronchitis aus der Gruppe akute Bronchitis, akute laryngotracheale Bronchitis, durch Erdnüsse ausgelöste Bronchitis, Bronchialkatarrh, kruppöse Bronchitis, Bronchitis ohne Auswurf, infektiöse Asthmabronchitis, Bronchitis mit Auswurf, Staphylokokken- oder Streptokokkenbronchitis; sowie Vesikulärbronchitis;
Bronchiektasie jeglicher Art, Ätiologie oder Pathogenese, oder Bronchiektasie aus der Gruppe zylindrische Bronchiektasie, sackförmige Bronchiektasie, spindelförmige Bronchiektasie, Bronchiolendilatation, zystische Bronchiektasie, Bronchiektasie ohne Auswurf, sowie follikuläre Bronchiektasie;
jahreszeitlich bedingte allergische Rhinitis, perenniale allergische Rhinitis, oder Sinusitis jeglicher Art, Ätiologie oder Pathogenese, oder Sinusitis aus der Gruppe eitriger oder nichteitriger Sinusitis, akute oder chronische Sinusitis, Ethmoiditis, Stirnhöhlenentzündung, Kieferhöhlenentzündung oder Sphenoiditis;
rheumatoide Arthritis jeglicher Art, Ätiologie oder Pathogenese, oder rheumatoide Arthritis aus der Gruppe akute Arthritis, akute Gichtarthritis, primär-chronische Polyarthritis, Osteoarthrose, Infektarthritis, Lyme-Arthritis, progrediente Arthritis, Arthritis psoriatica, sowie Spondylarthritis;
Gicht sowie mit Entzündung assoziiertes Fieber bzw. mit Entzündung assoziierter Schmerz;
eine mit Eosinophilen in Zusammenhang stehende krankhafte Störung jeglicher Art, Ätiologie oder Pathogenese, oder eine mit Eosinophilen in Zusammenhang stehende krankhafte Störung aus der Gruppe Eosinophilie, eosinophiles Lungeninfiltrat, Löffler-Syndrom, chronische eosinophile Pneumonie, tropische Lungeneosinophilie, bronchopneumonische Aspergillose, Aspergillom, eosinophiles Granulom, allergische granulomatöse Angiitis bzw. Churg-Strauss-Syndrom, Polyarteriitis nodosa (PAN), sowie systemische Vasculitis necroticans;
atopische Dermatitis, allergische Dermatitis, oder allergisches oder atopisches Ekzem;
Nesselsucht jeglicher Art, Ätiologie oder Pathogenese, oder Nesselsucht aus der Gruppe immunbedingte Nesselsucht, Komplementbedingte Nesselsucht, durch Nesselsucht auslösendes Material induzierte Nesselsucht, durch physikalische Reize ausgelöste Nesselsucht, durch Streß ausgelöste Nesselsucht, idiopatische Nesselsucht, akute Nesselsucht, chronische Nesselsucht, angioneurotisches Ödem, Urticaria cholinergica, Kälteurtikaria in ihrer autosomal-dominanten Form oder in ihrer erworbenen Form, Kontakturtikaria, Urticaria gigantean sowie Papelurtikaria;
Konjunktivitis jeglicher Art, Ätiologie oder Pathogenese, oder Konjunktivitis aus der Gruppe Conjunctivitis actinica, akute katarrhalische Konjunktivitis, akute contagiöse Konjunktivitis, allergische Konjunktivitis, atopische Konjunktivitis, chronische katarrhalische Konjunktivitis, eitrige Konjunktivitis sowie Frühjahrskonjunktivitis;
Uveitis jeglicher Art, Ätiologie oder Pathogenese oder Uveitis aus der Gruppe Entzündung der ganzen Uvea oder eines Teils davon, Uveitis anterior, Iritis, Cyclitis, Iridocyclitis, granulomatöse Uveitis, nichtgranulomatöse Uveitis, phakoantigene Uveitis, Uveitis posterior, Choroiditis sowie Choriorethinitis;
Schuppenflechte;
multiple Sklerose jeglicher Art, Ätiologie oder Pathogenese, oder multiple Sklerose aus der Gruppe primär progrediente multiple Sklerose sowie multiple Sklerose mit schubweisem Verlauf und Neigung zu Remissionen;
Autoimmun-/Entzündungserkrankungen jeglicher Art, Ätiologie oder Pathogenese, oder eine Autoimmun-/Entzündungserkrankung aus der Gruppe autoimmunhämatologische Störungen, hämolytische Anämie, aplastische Anämie, aregenerative Anämie, idiopatische thrombozytopene Purpura, systemischer Lupus erythematosus, Polychondritis, Skleroderm, Wegener-Granulomatose, Lichtkrankheit, chronisch-aktive Hepatitis, Myasthenia gravis, Stevens-Johnson-Syndrom, idiopathische Sprue, Autoimmun-Reizkolonerkrankungen, Colitis ulcerosa, Morbus Crohn, endokrine Opthamopathy, Basedow-Krankheit, Sarkoidose, Alveolitis, chronische Hypersensitivitätspneumonitis, primär biliäre Zirrhose, Insulinmangeldiabetes oder Typ 1 Diabetes mellitus, Uveitis anterior, granulomatöse Uveitis oder Uveitis posterior, Keratoconjunctivitis sicca, Keratoconjunctivitis epidemica, (diffuse) interstitielle Lungenfibrose, Lungenzirrhose, Mukoviszidose, Arthritis psoriatica, Glomerulonephritis mit und ohne Nephrose, akute Glomerulonephritis, idiopathische Nephrose, Minimal-Change-Nephropathie, entzündliche/ hyperproliferative Hauterkrankungen, Schuppenflechte, atopische Dermatitis, Kontaktdermatitis, allergische Kontaktdermatitis, familiärer gutartiger Pemphigus, Pemphigus erythematosus, Pemphigus foliaceus sowie Pemphigus vulgaris;
Vorbeugung einer Fremdtransplantatabstoßung nach Organtransplantation,
Reizdarm (inflammatory bowel disease, IBD) jeglicher Art, Ätiologie oder Pathogenese, oder Reizdarm aus der Gruppe ulzerative Kolitis (UC), kollagenöse Kolitis, Colitis polyposa, transmurale Kolitis sowie Morbus Crohn (CD);
septischer Schock jeglicher Art, Ätiologie oder Pathogenese, oder septischer Schock aus der Gruppe Nierenversagen, akutes Nierenversagen, Kachexie, Malariakachexie, hypophysäre Kachexie, uremämische Kachexie, Herzkachexie, Cachexia suprarenalis bzw. Addison-Krankheit, karzinomatöse Kachexie sowie Kachexie auf Grund von Infektion durch Human Immunodeficiency Virus (HIV);
Leberschädigung;
pulmonaler Hochdruck sowie durch Sauerstoffmangel hervorgerufener pulmonaler Hochdruck;
Knochenschwunderkrankungen, primäre Osteoporose und sekundäre Osteoporose;
krankhafte Störungen des Zentralnervensystems jeglicher Art, Ätiologie oder Pathogenese, oder eine krankhafte Störung des Zentralnervensystems aus der Gruppe Depression, Morbus Parkinson, Lern- und Gedächtnisstörungen, tardive Dyskinesie, Drogenabhängigkeit, arteriosklerotische Demenz, sowie Demenz als Begleiterscheinung von Chorea Huntington, Morbus Wilson, Paralysis agitans sowie Thalamusatrophien;
Infektionen, insbesondere Virusinfektionen, wobei diese Viren die Produktion von TNF-α in ihrem Wirt erhöhen oder wobei diese Viren gegenüber Hinaufregulierung von TNF-α in ihrem Wirt empfindlich sind, so daß ihre Replikation oder andere wichtigen Aktivitäten behindert werden, darunter Viren aus der Gruppe HIV-1, HIV-2 und HIV-3, Zytomegalievirus, CMV; Grippe, Adenoviren und Herpesviren, darunter Herpes zoster und Herpes simplex;
Hefe- und Pilzinfektionen, wobei diese Hefen und Pilze gegenüber Hinaufregulierung durch TNF-α empfindlich sind oder die TNF-α-Produktion in ihrem Wirt auslösen, z.B. Pilzmeningitis, insbesondere bei gemeinsamer Verabreichung mit anderen Arzneistoffen der Wahl zur Behandlung systemischer Hefe- und Pilzinfektionen, darunter den Polymycinen, z.B. Polymycin B, Imidazolen, z.B. Clotrimazol, Econazol, Miconazol und Ketoconazol, den Triazolen, z.B. Fluconazol und Itranazol, sowie den Amphotericinen, z.B. Amphotericin B und liposomales Amphotericin B, was jedoch keine Einschränkung darstellen soll.
Ischämie-Reperfusionsschädigung, Autoimmundiabetes, retinale Autoimmunität, chronische lymphozytische Leukämie, HIV-Infektionen, Lupus erythematosus, Nieren- und Harnleitererkrankungen, krankhafte Urogenital- und Gastrointestinalstörungen, sowie Prostataerkrankungen.

19. Verwendung nach Anspruch 16, 17 oder 18 einer Verbindung der Formel I gemäß Ansprüchen 1 bis 12 zur Herstellung eines Arzneimittels zur Behandlung von (1) Entzündungserkrankungen und -leiden, inklusive Gelenkentzündung, rheumatoide Arthritits, rheumatoide Spondylitis, Osteoarthritis, Reizdarm, ulzerative Kolitis, chronische Glomerulonephritis, Dermatitis sowie Morbus Crohn, (2) Erkrankungen und Leiden der Atemwege, inklusive Asthma, Schocklunge, chronische Pulmonitis, Bronchitits, chronische obstruktive Atemwegserkrankung sowie Silikose, (3) Infektionskrankheiten und -leiden inkluisve Sepsis, septischer Schock, endotoxischer Schock, gramnegative Sepsis, toxisches Schocksyndrom, durch Bakterien-, Virus- oder Pilzinfektionen hervorgerufenes Fieber bzw. Myalgie, sowie Grippe; (4) Immunerkrankungen und -leiden, inklusive Autoimmundiabetes, systemischer Lupus erythematosus, GvH-Reaktion, Abstoßung von Fremdtransplantaten, multiple Sklerose, Schuppenflechte und allergische Rhinitis, sowie (5) weitere Erkrankungen und Leiden, darunter Knochenresorptionserkrankungen, Reperfusionsschädigung, sekundäre Kachexie aufgrund Infektion oder Malignität, sekundäre Kachexie aufgrund AIDS, Infektion mit Human Immune Deficiency Virus (HIV), oder AIDS-related-Complex (ARC), Keloidbildung, Narbengewebsbildung, Typ 1 Diabetes mellitus sowie Leukämie.

20. Verwendung nach Anspruch 16 einer Verbindung der Formel I gemäß Ansprüchen 1 bis 12 zur Herstellung eines Arzneimittels zur Behandlung von Myokarderkrankungen.

21. Verwendung nach Anspruch 20 einer Verbindung der Formel I gemäß Ansprüchen 1 bis 12 zur Herstellung eines Arzneimittels zur Behandlung von Myokarderkrankungen, wobei diese Myokarderkrankungen entzündliche und immunologische Eigenschaften aufweisen.

22. Verwendung nach Anspruch 16 einer Verbindung der Formel I gemäß Ansprüchen 1 bis 12 zur Herstellung eines Arzneimittels zur Behandlung von koronarer Herzerkrankung, reversibler oder irreversibler Myokardischämie/Reperfusionsschädigung, akutem oder chronischem Herzversagen und Restenose, darunter In-Stent-Restenose und Stent-in-Stent-Restenose.

23. Kombination einer Verbindung gemäß Ansprüchen 1 bis 12 zusammen mit einer oder mehreren Mitgliedern der folgenden Gruppe:
(a) Leukotriene-Biosyntheseinhibitoren: 5-Lipoxygenase (5-LO)-Inhibitoren und Antagonisten des 5-Lipoxygenase-aktivierenden Proteins (FLAP) aus der Gruppe Zileuton, ABT-761, Fenleuton, Tepoxalin, Abbott-79175, Abbott-85761, N-(5-substituierte) Thiophen-2-alkylsulfonamide, 2,6-di-tert.-Butylphenolhydrazone, Zeneca ZD-2138, SB-210661, die pyridinylsubstituierte 2-Cyannaphthalinverbindung L-739,010, die 2-Cyanchinolinverbindung L-746,530, die Indol- und Chinolinverbindungen MK-591, MK-886 und BAY x 1005;
(b) Rezeptorantagonisten für die Leukotriene LTB₄, LTC₄, LTD₄ und LTE₄ aus der Gruppe der Phenothiazin-3-on-Verbindung L-651,392, der Amidinoverbindung CGS-25019c, der Benzoxazolaminverbindung Ontazolast, der Benzolcarboximidamidverbindung BIIL 284/260, der Verbindungen Zafirlukast, Ablukast, Montelukast, Pranlukast, Verlukast (MK-679), RG-12525, Ro-245913, Iralukast (CGP 45715A) und BAY x 7195;
(c) PDE IV-Inhibitoren;
(d) 5-Lipoxygenase (5-LO)-Inhibitoren; Antagonisten des 5-lipoxygenase-aktivierenden Proteins (FLAP);
(e) Doppelinhibitoren der 5-Lipoxygenase (5-LO) und Antagonisten des blutplättchenaktivierenden Faktors (platelet activating factor PAF);
(f) Leukotrienantagonisten (LTRAs), darunter Antagonisten von LTB₄ LTC₄, LTD₄ und LTE₄;
(g) Antihistamin-H₁-Rezeptorantagonisten, darunter Cetirizin, Loratadin, Desloratadin, Fexofenadin, Astemizol, Azelastin und Chlorpheniramin;
(h) gastroprotektive H₂-Rezeptorantagonisten;
(i) oral oder topisch verabreichte α₁- und α₂-Adrenorezeptor-Agonist-Vasokonstriktor-Sympathomimetika zur Schleimhautabschwellung aus der Gruppe Propylhexedrin, Phenylephrin, Phenylpropanolamin, Pseudoephedrin, Naphazolinhydrochlorid, Oxymetazolinhydrochlorid, Tetrahydrozolinhydrochlorid, Xylometazolinhydrochlorid sowie Ethylnorepinephrinhydrochlorid;
(j) ein oder mehrere α₁- und α₂-Adrenorezeptoragonisten wie oben unter (i) aufgezählt in Kombination mit einem oder mehreren Inhibitoren der 5-Lipoxygenase (5-LO) wie oben unter (a) aufgezählt;
(k) Anticholinergika, darunter Ipratropiumbromid, Tiotropiumbromid, Oxitropiumbromid, Pirenzepin sowie Telenzepin;
(l) β₁- bis β₄-Adrenorezeptoragonisten aus der Gruppe Metaproterenol, Isoproterenol, Isoprenalin, Albuterol, Salbutamol, Formoterol, Salmeterol, Terbutalin, Orciprenalin, Bitolterol und Pirbuterol;
(m) Theophyllin und Aminophyllin;
(n) Natriumcromoglycat;
(o) Muscarinreceptor (M1, M2 und M3)-Aantagonisten;
(p) COX-1-Inhibitoren (NSAIDs) sowie Stickoxid-NSAIDs;
(q) der COX-2-selektive Inhibitoren Rofecoxib;
(r) Mimetika des insulinähnlichen Wachstumsfaktors Typ I (IGF-1);
(s) Ciclesonid;
(t) Inhalations-Glucokortikoide mit verringerten systemischen Nebenwirkungen aus der Gruppe Prednison, Prednisolon, Flunisolid, Triamcinolonacetonid, Beclomethasondipropionat, Budesonid, Fluticasonpropionat sowie Mometasonfuroat;
(u) Tryptaseinhibitoren;
(v) Antagonisten des blutplättchenaktivierenden Faktors (PAF);
(w) monoklonale Antikörper gegen endogene entzündliche Körper;
(x) IPL 576;
(y) Anti-Tumor Nekrose Faktor (TNFα)-Mittel aus der Gruppe Etanercept, Infliximab und D2E7;
(z) DMARDs aus der Gruppe Leflunomid;
(aa) TCR-Peptide;
(bb) Inhibitoren des interleukinumwandelnden Enzyms (interleukin converting enzyme, ICE);
(cc) IMPDH-Inhibitoren;
(dd) Adhäsionsmolekülinhibitoren, darunter VLA-4-Antagonisten;
(ee) Kathepsine;
(ff) MAP-Kinaseinhibitoren;
(gg) Glucose-6-phosphat-dehydrogenase-Inhibitoren;
(hh) Kinin-B₁- und -B₂-Rezeptor-Antagonisten;
(ii) Gold in Form einer Aurothiogruppe zusammen mit verschiedenen hydrophilen Gruppen;
(jj) Immunosuppressiva aus der Gruppe Cyclosporin, Azathioprin und Methotrexat;
(kk) Mittel gegen Gicht aus der Gruppe Kolchizine;
(ll) Xanthinoxidaseinhibitoren aus der Gruppe Allopurinol;
(mm) Urikosurika aus der Gruppe Probenecid, Sulfinpyrazon und Benzbromaron;
(nn) Antineoplastica, bei denen es sich um antimitotische Arzneistoffe aus der Gruppe Vinblastin und Vincristin handelt;
(oo) Mittel zur Förderung der Wachstumshormonsekretion;
(pp) Inhibitoren der Matrixmetalloproteasen (MMPs) aus der Gruppe Stromelysine, Kollagenasen, Gelatinasen, Aggrecanase, Kollagenase-1 (MMP-1), Kollagenase-2 (MMP-8), Kollagenase-3 (MMP-13), Stromelysin-1 (MMP-3), Stromelysin-2 (MMP-10) und Stromelysin-3 (MMP-11);
(qq) "transforming growth factor" (TGFβ);
(rr) "platelet-derived growth factor" (PDGF);
(ss) Fibroblasten-Wachstumsfaktor aus der Gruppe "basic fibroblast growth factor" (bFGF);
(tt) "granulocyte macrophage colony stimulating factor" (GM-CSF);
(uu) Capsaicin;
(w) Tachykinin-NK₁- und -NK₃-Rezeptor-Antagonisten aus der Gruppe NKP-608C; SB233412 (Talnetant) und D-4418;
(ww) Elastaseinhibitoren aus der Gruppe UT-77 und ZD-0892;
sowie
(xx) Adenosin-A2a-Rezeptoragonisten.

24. Arzneimittel mit mindestens einer Verbindung der Formel I nach einem oder mehreren der Ansprüche 1 bis 12 und/oder ihren pharmazeutisch verwendbare Salzen, Solvaten und Stereoisomeren, einschließlich deren Mischungen in allen Verhältnissen, sowie mindestens einem weiteren Arzneimittelwirkstoff.

25. Set (Kit) bestehend aus getrennten Packungen
(a) einer wirksamen Menge einer Verbindung der Formel I nach einem oder mehreren der Ansprüche 1 bis 12 und/oder ihren pharmazeutisch verwendbaren Derivaten, Salzen und Stereoisomeren, einschließlich deren Mischungen in allen Verhältnissen,
und
(b) einer wirksamen Menge eines weiteren Arzneimittelwirkstoffs.

## Claims

1. Compounds of the formula I 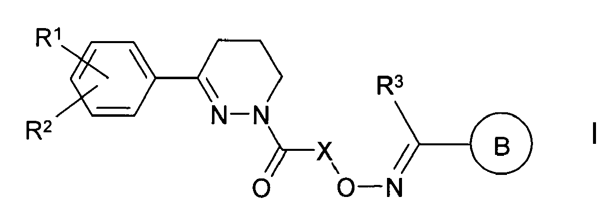 in which
R¹, R² each, independently of one another, denote H, OH, OR⁸, -SR⁸, -SOR⁸, -SO₂R⁸ or Hal,
R¹ and R² together also denote -OCH₂O- or -OCH₂CH₂O-,
R³ denotes H, A"R⁷, COA"R⁷, COOA"R⁷, CONH₂, CONHA"R⁷, CON(A"R⁷)(A"'R⁷), CONR¹⁰Het, NH₂, NHA"R⁷, N(A"R⁷)(A"'R⁷), NCOA"R⁷ or NCOOA"R⁷,
B denotes phenyl, pyridyl, pyridyl N-oxide, thienyl, furyl, pyrrolyl, pyridazinyl, pyrimidinyl, pyrazinyl, triazinyl, isoxazolinyl, oxazolinyl, thiazolinyl, pyrazolinyl, imidazolinyl, naphthyl, quinolinyl, isoquinolinyl, cinnolinyl, phthalazinyl, quinazolinyl or quinoxalinyl, each of which is unsubstituted or may be mono-, di- or trisubstituted by R⁴, R⁵ and/or R⁶,
X denotes alkylene having 1-10 C atoms or alkenylene having 2-8 C atoms,
in which one, two or three CH₂ groups may be replaced by O, S, SO, SO₂, NH or NA"R⁷,
1-7 H atoms may be replaced by F and/or Cl,
R⁴, R⁵, R⁶ each, independently of one another, denote H, A"R⁷, OH, OA"R⁷, NO₂, NH₂, NHA"R⁷, N(A"R⁷)(A"'R⁷), NHCOA"R⁷, NHCOOA"R⁷, NHCONH₂, NHCONHA"R⁷, NHCON(A"R⁷)(A"'R⁷), Hal, COOH, COOA"R⁷, CONH₂, CONHA"R⁷, CON(A"R⁷)(A"'R⁷), 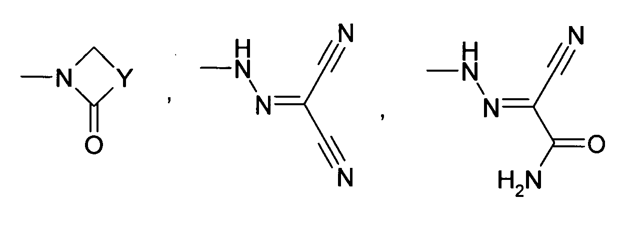 or 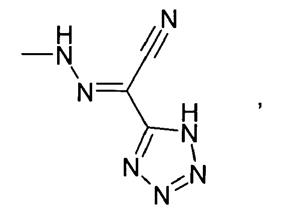
R⁷ denotes H, COOH, COOA, CONH₂, CONHA, CONAA', NH₂, NHA, NAA', NCOA, NCOOA, OH or OA,
R⁸ denotes A, cycloalkyl having 3-7 C atoms, alkylenecycloalkyl having 4-8 C atoms or alkenyl having 2-8 C atoms,
R⁹ denotes alkyl having 1-10 C atoms, cycloalkyl having 3-7 C atoms, alkylenecycloalkyl having 4-8 C atoms or alkenyl having 2-8 C atoms,
in which one, two or three CH₂ groups may be replaced by O, S, SO, SO₂, NH, NMe, NEt and/or by -CH=CH- groups,
1-7 H atoms may be replaced by F and/or Cl
and/or 1 H atom may be replaced by R⁷,
Y denotes alkylene having 1-10 C atoms or alkenylene having 2-8 C atoms,
in which one, two or three CH₂ groups may be replaced by O, S, SO, SO₂, NH or NR⁹ and/or
1-7 H atoms may be replaced by F and/or Cl,
A, A' each, independently of one another, denote alkyl having 1-10 C atoms or alkenyl having 2-8 C atoms,
in which one, two or three CH₂ groups may be replaced by O, S, SO, SO₂, NH or NR⁹ and/or
1-7 H atoms may be replaced by F and/or Cl,
or
aryl or Het,
A and A' together also denote an alkylene chain having 2-7 C atoms, in which one, two or three CH₂ groups may be replaced by O, S, SO, SO₂, NH, NR⁹, NCOR⁹ or NCOOR⁹,
A", A"' are each, independently of one another,
absent, denote alkylene having 1-10 C atoms, alkenylene having 2-8 C atoms or cycloalkylene having 3-7 C atoms, in which one, two or three CH₂ groups may be replaced by O, S, SO, SO₂, NH or NR⁹ and/or
1-7 H atoms may be replaced by F and/or Cl,
A" and A"' together also denote an alkylene chain having 2-7 C atoms, in which one, two or three CH₂ groups may be replaced by O, S, SO, SO₂, NH, NR⁹, NCOR⁹ or NCOOR⁹,
aryl denotes phenyl, naphthyl, fluorenyl or biphenyl, each of which is unsubstituted or mono-, di- or trisubstituted by Hal, R¹¹, OR¹⁰, N(R¹⁰)₂, NO₂, CN, COOR¹⁰, CON(R¹⁰)₂, NR¹⁰COR¹⁰, NR¹⁰CON(R¹⁰)₂, NR¹⁰SO₂A, COR¹⁰, SO₂N(R¹⁰)₂, S(O)ₘR¹¹,
R¹⁰ denotes H or alkyl having 1-6 C atoms,
R¹¹ denotes alkyl having 1-6 C atoms,
Het denotes a mono- or bicyclic saturated, unsaturated or aro- matic heterocyclic ring having 1 to 2 N, O and/or S atoms, which may be unsubstituted or mono- or disubstituted by carbonyl oxygen, Hal, R¹¹, OR¹⁰, N(R¹⁰)₂, NO₂, CN, COOR¹⁰, CON(R¹⁰)₂, NR¹⁰COR¹⁰, NR¹⁰CON(R¹⁰)₂, NR¹⁰SO₂R¹¹, COR¹⁰, SO₂NR¹⁰ and/or S(O)ₘR¹¹,
Hal denotes F, Cl, Br or I,
m denotes 0, 1 or 2,
and pharmaceutically usable salts, solvates, stereoisomers and E/Z isomers thereof, including mixtures thereof in all ratios.

2. Compounds according to Claim 1 in which
R¹, R² each, independently of one another, denote alkoxy having 1, 2, 3, 4, 5 or 6 C atoms,
and pharmaceutically usable salts, solvates, stereoisomers and E/Z isomers thereof, including mixtures thereof in all ratios.

3. Compounds according to Claim 1, in which
R¹, R² each, independently of one another, denote H, methoxy, ethoxy, benzyloxy, propoxy, isopropoxy, difluoromethoxy, F, Cl, cyclopentyloxy, cyclohexyloxy or cycloheptyloxy,
and pharmaceutically usable salts, solvates, stereoisomers and E/Z isomers thereof, including mixtures thereof in all ratios.

4. Compounds according to Claim 1, in which
R¹, R² each, independently of one another, denote methoxy, ethoxy, pro- poxy, isopropoxy, cyclopentyloxy or F,
and pharmaceutically usable salts, solvates, stereoisomers and E/Z isomers thereof, including mixtures thereof in all ratios.

5. Compounds according to one or more of Claims 1-4,
in which
R¹ denotes 4-methoxy,
R² denotes 3-ethoxy or 3-propoxy,
and pharmaceutically usable salts, solvates, stereoisomers and E/Z isomers thereof, including mixtures thereof in all ratios.

6. Compounds according to one or more of Claims 1-5,
in which
R³ denotes H, A"R⁷, COA"R⁷, CON(A"R⁷)(A"'R⁷) or CO-NR¹⁰-Het,
and pharmaceutically usable salts, solvates, stereoisomers and E/Z isomers thereof, including mixtures thereof in all ratios.

7. Compounds according to one or more of Claims 1-6,
in which
X denotes methylene, ethylene, propylene or butylene,
and pharmaceutically usable salts, solvates, stereoisomers and E/Z isomers thereof, including mixtures thereof in all ratios.

8. Compounds according to one or more of Claims 1-7,
in which
B denotes phenyl, pyridyl, pyridyl N-oxide, thienyl, furyl, pyrrolyl, pyridazinyl, pyrimidinyl, pyrazinyl, triazinyl, isoxazolinyl, oxazolinyl, thiazolinyl, pyrazolinyl, imidazolinyl, naphthyl, quinolinyl, isoquino- linyl, cinnolinyl, phthalazinyl, quinazolinyl or quinoxalinyl, each of which is unsubstituted or may be mono-, di- or trisubstituted by OH, OA, NO₂, NH₂, NAA', 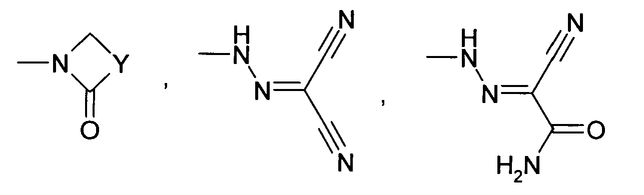 or 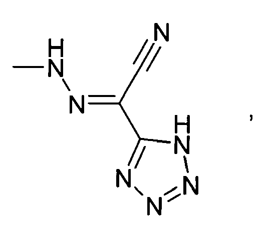
and pharmaceutically usable salts, solvates, stereoisomers and E/Z isomers thereof, including mixtures thereof in all ratios.

9. Compounds according to one or more of Claims 1-8,
in which
B denotes phenyl which is unsubstituted or monosubstituted by OR¹⁰, NO₂ or 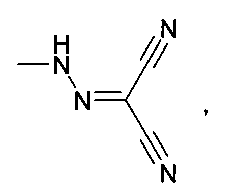 or unsubstituted pyridyl or pyridyl N-oxide,
and pharmaceutically usable salts, solvates, stereoisomers and E/Z isomers thereof, including mixtures thereof in all ratios.

10. Compounds according to one or more of Claims 1-9,
in which
R¹, R² each, independently of one another, denote alkoxy having 1, 2, 3, 4, 5 or 6 C atoms,
X denotes methylene, ethylene, propylene or butylene,
R³ denotes H, A"R⁷, COA"R⁷, CON(A'R⁷)(A"'R⁷) or CO-NR¹⁰-Het,
A", A"' are each, independently of one another, absent or denote alkylene having 1-10 C atoms, in which one CH₂ group may be replaced by NH or NR⁹,
A", A"' together also denote an alkylene chain having 2-7 C atoms, in which one CH₂ group may be replaced by NH or NR⁹,
B denotes phenyl which is unsubstituted or monosubstituted by OR¹⁰, NO₂, 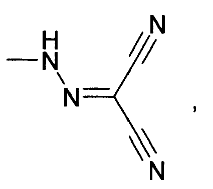 NH₂ or NHCOOA"R⁷, or unsubstituted pyridyl or pyridyl N-oxide,
R⁷ denotes H, COOH, NHA or NAA',
R⁹ denotes alkyl having 1-6 C atoms,
R¹⁰ denotes H or alkyl having 1-6 C atoms,
A, A' each, independently of one another, denote alkyl having 1-10 C atoms, in which 1-7 H atoms may be replaced by F and/or Cl,
Het denotes a monocyclic saturated heterocyclic radical having 1 to 2 N atoms, which may be mono- or disubstituted by alkyl having 1-6 C atoms,
and pharmaceutically usable salts, solvates, stereoisomers and E/Z isomers thereof, including mixtures thereof in all ratios.

11. Compounds according to one or more of Claims 1-10,
in which
R¹, R² each, independently of one another, denote alkoxy having 1, 2, 3, 4, 5 or 6 C atoms,
X denotes methylene, ethylene, propylene or butylene,
R³ denotes H, A"R⁷, COA"R⁷, CON(A"R⁷)(A"'R⁷) or CO-NR¹⁰-Het,
A", A"' are each, independently of one another, absent or denote alkylene having 1-10 C atoms, in which one CH₂ group may be replaced by NH or NR⁹,
A", A"' together also denote an alkylene chain having 2-7 C atoms, in which one CH₂ group may be replaced by NH or NR⁹,
B denotes phenyl which is unsubstituted or monosubstituted by OR¹⁰, NO₂, 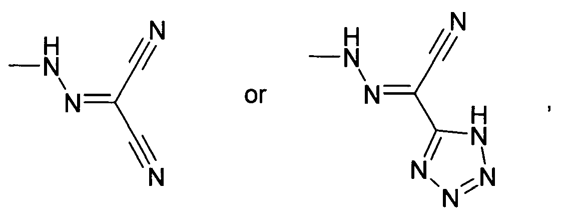 NH₂ or NHCOOA"R⁷, or unsubstituted pyridyl or pyridyl N-oxide,
R⁷ denotes H, COOH, NHA or NAA',
R⁹ denotes alkyl having 1-6 C atoms,
R¹⁰ denotes H or alkyl having 1-6 C atoms,
A, A' each, independently of one another, denote alkyl having 1-10 C atoms, in which 1-7 H atoms may be replaced by F and/or Cl,
Het denotes a monocyclic saturated heterocyclic radical having 1 to 2 N atoms, which may be mono- or disubstituted by alkyl having 1-6 C atoms,
and pharmaceutically usable salts, solvates, stereoisomers and E/Z isomers thereof, including mixtures thereof in all ratios.

12. Compounds of the formula I according to Claim 1, from the group
a) 4-methoxybenzaldehyde O-{2-[3-(3-ethoxy-4-methoxyphenyl)-5,6-dihydro-4H-pyridazin-1-yl]-2-oxoethyl} oxime,
b) benzaldehyde O-{2-[3-(3-ethoxy-4-methoxyphenyl)-5,6-dihydro-4H-pyridazin-1-yl]-2-oxoethyl} oxime,
c) 4-hydroxybenzaldehyde O-{2-[3-(3-ethoxy-4-methoxyphenyl)-5,6-dihydro-4H-pyridazin-1-yl]-2-oxoethyl} oxime,
d) pyridine-4-carbaldehyde O-{2-[3-(3-ethoxy-4-methoxyphenyl)-5,6-dihydro-4H-pyridazin-1-yl]-2-oxoethyl} oxime,
e) 1-oxypyridine-4-carbaldehyde O-{2-[3-(3-ethoxy-4-methoxyphenyl)-5,6-dihydro-4H-pyridazin-1-yl]-2-oxoethyl} oxime,
f) 4-methoxybenzaldehyde O-{2-[3-(4-methoxy-3-propoxyphenyl)-5,6-dihydro-4H-pyridazin-1-yl]-2-oxoethyl} oxime,
g) benzaldehyde O-{2-[3-(4-methoxy-3-propoxyphenyl)-5,6-dihydro-4H-pyridazin-1-yl]-2-oxoethyl} oxime,
h) pyridine-4-carbaldehyde O-{2-[3-(4-methoxy-3-propoxyphenyl)-5,6-dihydro-4H-pyridazin-1-yl]-2-oxoethyl} oxime,
i) 1-oxypyridine-4-carbaldehyde O-{2-[3-(4-methoxy-3-propoxyphenyl)-5,6-dihydro-4H-pyridazin-1-yl]-2-oxoethyl} oxime,
j) 4-nitrobenzaldehyde O-{2-[3-(4-methoxy-3-propoxyphenyl)-5,6-dihydro-4H-pyridazin-1-yl]-2-oxoethyl} oxime,
k) 4-aminobenzaldehyde O-{2-[3-(4-methoxy-3-propoxyphenyl)-5,6-dihydro-4H-pyridazin-1-yl]-2-oxoethyl} oxime,
l) 4-tert-butyloxycarbonylaminobenzaldehyde O-{2-[3-(4-methoxy-3-propoxyphenyl)-5,6-dihydro-4H-pyridazin-1-yl]-2-oxoethyl} oxime,
m) 2-{[4-({2-[3-(3-ethoxy-4-methoxyphenyl)-5,6-dihydro-4H-pyridazin-1-yl]-2-oxoethoxyimino}methyl)phenyl]hydrazono}malononitrile,
n) 2-{[3-({2-[3-(3-ethoxy-4-methoxyphenyl)-5,6-dihydro-4H-pyridazin-1-yl]-2-oxoethoxyimino}methyl)phenyl]hydrazono}malononitrile,
o) 2-{[4-({2-[3-(4-methoxy-3-propoxyphenyl)-5,6-dihydro-4H-pyridazin-1-yl]-2-oxoethoxyimino}methyl)phenyl]hydrazono}malononitrile,
p) {2-[3-(3-ethoxy-4-methoxyphenyl)-5,6-dihydro-4H-pyridazin-1-yl]-2-oxoethoxyiminol-2-phenylacetic acid,
q) 2-{2-[3-(3-ethoxy-4-methoxyphenyl)-5,6-dihydro-4H-pyridazin-1-yl]-2-oxoethoxyimino}-N-methyl-N-(1-methylpiperidin-4-yl)-2-phenylacetamide,
r) 1-(4-methylpiperazin-1-yl)-2-phenylethane-1,2-dione 2-(O-{2-[3-(3-ethoxy-4-methoxyphenyl)-5,6-dihydro-4H-pyridazin-1-yl]-2-oxoethyl} oxime,
s) N-(2-dimethylaminoethyl)-2-{2-[3-(3-ethoxy-4-methoxyphenyl)-5,6-dihydro-4H-pyridazin-1-yl]-2-oxoethoxyimino}-2-phenylacetamide,
t) 2-{[4-({2-[3-(3-ethoxy-4-methoxyphenyl)-5,6-dihydro-4H-pyridazin-1-yl]-2-oxoethoxyimino}methyl)phenyl]hydrazono}-2-(1*H*-tetrazol-5-yl)acetonitrile,
and pharmaceutically usable salts, solvates, stereoisomers and E/Z isomers thereof, including mixtures thereof in all ratios.

13. Compounds of the formula I according to one or more of Claims 1 to 12 as phosphodiesterase IV inhibitors.

14. Process for the preparation of compounds of the formula I and salts and solvates thereof, **characterised in that**
a) a compound of the formula II 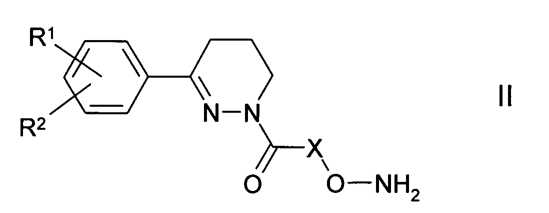 in which
X, R¹ and R² have the meanings indicated in Claim 1,
is reacted with a compound of the formula III 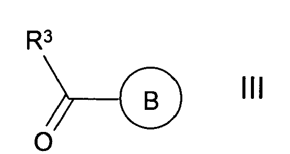 in which
R³ and B have the meanings indicated in Claim 1,
with the proviso that, if a further OH and/or amino group is present, this is protected,
and subsequently a protecting group is optionally cleaved off,
or
b) a compound of the formula IV 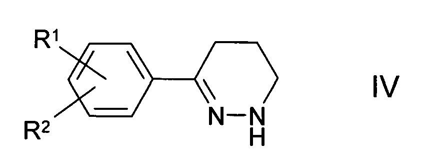 in which
R¹ and R² have the meanings indicated in Claim 1,
is reacted with a compound of the formula V 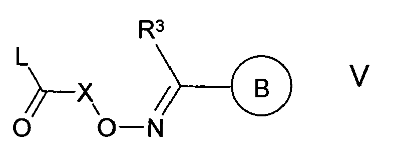 in which
L denotes Cl, Br, I or a free or reactively functionally modified OH group, and R³, X and B have the meanings indicated in Claim 1,
with the proviso that, if a further OH and/or amino group is present, this is protected,
and subsequently a protecting group is optionally cleaved off,
or
c) a compound of the formula VI 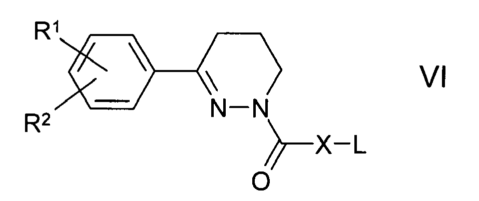 in which
X, R¹ and R² have the meanings indicated in Claim 1, and
L denotes Cl, Br, I or a free or reactively functionally modified OH group, is reacted with a compound of the formula VII 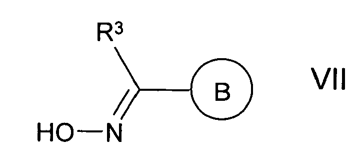 in which R³ and B have the meanings indicated in Claim 1,
with the proviso that, if a further OH and/or amino group is present, this is protected,
and subsequently a protecting group is optionally cleaved off,
or
d) one or more radicals R¹, R², R³ and/or B in a compound of the formula I are converted into one or more other radicals R¹, R², R³ and/or B by
i) cleaving an ether or ester,
ii) alkylating or acylating an OH function,
iii) reductively alkylating an amino group,
iv) reacting an amino group with malononitrile,
v) converting a cyano group into a tetrazole group,
and/or **in that** a basic compound of the formula I is converted into one of its salts by treatment with an acid.

15. Medicaments comprising at least one compound of the formula I according to one or more of Claims 1 to 12 and/or pharmaceutically usable salts, solvates, stereoisomers and E/Z isomers thereof, including mixtures thereof in all ratios, and, if desired, excipients and/or adjuvants.

16. Use of compounds of the formula I according to one or more of Claims 1 to 12 and/or physiologically acceptable salts or solvates thereof for the preparation of a medicament for the treatment of a patient suffering from a disease or complaint caused by the PDE IV isozyme in its role in regulating the activation and degranulation of human eosinophils.

17. Use according to Claim 16 of compounds of the formula I according to one or more of Claims 1 to 12 and/or physiologically acceptable salts or solvates thereof for the preparation of a medicament for combating allergic diseases, asthma, chronic bronchitis, atopic dermatitis, psoriasis and other skin diseases, inflammatory diseases, autoimmune diseases, such as, for example, rheumatoid arthritis, multiple sclerosis, Crohn's disease, diabetes mellitus or ulcerative colitis, osteoporosis, transplant rejection reactions, cachexia, tumour growth or tumour metastases, sepsis, memory disorders, atherosclerosis and AIDS.

18. Use according to Claim 16 or 17 of a compound of the formula I according to Claims 1 to 12 for the preparation of a medicament for the treatment or prevention of one or more diseases, pathological disorders and conditions from the following group:
asthma of whatever type, etiology or pathogenesis, or asthma from the group atopic asthma, non-atopic asthma, allergic asthma, atopic IgE-mediated asthma, bronchial asthma, essential asthma, true asthma, intrinsic asthma caused by pathophysiological disturbances, extrinsic asthma caused by environmental factors, essential asthma of unknown or unapparent cause, non-atopic asthma, bronchitic asthma, emphysematous asthma, exercise-induced asthma, occupational asthma, infective asthma caused by bacterial, fungal, protozoic or viral infection, non-allergic asthma, incipient asthma, wheezy infant syndrome;
chronic or acute bronchoconstriction, chronic bronchitis, small airway obstruction and emphysema;
obstructive or inflammatory airway disease of whatever type, etiology or pathogenesis, or an obstructive or inflammatory airway disease from the group asthma, pneumoconiosis, chronic eosinophilic pneumonia, chronic obstructive pulmonary disease (COPD), COPD including chronic bronchitis, pulmonary emphysema or dyspnoea associated therewith, COPD that is **characterised by** irreversible, progressive airway obstruction, adult respiratory distress syndrome (ARDS), and exacerbation of airway hypersensitivity consequent to other medicament therapy;
pneumoconiosis of whatever type, etiology or pathogenesis, or pneumoconiosis from the group aluminosis or bauxite workers' disease, anthracosis or miners' asthma, asbestosis or steam-fitters' asthma, chalicosis or flint disease, ptilosis caused by inhaling the dust from ostrich feathers, siderosis caused by the inhalation of iron particles, silicosis or grinders' disease, byssinosis or cotton-dust pneumoconiosis and talc pneumoconiosis;
bronchitis of whatever type, etiology or pathogenesis, or bronchitis from the group acute bronchitis, acute laryngotracheal bronchitis, arachidic bronchitis, catarrhal bronchitis, croupous bronchitis, dry bronchitis, infectious asthmatic bronchitis, productive bronchitis, staphylococcal or streptococcal bronchitis and vesicular bronchitis;
bronchiectasis of whatever type, etiology or pathogenesis, or bronchiectasis from the group cylindric bronchiectasis, sacculated bronchiectasis, fusiform bronchiectasis, capillary bronchiectasis, cystic bronchiectasis, dry bronchiectasis and follicular bronchiectasis;
seasonal allergic rhinitis, perennial allergic rhinitis, or sinusitis of whatever type, etiology or pathogenesis, or sinusitis from the group purulent or non purulent sinusitis, acute or chronic sinusitis, and ethmoid, frontal, maxillary or sphenoid sinusitis;
rheumatoid arthritis of whatever type, etiology or pathogenesis, or rheumatoid arthritis from the group acute arthritis, acute gouty arthritis, primary chronic arthritis, osteoarthritis, infectious arthritis, Lyme arthritis, progressive arthritis, psoriatic arthritis and vertebral arthritis;
gout, and fever and pain associated with inflammation;
an eosinophil-related pathological disorder of whatever type, etiology or pathogenesis, or an eosinophil-related pathological disorder from the group eosinophilia, pulmonary infiltration eosinophilia, Löffler's syndrome, chronic eosinophilic pneumonia, tropical pulmonary eosinophilia, bronchopneumonic aspergillosis, aspergilloma, eosinophilic granuloma, allergic granulomatous angiitis or Churg-Strauss syndrome, polyarteritis nodosa (PAN) and systemic necrotising vasculitis;
atopic dermatitis, allergic dermatitis, or allergic or atopic eczema;
urticaria of whatever type, etiology or pathogenesis, or urticaria from the group immune-mediated urticaria, complement-mediated urticaria, urticariogenic material-induced urticaria, physical stimulus-induced urticaria, stress-induced urticaria, idiopathic urticaria, acute urticaria, chronic urticaria, angiooedema, cholinergic urticaria, cold urticaria in the autosomal dominant form or in the acquired form, contact urticaria, giant urticaria and papular urticaria;
conjunctivitis of whatever type, etiology or pathogenesis, or conjunctivitis from the group actinic conjunctivitis, acute catarrhal conjunctivitis, acute contagious conjunctivitis, allergic conjunctivitis, atopic conjunctivitis, chronic catarrhal conjunctivitis, purulent conjunctivitis and vernal conjunctivitis;
uveitis of whatever type, etiology or pathogenesis, or uveitis from the group inflammation of all or part of the uvea, anterior uveitis, iritis, cyclitis, iridocyclitis, granulomatous uveitis, nongranulomatous uveitis, phacoantigenic uveitis, posterior uveitis, choroiditis and chorioretinitis;
psoriasis;
multiple sclerosis of whatever type, etiology or pathogenesis, or multiple sclerosis from the group primary progressive multiple sclerosis and relapsing/remitting multiple sclerosis;
autoimmune/inflammatory diseases of whatever type, etiology or pathogenesis, or an autoimmune/inflammatory disease from the group autoimmune haematological disorders, haemolytic anaemia, aplastic anaemia, aregenerative anaemia, idiopathic thrombocytopenic purpura, systemic lupus erythematosus, polychondritis, scleroderma, Wegener's granulomatosis, photodermatosis, chronic active hepatitis, myasthenia gravis, Stevens-Johnson syndrome, idiopathic sprue, autoimmune inflammatory bowel diseases, ulcerative colitis, Crohn's disease, endocrine ophthamopathy, Graves' disease, sarcoidosis, alveolitis, chronic hypersensitivity pneumonitis, primary biliary cirrhosis, insulin deficiency diabetes or type 1 diabetes mellitus, anterior uveitis, granulomatous or posterior uveitis, keratoconjunctivitis sicca, epidemic keratoconjunctivitis, (diffuse) interstitial pulmonary fibrosis, pulmonary cirrhosis, cystic fibrosis, psoriatic arthritis, glomerulonephritis with and without nephrosis, acute glomerulonephritis, idiopathic nephrosis syndrome, minimal change nephropathy, inflammatory/hyperproliferative skin diseases, psoriasis, atopic dermatitis, contact dermatitis, allergic contact dermatitis, benign familial pemphigus, pemphigus erythematosus, pemphigus foliaceus and pemphigus vulgaris;
prevention of foreign transplant rejection following organ transplantation;
inflammatory bowel disease (IBD) of whatever type, etiology or pathogenesis, or inflammatory bowel disease from the group ulcerative colitis (UC), collagenous colitis, colitis polyposa, transmural colitis and Crohn's disease (CD);
septic shock of whatever type, etiology or pathogenesis, or septic shock from the group renal failure, acute renal failure, cachexia, malarial cachexia, hypophysial cachexia, uremic cachexia, cardiac cachexia, cachexia suprarenalis or Addison's disease, cancerous cachexia, and cachexia as a consequence of infection by the human immunodeficiency virus (HIV);
liver damage;
pulmonary hypertension and hypoxia-induced pulmonary hypertension;
bone loss diseases, primary osteoporosis and secondary osteoporosis;
pathological disorders of the central nervous system of whatever type, etiology or pathogenesis, or a pathological disorder of the central nervous system from the group depression, Parkinson's disease, learning and memory disorders, tardive dyskinesia, drug dependence, arteriosclerotic dementia, and dementia accompanying Huntington's disease, Wilson's disease, paralysis agitans and thalamic atrophies;
infections, especially viral infections, where these viruses increase the production of TNF-α in their host or where these viruses are sensitive to up-regulation of TNF-α in their host so that their replication or other vital activities are adversely affected, including viruses from the group HIV-1, HIV-2 and HIV-3, cytomegalovirus, CMV, influenza, adenoviruses and Herpes viruses, including Herpes zoster and Herpes simplex;
yeast and fungal infections, where these yeasts and fungi are sensitive to up-regulation by TNF-α or elicit TNF-α production in their host, for example fungal meningitis, particularly when administered in conjunction with other medicaments of choice for the treatment of systemic yeast and fungal infections, including, but not limited to, polymycins, for example polymycin B, imidazoles, for example clotrimazole, econazole, miconazole and ketoconazole, triazoles, for example fluconazole and itranazole, and amphotericins, for example amphotericin B and liposomal amphotericin B;
ischaemia-reperfusion damage, autoimmune diabetes, retinal autoimmunity, chronic lymphocytic leukaemia, HIV infections, lupus erythematosus, kidney and ureter diseases, pathological urogenital and gastrointestinal disorders, and prostate diseases.

19. Use according to Claim 16, 17 or 18 of a compound of the formula I according to Claims 1 to 12 for the preparation of a medicament for the treatment of (1) inflammatory diseases and conditions, including joint inflammation, rheumatoid arthritis, rheumatoid spondylitis, osteoarthritis, inflammatory bowel disease, ulcerative colitis, chronic glomerulonephritis, dermatitis and Crohn's disease, (2) respiratory tract diseases and conditions, including asthma, adult respiratory distress syndrome, chronic pulmonitis, bronchitis, chronic obstructive airway disease and silicosis, (3) infectious diseases and conditions, including sepsis, septic shock, endotoxic shock, Gram-negative sepsis, toxic shock syndrome, fever and myalgia due to bacterial, viral or fungal infections, and influenza, (4) immune diseases and conditions, including autoimmune diabetes, systemic lupus erythematosus, GvH reaction, rejection of foreign transplants, multiple sclerosis, psoriasis and allergic rhinitis, and (5) other diseases and conditions, including bone absorption diseases, reperfusion damage, cachexia secondary to infection or malignancy, cachexia secondary to AIDS, human immunodeficiency virus (HIV) infection, or AIDS-related complex (ARC), keloid formation, scar tissue formation, type 1 diabetes mellitus and leukaemia.

20. Use according to Claim 16 of a compound of the formula I according to Claims 1 to 12 for the preparation of a medicament for the treatment of myocardial diseases.

21. Use according to Claim 20 of a compound of the formula I according to Claims 1 to 12 for the preparation of a medicament for the treatment of myocardial diseases, where these myocardial diseases have inflammatory and immunological properties.

22. Use according to Claim 16 of a compound of the formula I according to Claims 1 to 12 for the preparation of a medicament for the treatment of coronary heart disease, reversible or irreversible myocardial ischaemia/reperfusion damage, acute or chronic heart failure and restenosis, including in-stent restenosis and stent-in-stent restenosis.

23. Combination of a compound according to Claims 1 to 12 together with one or more members of the following group:
(a) leukotriene biosynthesis inhibitors: 5-lipoxygenase (5-LO) inhibitors and antagonists of 5-lipoxygenase-activating protein (FLAP) from the group zileuton, ABT-761, fenleuton, tepoxalin, Abbott-79175, Abbott-85761, N-(5-substituted) thiophene-2-alkylsulfonamides, 2,6-di-tert-butylphenol hydrazones, Zeneca ZD-2138, SB-210661, the pyridinyl-substituted 2-cyanonaphthalene compound L-739,010, the 2-cyanoquinoline compound L-746,530, the indole and quinoline compounds MK-591, MK-886 and BAY x 1005;
(b) receptor antagonists for the leukotrienes LTB₄, LTC₄, LTD₄ and LTE₄ from the group of the phenothiazin-3-one compound L-651,392, the amidino compound CGS-25019c, the benzoxazolamine compound ontazolast, the benzenecarboximidamide compound BIIL 284/260, the compounds zafirlukast, ablukast, montelukast, pranlukast, verlukast (MK-679), RG-12525, Ro-245913, iralukast (CGP 45715A) and BAY x 7195;
(c) PDE IV inhibitors;
(d) 5-lipoxygenase (5-LO) inhibitors, antagonists of 5-lipoxygenase-activating protein (FLAP);
(e) dual inhibitors of 5-lipoxygenase (5-LO) and antagonists of platelet activating factor (PAF);
(f) leukotriene antagonists (LTRAs), including LTB₄, LTC₄, LTD₄ and LTE₄ antagonists;
(g) antihistamine H₁ receptor antagonists, including cetirizine, loratadine, desloratadine, fexofenadine, astemizole, azelastine and chlorpheniramine;
(h) gastroprotective H₂ receptor antagonists;
(i) α₁- and α₂-adrenoreceptor agonist vasoconstrictor sympathomimetic agents administered orally or topically for decongestant use, from the group propylhexedrine, phenylephrine, phenylpropanolamine, pseudoephedrine, naphazoline hydrochloride, oxymetazoline hydrochloride, tetrahydrozoline hydrochloride, xylometazoline hydrochloride and ethylnorepinephrine hydrochloride;
(j) one or more α₁- and α₂-adrenoreceptor agonists as listed above under (i) in combination with one or more inhibitors of 5-lipoxygenase (5-LO) as listed above under (a);
(k) anticholinergic agents, including ipratropium bromide, tiotropium bromide, oxitropium bromide, pirenzepine and telenzepine;
(l) β₁- to β₄-adrenoreceptor agonists from the group con metaproterenol, isoproterenol, isoprenaline, albuterol, salbutamol, formoterol, salmeterol, terbutaline, orciprenaline, bitolterol and pirbuterol;
(m) theophylline and aminophylline;
(n) sodium cromoglycate;
(o) muscarinic receptor (M1, M2 and M3) antagonists;
(p) COX-1 inhibitors (NSAIDs) and nitric oxide NSAIDs;
(q) the COX-2 selective inhibitor rofecoxib;
(r) insulin-like growth factor type I (IGF-1) mimetics;
(s) ciclesonide;
(t) inhalation glucocorticoids with reduced systemic side effects from the group prednisone, prednisolone, flunisolide, triamcinolone acetonide, beclomethasone dipropionate, budesonide, fluticasone propionate and mometasone furoate;
(u) tryptase inhibitors;
(v) antagonists of platelet activating factor (PAF);
(w) monoclonal antibodies against endogenous inflammatory entities;
(x) IPL 576;
(y) antitumour necrosis factor (TNFα) agents from the group etanercept, infliximab and D2E7;
(z) DMARDs from the group leflunomide;
(aa) TCR peptides;
(bb) interleukin converting enzyme (ICE) inhibitors;
(cc) IMPDH inhibitors;
(dd) adhesion molecule inhibitors, including VLA-4 antagonists;
(ee) cathepsins;
(ff) MAP kinase inhibitors;
(gg) glucose 6-phosphate dehydrogenase inhibitors;
(hh) kinin B₁ and B₂ receptor antagonists;
(ii) gold in the form of an aurothio group together with various hydrophilic groups;
(jj) immunosuppressive agents from the group cyclosporine, azathioprine and methotrexate;
(kk) anti-gout agents from the group colchicines;
(ll) xanthine oxidase inhibitors from the group allopurinol;
(mm) uricosuric agents from the group probenecid, sulfinpyrazone and benzbromarone;
(nn) antineoplastic agents, which are antimitotic medicaments from the group vinblastine and vincristine;
(oo) agents for promoting growth hormone secretion;
(pp) inhibitors of matrix metalloproteases (MMPs) from the group stromelysins, collagenases, gelatinases, aggrecanase, collagenase-1 (MMP-1), collagenase-2 (MMP-8), collagenase-3 (MMP-13), stromelysin-1 (MMP-3), stromelysin-2 (MMP-10) and stromelysin-3 (MMP-11);
(qq) transforming growth factor (TGFβ);
(rr) platelet-derived growth factor (PDGF);
(ss) fibroblast growth factor from the group basic fibroblast growth factor (bFGF);
(tt) granulocyte macrophage colony stimulating factor (GM-CSF);
(uu) capsaicin;
(vv) tachykinin NK₁ and NK₃ receptor antagonists from the group NKP-608C, SB233412 (talnetant) and D-4418;
(ww) elastase inhibitors from the group UT-77 and ZD-0892;
and
(xx) adenosine A2a receptor agonists.

24. Medicaments comprising at least one compound of the formula I according to one or more of Claims 1 to 12 and/or pharmaceutically usable salts, solvates and stereoisomers thereof, including mixtures thereof in all ratios, and at least one further medicament active compound.

25. Set (kit) consisting of separate packs of
(a) an effective amount of a compound of the formula I according to one or more of Claims 1 to 12 and/or pharmaceutically usable derivatives, salts and stereoisomers thereof, including mixtures thereof in all ratios,
and
(b) an effective amount of a further medicament active compound.

## Revendications

1. Composés de formule I 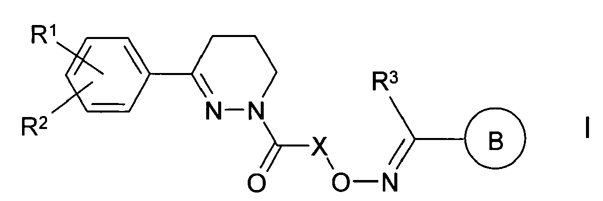 dans laquelle
R¹, R² chacun, indépendamment l'un de l'autre, désignent H, OH, OR⁸, -SR⁸, -SOR⁸, -SO₂R⁸ ou Hal,
R¹ et R² désignent aussi ensemble -OCH₂O- ou -OCH₂CH₂O-,
R³ désigne H, A"R⁷, COA"R⁷, COOA"R⁷, CONH₂, CONHA"R⁷, CON(A"R⁷)(A"'R⁷), CONR¹⁰Hét, NH₂, NHA"R⁷, N(A"R⁷)(A'"R⁷), NCOA"R⁷ ou NCOOA"R⁷,
B désigne phényle, pyridyle, pyridyle N-oxyde, thiényle, furyle, pyrrolyle, pyridazinyle, pyrimidinyle, pyrazinyle, triazinyle, isoxazolinyle, oxazolinyle, thiazolinyle, pyrazolinyle, imidazo- linyle, naphtyle, quinoléinyle, isoquinoléinyle, cinnolinyle, phtalazinyle, quinazolinyle ou quinoxalinyle, chacun d'entre eux étant non substitué ou pouvant être mono-, di- ou trisubs- titué par R⁴, R⁵ et/ou R⁶,
X désigne alkylène ayant 1-10 atomes de C ou alcénylène ayant 2-8 atomes de C,
où un, deux ou trois groupements CH₂ peuvent être rempla- cés par O, S, SO, SO₂, NH ou NA"R⁷, 1-7 atomes de H peu- vent être remplacés par F et/ou Cl,
R⁴ R⁵, R⁶ chacun, indépendamment l'un de l'autre, désignent H, A"R⁷ OH, OA"R⁷, NO₂, NH₂, NHA"R⁷, N(A"R⁷)(A'"R⁷) , NHCOA"R⁷, NHCOOA"R⁷, NHCONH₂, NHCONHA"R⁷, NHCON(A"R⁷)(A"'R⁷), Hal, COOH, COOA"R⁷, CONH₂, CONHA"R⁷, CON(A"R⁷)(A"'R⁷), 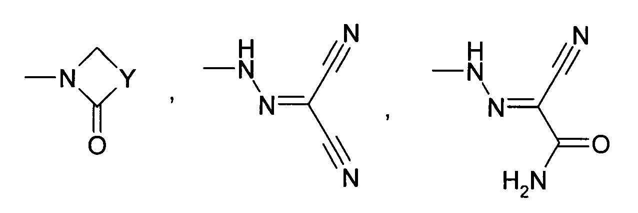 ou 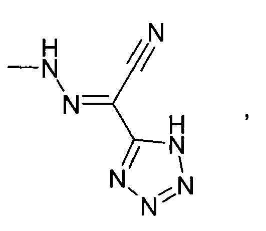
R⁷ désigne H, COOH, COOA, CONH₂, CONHA, CONAA', NH₂, NHA, NAA', NCOA, NCOOA, OH ou OA,
R⁸ désigne A, cycloalkyle ayant 3-7 atomes de C, alkylènecyclo- alkyle ayant 4-8 atomes de C ou alcényle ayant 2-8 atomes de C,
R⁹ désigne alkyle ayant 1-10 atomes de C, cycloalkyle ayant 3-7 atomes de C, alkylènecycloalkyle ayant 4-8 atomes de C ou alcényle ayant 2-8 atomes de C,
où un, deux ou trois groupements CH₂ peuvent être rempla- cés par O, S, SO, SO₂, NH, NMe, NEt et/ou par des groupe- ments -CH=CH-, 1-7 atomes de H peuvent être remplacés par F et/ou CI
et/ou 1 atome de H peut être remplacé par R⁷,
Y désigne alkylène ayant 1-10 atomes de C ou alcénylène ayant 2-8 atomes de C,
où un, deux ou trois groupements CH₂ peuvent être rempla- cés par O, S, SO, SO₂, NH ou NR⁹ et/ou 1-7 atomes de H peuvent être remplacés par F et/ou CI,
A, A' chacun, indépendamment l'un de l'autre, désignent alkyle ayant 1-10 atomes de C ou alcényle ayant 2-8 atomes de C,
où un, deux ou trois groupements CH₂ peuvent être rempla- cés par O, S, SO, SO₂, NH ou NR⁹ et/ou 1-7 atomes de H peuvent être remplacés par F et/ou CI,
ou aryle ou Hét,
A et A' désignent aussi ensemble une chaîne alkylène ayant 2-7 atomes de C, où un, deux ou trois groupements CH₂ peuvent être remplacés par O, S, SO, SO₂, NH, NR⁹, NCOR⁹ ou NCOOR⁹,
A", A"' sont chacun, indépendamment l'un de l'autre, absents, dési- gnent alkylène ayant 1-10 atomes de C, alcénylène ayant 2-8 atomes de C ou cycloalkylène ayant 3-7 atomes de C, où un, deux ou trois groupements CH₂ peuvent être remplacés par O, S, SO, SO₂, NH ou NR⁹ et/ou 1-7 atomes de H peuvent être remplacés par F et/ou CI,
A" et A"' désignent aussi ensemble une chaîne alkylène ayant 2-7 atomes de C, où un, deux ou trois groupements CH₂ peuvent être remplacés par O, S, SO, SO₂, NH, NR⁹, NCOR⁹ ou NCOOR⁹,
aryle désigne phényle, naphtyle, fluorényle ou biphényle, chacun d'entre eux étant non substitué ou mono-, di- ou trisubstitué par Hal, R¹¹, OR¹⁰ N(R¹⁰)2, NO₂, CN, COOR¹⁰, CON(R¹⁰)2, NR¹⁰COR¹⁰, NR¹⁰CON(R¹⁰)2, NR¹⁰SO₂A, COR¹⁰, SO₂N(R¹⁰)_{2,} S(0)ₘR¹1^{,}
R¹⁰ désigne H ou alkyle ayant 1-6 atomes de C,
R¹¹ désigne alkyle ayant 1-6 atomes de C,
Hét désigne un cycle hétérocyclique mono- ou bicyclique saturé, insaturé ou aromatique ayant de 1 à 2 atomes de N, O et/ou S, pouvant être non substitué ou mono- ou disubstitué par oxygène carbonyle, Hal, R¹¹, OR¹⁰, N(R¹⁰)2, NO₂, CN, COOR¹⁰, CON(R¹⁰)2, NR¹⁰COR¹⁰, NR¹⁰CON(R¹⁰)2, NR¹⁰SO₂R¹¹, COR¹⁰, SO₂NR¹⁰ et/ou S(O)ₘR¹¹
Hal désigne F, CI, Br ou l,
m désigne 0, 1 ou 2,
et les sels, solvats, stéréoisomères et isomères E/Z pharmaceutiquement utilisables de ceux-ci, y compris leurs mélanges en toutes proportions.

2. Composés selon la revendication 1, dans lesquels
R¹, R² chacun, indépendamment l'un de l'autre, désignent alcoxy ayant 1, 2, 3, 4, 5 ou 6 atomes de C,
et les sels, solvats, stéréoisomères et isomères E/Z pharmaceutiquement utilisables de ceux-ci, y compris leurs mélanges en toutes proportions.

3. Composés selon la revendication 1, dans lesquels
R¹, R² chacun, indépendamment l'un de l'autre, désignent H, méthoxy, éthoxy, benzyloxy, propoxy, isopropoxy, difluorométhoxy, F, CI, cyclopentyloxy, cyclohexyloxy ou cycloheptyloxy,
et les sels, solvats, stéréoisomères et isomères E/Z pharmaceutiquement utilisables de ceux-ci, y compris leurs mélanges en toutes proportions.

4. Composés selon la revendication 1, dans lesquels
R¹, R² chacun, indépendamment l'un de l'autre, désignent méthoxy, éthoxy, propoxy, isopropoxy, cyclopentyloxy ou F,
et les sels, solvats, stéréoisomères et isomères E/Z pharmaceutiquement utilisables de ceux-ci, y compris leurs mélanges en toutes proportions.

5. Composés selon l'une ou plusieurs des revendications 1-4, dans lesquels
R¹ désigne 4-méthoxy,
R² désigne 3-éthoxy ou 3-propoxy,
et les sels, solvats, stéréoisomères et isomères E/Z pharmaceutiquement utilisables de ceux-ci, y compris leurs mélanges en toutes proportions.

6. Composés selon l'une ou plusieurs des revendications 1-5,
dans lesquels
R³ désigne H, A"R⁷, COA"R⁷, CON(A"R⁷)(A"'R⁷) ou CO-NR¹⁰-Hét,
et les sels, solvats, stéréoisomères et isomères E/Z pharmaceutiquement utilisables de ceux-ci, y compris leurs mélanges en toutes proportions.

7. Composés selon l'une ou plusieurs des revendications 1-6, dans lesquels
X désigne méthylène, éthylène, propylène ou butylène,
et les sels, solvats, stéréoisomères et isomères E/Z pharmaceutiquement utilisables de ceux-ci, y compris leurs mélanges en toutes proportions.

8. Composés selon l'une ou plusieurs des revendications 1-7, dans lesquels
B désigne phényle, pyridyle, pyridyle N-oxyde, thiényle, furyle, pyrro- lyle, pyridazinyle, pyrimidinyle, pyrazinyle, triazinyle, isoxazolinyle, oxazolinyle, thiazolinyle, pyrazolinyle, imidazolinyle, naphtyle, quinoléinyle, isoquinoléinyle, cinnolinyle, phtalazinyle, quinazolinyle ou quinoxalinyle, chacun d'entre eux étant non substitué ou pou- vant être mono-, di- ou trisubstitué par OH, OA, NO₂, NH₂, NAA', 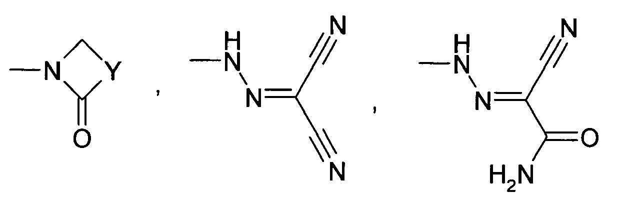 ou 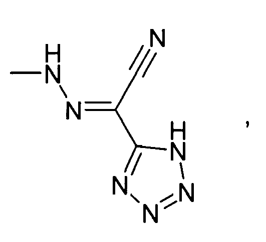
et les sels, solvats, stéréoisomères et isomères E/Z pharmaceutiquement utilisables de ceux-ci, y compris leurs mélanges en toutes proportions.

9. Composés selon l'une ou plusieurs des revendications 1-8, dans lesquels
B désigne phényle qui est non substitué ou monosubstitué par OR¹⁰, NO₂ ou 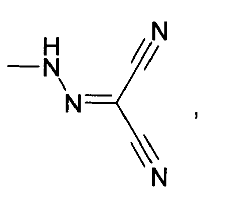 ou pyridyle N-oxyde ou pyridyle non substitué,
et les sels, solvats, stéréoisomères et isomères E/Z pharmaceutiquement utilisables de ceux-ci, y compris leurs mélanges en toutes proportions.

10. Composés selon l'une ou plusieurs des revendications 1-9, dans lesquels
R¹, R² chacun, indépendamment l'un de l'autre, désignent alcoxy ayant 1, 2, 3, 4, 5 ou 6 atomes de C,
X désigne méthylène, éthylène, propylène ou butylène,
R³ désigne H, A"R⁷, COA"R⁷, CON(A"R⁷)(A"'R⁷) ou CO-NR¹⁰-Hét,
A", A"' sont chacun, indépendamment l'un de l'autre, absents ou dési- gnent alkylène ayant 1-10 atomes de C, où l'un des groupements CH₂ peut être remplacé par NH ou NR⁹,
A", A"' désignent aussi ensemble une chaîne alkylène ayant 2-7 atomes de C, où l'un des groupements CH₂ peut être remplacé par NH ou NR⁹
B désigne phényle qui est non substitué ou monosubstitué par OR¹⁰, NO₂, 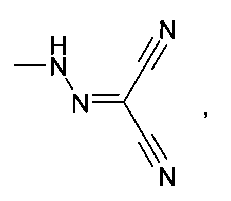 NH₂ ou NHCOOA"R⁷, ou pyridyle N-oxyde ou pyridyle non substitué,
R⁷ désigne H, COOH, NHA ou NAA',
R⁹ désigne alkyle ayant 1-6 atomes de C,
R¹⁰ désigne H ou alkyle ayant 1-6 atomes de C,
A, A' chacun, indépendamment l'un de l'autre, désignent alkyle ayant 1- 10 atomes de C, où 1-7 atomes de H peuvent être remplacés par F et/ou CI,
Hét désigne un radical hétérocyclique monocyclique saturé ayant de 1 à 2 atomes de N, pouvant être mono- ou disubstitué par alkyle ayant 1-6 atomes de C,
et les sels, solvats, stéréoisomères et isomères E/Z pharmaceutiquement utilisables de ceux-ci, y compris leurs mélanges en toutes proportions.

11. Composés selon l'une ou plusieurs des revendications 1-10, dans lesquels
R¹, R² chacun, indépendamment l'un de l'autre, désignent alcoxy ayant 1, 2, 3, 4, 5 ou 6 atomes de C,
X désigne méthylène, éthylène, propylène ou butylène,
R³ désigne H, A"R⁷, COA"R⁷, CON(A"R⁷)(A"'R⁷) ou CO-NR¹⁰-Hét,
A", A"' sont chacun, indépendamment l'un de l'autre, absents ou dési- gnent alkylène ayant 1-10 atomes de C, où l'un des groupements CH₂ peut être remplacé par NH ou NR⁹,
A", A"' désignent aussi ensemble une chaîne alkylène ayant 2-7 atomes de C, où l'un des groupements CH₂ peut être remplacé par NH ou NR⁹
B désigne phényle qui est non substitué ou monosubstitué par OR¹⁰, NO₂, 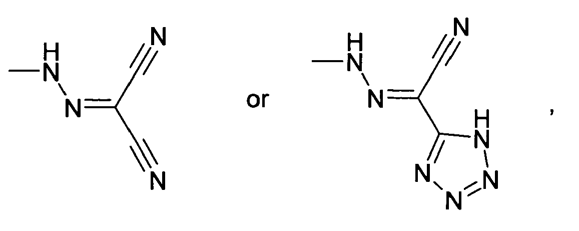 NH₂ ou NHCOOA"R⁷, ou pyridyle N-oxyde ou pyridyle non substitué,
R⁷ désigne H, COOH, NHA ou NAA',
R⁹ désigne alkyle ayant 1-6 atomes de C,
R¹⁰ désigne H ou alkyle ayant 1-6 atomes de C,
A, A' chacun, indépendamment l'un de l'autre, désignent alkyle ayant 1-10 atomes de C, où 1-7 atomes de H peuvent être remplacés par F et/ou Cl,
Hét désigne un radical hétérocyclique monocyclique saturé ayant de 1 à 2 atomes de N, pouvant être mono- ou disubstitué par alkyle ayant 1-6 atomes de C,
et les sels, solvats, stéréoisomères et isomères E/Z pharmaceutiquement utilisables de ceux-ci, y compris leurs mélanges en toutes proportions.

12. Composés de formule I selon la revendication 1, issus du groupe
a) le 4-méthoxybenzaldéhyde O-{2-[3-(3-éthoxy-4-méthoxyphényl)-5,6-dihydro-4H-pyridazin-1-yl]-2-oxoéthyl} oxime,
b) le benzaldéhyde O-{2-[3-(3-éthoxy-4-méthoxyphényl)-5,6-dihydro-4H-pyridazin-1-yl]-2-oxoéthyl} oxime,
c) le 4-hydroxybenzaldéhyde O-{2-[3-(3-éthoxy-4-méthoxyphényl)-5,6-dihydro-4H-pyridazin-1-yl]-2-oxoéthyl} oxime,
d) le pyridine-4-carbaldéhyde O-{2-[3-(3-éthoxy-4-méthoxyphényl)-5,6-dihydro-4H-pyridazin-1-yl]-2-oxoéthyl} oxime,
e) le 1-oxypyridine-4-carbaldéhyde O-{2-[3-(3-éthoxy-4-méthoxyphényl)-5,6-dihydro-4H-pyridazin-1-yl]-2-oxoéthyl} oxime,
f) le 4-méthoxybenzaldéhyde O-{2-[3-(4-méthoxy-3-propoxyphényl)-5,6-dihydro-4H-pyridazin-1-yl]-2-oxoéthyl} oxime,
g) le benzaldéhyde O-{2-[3-(4-méthoxy-3-propoxyphényl)-5,6-dihydro-4H-pyridazin-1-yl]-2-oxoéthyl} oxime,
h) le pyridine-4-carbaldéhyde O-{2-[3-(4-méthoxy-3-propoxyphényl)-5,6-dihydro-4H-pyridazin-1-yl]-2-oxoéthyl} oxime,
i) le 1-oxypyridine-4-carbaldéhyde O-{2-[3-(4-méthoxy-3-propoxyphényl)-5,6-dihydro-4H-pyridazin-1-yl]-2-oxoéthyl} oxime,
j) le 4-nitrobenzaldéhyde O-{2-[3-(4-méthoxy-3-propoxyphényl)-5,6-dihydro-4H-pyridazin-1-yl]-2-oxoéthyl} oxime,
k) le 4-aminobenzaldéhyde O-{2-[3-(4-méthoxy-3-propoxyphényl)-5,6-dihydro-4H-pyridazin-1-yl]-2-oxoéthyl} oxime,
l) le 4-tertio-butyloxycarbonylaminobenzaldéhyde O-{2-[3-(4-méthoxy-3-propoxyphényl)-5,6-dihydro-4H-pyridazin-1-yl]-2-oxoéthyl} oxime,
m) le 2-{[4-({2-[3-(3-éthoxy-4-méthoxyphényl)-5,6-dihydro-4H-pyridazin-1-yl]-2-oxoéthoxyimino}méthyl)phényl]hydrazono}malononitrile,
n) le 2-{[3-({2-[3-(3-éthoxy-4-méthoxyphényl)-5,6-dihydro-4H-pyridazin-1-yl]-2-oxoéthoxyimino}méthyl)phényl]hydrazono}malononitrile,
o) le 2-{[4-({2-[3-(4-méthoxy-3-propoxyphényl)-5,6-dihydro-4H-pyridazin-1-yl]-2-oxoéthoxyimino}méthyl)phényl]hydrazono}malononitrile,
p) l'acide {2-[3-(3-éthoxy-4-méthoxyphényl)-5,6-dihydro-4H-pyridazin-1-yl]-2-oxoéthoxyimino}-2-phénylacétique,
q) le 2-{2-[3-(3-éthoxy-4-méthoxyphényl)-5,6-dihydro-4H-pyridazin-1-yl]-2-oxoéthoxyimino}-N-méthyl-N-(1-méthylpipéridin-4-yl)-2-phénylacétamide,
r) le 1-(4-méthylpipérazin-1-yl)-2-phényléthane-1,2-dione 2-(O-{2-[3-(3-éthoxy-4-méthoxyphényl)-5,6-d ihydro-4H-pyridazin-1-yl]-2-oxoéthyl} oxime,
s) le N-(2-diméthylaminoéthyl)-2-{2-[3-(3-éthoxy-4-méthoxyphényl)-5,6-dihydro-4H-pyridazin-1-yl]-2-oxoéthoxyimino}-2-phénylacétamide,
t) le 2-{[4-({2-[3-(3-éthoxy-4-méthoxyphényl)-5,6-dihydro-4H-pyridazin-1-yl]-2-oxoéthoxyimino}méthyl)phényl]hydrazono}-2-(1 H-tétrazol-5-yl)acétonitrile,
et les sels, solvats, stéréoisomères et isomères E/Z pharmaceutiquement utilisables de ceux-ci, y compris leurs mélanges en toutes proportions.

13. Composés de formule I selon l'une ou plusieurs des revendications 1 à 12, comme inhibiteurs de la phosphodiestérase IV.

14. Procédé de préparation de composés de formule I et de sels et solvats de ceux-ci, **caractérisé en ce que**
a) un composé de formule II 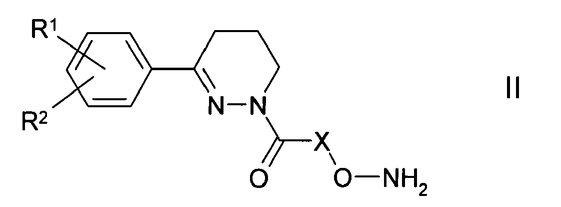 dans laquelle
X, R¹ et R² ont les significations indiquées selon la revendication 1,
est réagi avec un composé de formule III 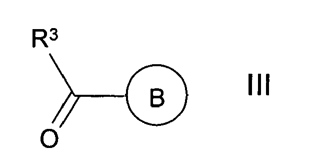 dans laquelle
R³ et B ont les significations indiquées selon la revendication 1,
à condition que, si un autre groupement OH et/ou amino est présent, celui-ci est protégé,
puis un groupement protecteur est éventuellement éliminé par clivage,
ou
b) un composé de formule IV 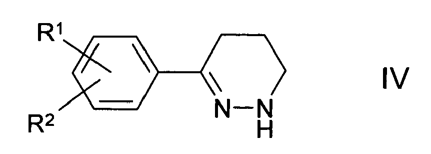 dans laquelle
R¹ et R² ont les significations indiquées selon la revendication 1, est réagi avec un composé de formule V 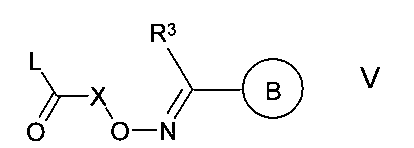 dans laquelle
L désigne Cl, Br, I ou un groupement OH libre ou modifié de manière réactive et fonctionnelle,
et R³ X et B ont les significations indiquées selon la revendication 1,
à condition que, si un autre groupement OH et/ou amino est présent, celui-ci est protégé,
puis un groupement protecteur est éventuellement éliminé par clivage,
ou
c) un composé de formule VI 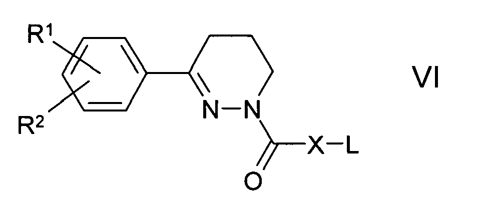 dans laquelle
X, R¹ et R² ont les significations indiquées selon la revendication 1, et
L désigne Cl, Br, I ou un groupement OH libre ou modifié de manière réactive et fonctionnelle,
est réagi avec un composé de formule VII 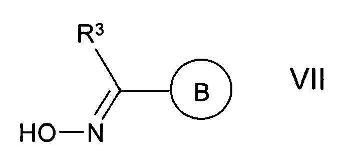 dans laquelle R³ et B ont les significations indiquées selon la revendication 1,
à condition que, si un autre groupement OH et/ou amino est présent, celui-ci est protégé,
puis un groupement protecteur est éventuellement éliminé par clivage,
ou
d) un ou plusieurs radicaux R¹, R² , R³ et/ou B dans un composé de formule I sont convertis en un ou plusieurs autres radicaux R¹, R², R³ et/ou B par
i) clivage d'un éther ou ester,
ii) alkylation ou acylation d'une fonction OH,
iii) alkylation réductrice d'un groupement amino,
iv) réaction d'un groupement amino avec du malononitrile,
v) conversion d'un groupement cyano en groupement tétrazole,
et/ou **en ce qu'**un composé basique de formule I est converti en l'un de ses sels par un traitement par un acide.

15. Médicaments comprenant au moins un composé de la formule I selon l'une ou plusieurs des revendications 1 à 12 et/ou les sels, solvats, stéréoisomères et isomères E/Z pharmaceutiquement utilisables de ceux-ci, y compris leurs mélanges en toutes proportions, et en option des excipients et/ou des adjuvants.

16. Utilisation de composés de la formule I selon l'une ou plusieurs des revendications 1 à 12 et/ou des sels ou solvates physiologiquement acceptables de ceux-ci pour la préparation d'un médicament pour le traitement d'un patient qui souffre d'une maladie ou d'une condition favorisée par l'isozyme de PDE IV dans son rôle de régulation de l'activation et de la dégranulation des éosinophiles humains.

17. Utilisation selon la revendication 16 de composés de la formule I selon l'une ou plusieurs des revendications 1 à 12 et/ou des sels ou solvates physiologiquement acceptables de ceux-ci pour la préparation d'un médicament pour combattre des maladies allergiques, l'asthme, la bronchite chronique, la dermatite atopique, le psoriasis et d'autres maladie de la peau, des maladies inflammatoires, des troubles auto-immunes tels que par exemple l'arthrite rhumatoïde, la sclérose en plaques, la maladie de Crohn, le diabète sucré ou la recto-colite hémorragique, l'ostéoporose, les réactions de rejet de transplants, le marasme, la croissance tumorale ou les métastases tumorales, la sepsie, les troubles de la mémoire, l'athérosclérose et le SIDA.

18. Utilisation selon la revendication 16 ou 17 d'un composé de la formule I selon les revendications 1 à 12 pour la préparation d'un médicament pour le traitement ou la prévention d'une ou de plusieurs maladies, d'un ou de plusieurs troubles et conditions pris parmi le groupe :
l'asthme de tout type, toute étiologie ou toute pathogenèse ; ou l'asthme qui est un élément choisi parmi le groupe comprenant l'asthme atopique ; l'asthme non atopique ; l'asthme allergique ; l'asthme favorisé par IgE bronchial atopique ; l'asthme bronchial ; l'asthme essentiel ; l'asthme vrai ; l'asthme intrinsèque généré par des perturbations pathophysiologiques ; l'asthme extrinsèque généré par des facteurs environnementaux ; l'asthme essentiel de cause inconnue ou inapparente ; l'asthme non atopique ; l'asthme bronchitique ; l'asthme emphysémateux ; l'asthme induit par l'exercice ; l'asthme professionnel ; l'asthme infectieux-allergique généré par une infection bactérienne, fongique, protozoale ou virale ; l'asthme non allergique ; l'asthme incipiens ; le syndrome du petit enfant asthmatique ;
la bronchoconstriction chronique ou aiguë ; la bronchite chronique ; une obstruction légère des voies aériennes ; et l'emphysème ;
la maladie obstructrice ou inflammatoire des voies aériennes de tout type, toute étiologie ou toute pathogénèse ; ou une maladie obstructrice ou inflammatoire des voies aériennes qui est un élément choisi parmi le groupe comprenant l'asthme ; la pneumoconiose ; la pneumonie éosinophilique chronique ; la maladie pulmonaire obstructrice chronique (COPD) ; la COPD qui inclut la bronchite chronique, l'emphysème pulmonaire ou la dyspnée associée ; la COPD qui est **caractérisée par** une obstruction progressive irréversible des voies aériennes ; le syndrome de détresse respiratoire de l'adulte (ARDS) et une exacerbation d'une hyperactivité des voies aériennes en tant que résultat d'une thérapie avec d'autres médicaments ;
la pneumoconiose de tout type, toute étiologie ou toute pathogenèse ; ou la pneumoconiose qui est un élément choisi parmi le groupe comprenant l'aluminose ou la maladie des travailleurs de la bauxite ; l'anthracose ou l'asthme des mineurs ; l'amiantose ou l'asthme des filtres à vapeur ; la silicose ou la maladie de flint ; la trichoptilose générée par l'inhalation de poussières issues de plumes d'autruches ; la sidérose générée par l'inhalation de particules de fer ; la silicose ou la maladie des meuleurs ; la byssinose ou l'asthme de la poussière de coton ; et la pneumoconiose du talc ;
la bronchite de tout type, toute étiologie ou toute pathogenèse ; ou la bronchite qui est un élément choisi parmi le groupe comprenant la bronchite aiguë ; la bronchite laryngotrachéale aiguë ; la bronchite arachidique ; la bronchique catarrhale ; la bronchite croupus ; la bronchite sèche ; la bronchite asthmatique infectieuse ; la bronchite productive ; la bronchite à staphylocoque ou streptocoque ; et la bronchopneumonie ;
la bronchectasie de tout type, toute étiologie ou toute pathogenèse ; ou la bronchectasie qui est un élément choisi parmi le groupe comprenant la bronchectasie cylindrique ; la bronchectasie sacculaire ; la bronchectasie fusocellulaire ; la bronchectasie capillaire ; la bronchectasie cystique ; la bronchectasie sèche ; et la bronchectasie folliculaire ;
la rhinite allergique saisonnière ; ou la rhinite allergique apériodique ; ou la sinusite de tout type, toute étiologie ou toute pathogénèse ; ou la sinusite qui est un élément choisi parmi le groupe comprenant la sinusite purulente ou non purulente ; la sinusite aiguë ou chronique ; et la sinusite ethmoïde, frontale, maxillaire ou sphénoïde ;
l'arthrite rhumatoïde de tout type, toute étiologie ou toute pathogénèse ; ou l'arthrite rhumatoïde qui est un élément choisi parmi le groupe comprenant l'arthrite aiguë ; l'arthrite goutteuse aiguë ; la polyarthrite chronique primaire ; l'ostéoarthrite ; l'arthrite infectieuse ; l'arthrite de Lyme ; l'arthrite progressive; l'arthrite psoriatique ; et l'arthrite vertébrale ;
la goutte ainsi que la fièvre et la douleur associées à une inflammation ;
un trouble pathologique rapporté à l'éosinophile de tout type, toute étiologie ou toute pathogénèse ; ou un trouble rapporté à l'éosinophile qui est un élément choisi parmi le groupe comprenant l'éosinophilie ; l'éosinophilie par infiltration pulmonaire ; le syndrome de Loffier ; la pneumonie éosinophilique chronique ; l'éosinophilie pulmonaire tropicale ; l'aspergillose bronchopneumonique ; l'aspergillome ; les granulomes éosinophiliques ; l'angéite granulomateuse allergique ou le syndrome de Churg-Strauss ; la polyartérite noueuse (PAN) ; et l'angéite nécrosante systémique ;
la dermatite atopique ; ou la dermatite allergique ; ou l'eczéma allergique ou atopique ;
l'urticaire de tout type, toute étiologie ou toute pathogénèse ; ou l'urticaire qui est un élément choisi parmi le groupe comprenant l'urticaire immunofavorisée ; l'urticaire favorisée par complément ; l'urticaire induite par des matériaux urticariogéniques ; l'urticaire induite par agent physique ; l'urticaire induite par stress ; l'urticaire idiopathique ; l'urticaire aiguë ; l'urticaire chronique ; l'oedème angioneurotique ; l'urticaire cholinergique ; l'urticaire au froid sous la forme dominante autosomique ou sous la forme acquise ; l'urticaire par contact ; l'urticaire géante ; et l'urticaire papuleuse;
la conjonctivite de tout type, toute étiologie ou toute pathogénèse ; ou la conjonctivite qui est un élément choisi parmi le groupe comprenant la conjonctivite actinique, la conjonctivite catarrhale aiguë ; la conjonctivite contagieuse aiguë ; la conjonctivite allergique ; la conjonctivite atopique ; la conjonctivite catarrhale chronique ; la conjonctivite purulente ; et la conjonctivite printanière ;
l'uvéite de tout type, toute étiologie ou toute pathogénèse ; ou l'uvéite qui est un élément choisi parmi le groupe comprenant l'inflammation de la totalité ou d'une partie de l'uvée ; l'uvéite antérieure ; l'iritis ; la cyclite ; l'iridocyclite ; l'uvéite granulomateuse ; l'uvéite non granulomateuse ; l'uvéite phacoantigénique ; l'uvéite postérieure ; la choroïdite ; et la choriorétinite ;
le psoriasis ;
la sclérose en plaques de tout type, toute étiologie ou toute pathogénèse ; ou la sclérose en plaques qui est un élément choisi parmi le groupe comprenant la sclérose en plaques progressive primitive ; et la sclérose en plaques cyclique ;
les maladies auto-immunes/inflammatoires de tout type, toute étiologie ou toute pathogénèse ; ou une maladie auto-immune/inflammatoire qui est un élément choisi parmi le groupe comprenant des troubles hématologiques auto-immunes ; l'anémie hémolytique ; l'anémie aplastique ; l'anémie arégénérative ; la purpura thrombopénique idiopathique ; le Lupus Erythematosus systémique; la polychondrite ; la sclérodermie ; la granulomatose de Wegner ; la dermatomyosite ; l'hépatite active chronique ; la myasthénie gravis ; le syndrome de Stevens-Johnson ; la sprue idiopathique ; les maladies inflammatoires auto-immunes de l'intestin ; la recto-colite hémorragique ; la maladie de Crohn ; l'ophthalmopathie endocrine ; la maladie de Basedow ; la sarcoïdose ; l'alvéolite ; la pneumonite à hypersensibilité chronique ; la cirrhose biliaire primitive ; le diabète juvénile ou le diabète sucré de type 1; l'uvéite antérieure; l'uvéite granulomateuse ou postérieure ; la kératoconjonctivite sèche ; la kératoconjonctivite épidémique ; la fibrose pulmonaire interstitielle diffuse ou la fibrose des poumons interstitielle (diffuse) ; la cirrhose pulmonaire ; la fibrose cystique ; l'arthrite psoriatique ; la glomérulonéphrite avec et sans syndrome néphrotique ; la glomérulonéphrite aiguë ; le syndrome néphrotique idiopathique ; la néphropathie à variation minimum, les maladies de la peau inflammatoires/hyperprolifératives ; le psoriasis ; la dermatite atopique ; la dermatite par contact ; la dermatite par contact allergique ; le pemphigus familial bénin ; le Pemphigus Erythematosus ; le Pemphigus Foliaceus ; et le Pemphigus Vulgaris ;
la prévention du rejet de transplant étranger suite à une transplantation d'organe ;
une affection abdominale inflammatoire (IBD) de tout type, toute étiologie ou toute pathogénèse ; ou une affection abdominale inflammatoire qui est un élément choisi parmi le groupe comprenant la recto-colite hémorragique (UC) ; la colite collagène ; la colite polypose ; la colite transmurale ; et la maladie de Crohn (CD) ;
un choc septique de tout type, toute étiologie ou toute pathogénèse ; ou un choc septique qui est un élément choisi parmi le groupe comprenant une insuffisance rénale ; l'insuffisance rénale aiguë ; le marasme ; le marasme paludéen ; le marasme hypophysique ; le marasme urémique ; le marasme cardiaque ; le marasme suprarénal ou la maladie d'Addison ; le marasme cancéreux ; et le marasme en tant que conséquence d'une infection par le virus immunodéficitaire humain (VIH) ;
un endommagement du foie ;
une hypertension pulmonaire ; et une hypertension pulmonaire induite par hypoxie ;
les maladies de perte osseuse ; l'ostéoporose primitive ; et l'ostéoporose secondaire ;
les troubles pathologiques du système nerveux central de tout type, toute étiologie ou toute pathogénèse ; ou un trouble pathologique du système nerveux central qui est un élément choisi parmi le groupe comprenant une dépression ; la maladie de Parkinson ; une altération de l'apprentissage et de la mémoire ; la dyskinésie tardive ; la dépendance aux médicaments ; la démence artériosclérotique ; et les démences qui accompagnent la chorée de Huntington, la maladie de Wilson, la paralysie agitante et les atrophies thalamiques ;
une infection, tout particulièrement une infection due à des virus selon laquelle ces virus augmentent la production de TNF-α dans leurs hôtes ou selon laquelle ces virus sont sensibles à une régulation à la hausse de TNF-α dans leurs hôtes de telle sorte que leur réplication ou leurs autres activités vitales subissent un impact défavorable, incluant un virus qui est un élément choisi parmi le groupe comprenant VIH-1, VIH-2 et VIH-3 ; le cytomégalovirus, soit CMV ; la grippe ; les adénovirus ; et les virus de l'herpès, incluant le Herpes Zoster ou zona d'herpès et le Herpes Simplex ;
les infections dues aux levures et aux champignons où lesdites levures et lesdits champignons sont sensibles à une régulation à la hausse par TNF-α ou élisent une production de TNF-α au niveau de leurs hôtes, par exemple une méningite fongique ; plus particulièrement lors d'une administration en conjonction avec d'autres médicaments de choix pour le traitement des infections par levures et champignons systémiques incluant, sans qu'on soit limité à cela, les polymycines, par exemple Polymycin B ; les imidazoles, par exemple clotrimazole, éconazole, miconazole et cétoconazole ; les triazoles, par exemple fluconazole et itranazole ; et les amphotéricines, par exemple Amphotericin B et Amphotericin B liposomal ;
la blessure de l'ischémie-reperfusion ; les diabètes auto-immunes ; l'auto-immunité rétinienne ; la leucémie lymphocytique chronique ; les infections VIH ; le Lupus Erythematosus ; une maladie du foie et de l'uretère ; les troubles pathologiques urogénitaux et gastro-intestinaux ; et les maladies de la prostate.

19. Utilisation selon la revendication 16, 17 ou 18 d'un composé de la formule I selon des revendications 1 à 12 pour la préparation d'un médicament pour le traitement (1) des maladies inflammatoires et des conditions inflammatoires comprenant une inflammation articulaire, l'arthrite rhumatoïde, la spondylite rhumatoïde, l'ostéoarthrite, l'affection abdominale inflammatoire, la recto-colite hémorragique, la glomérulonéphrite chronique ; la dermatite et la maladie de Crohn ; (2) des maladies respiratoires et des conditions respiratoires comprenant : l'asthme, le syndrome de détresse respiratoire de l'adulte, la maladie inflammatoire pulmonaire chronique, la bronchite, la maladie obstructrice chronique des voies aériennes et la silicose ; (3) des maladies infectieuses et des conditions infectieuses comprenant : la sepsie, le choc septique, le choc endotoxique, le gram-négatif, la sepsie, le syndrome de choc toxique, la fièvre et la myalgie dues à une infection bactérienne, virale ou fongique et la grippe ; (4) des maladies et des conditions immunes comprenant : des diabètes auto-immunes, le lupus erythematosus systémique ; une réaction GvH ; des rejets de transplants étrangers, la sclérose en plaques, le psoriasis et la rhinite allergique ; et (5) d'autres maladies et d'autres conditions comprenant des maladies de la résorption osseuse ; une blessure de reperfusion ; un marasme secondaire résultant d'une infection ou d'une tumeur maligne ; un marasme secondaire résultant du SIDA, une infection du virus immunodéficitaire humain (VIH) ou un complexe rapporté au SIDA (ARC) ; une formation de chéloïde ; une formation de tissu cicatriciel ; un diabète sucré de type 1 ; et la leucémie.

20. Utilisation selon la revendication 16 d'un composé de la formule I selon les revendications 1 à 12 pour la préparation d'un médicament pour le traitement des maladies du myocarde.

21. Utilisation selon la revendication 20 d'un composé de la formule I selon les revendications 1 à 12 pour la préparation d'un médicament pour le traitement de maladies du myocarde, où ces maladies du myocarde présentent des propriétés inflammatoires et immunologiques.

22. Utilisation selon la revendication 16 d'un composé de la formule I selon les revendications 1 à 12 pour la préparation d'un médicament pour le traitement d'une maladie des artères coronaires, un dommage par ischémie/perfusion myocardiale réversible ou irréversible, une défaillance cardiaque aiguë ou chronique et une resténose, incluant une resténose intra-stent et une resténose stent-dans-stent.

23. Combinaison d'un composé selon les revendications 1 à 12 en association avec un ou plusieurs éléments choisis parmi le groupe suivant :
(a) des inhibiteurs de biosynthèse leucotriène, des inhibiteurs de 5-lipoxygénase (5-LO) et des antagonistes de protéine d'activation de 5-lipoxygénase (FLAP) choisis parmi le groupe comprenant zileuton ; ABT-761 ; fenleuton ; tepoxalin ; Abbott-79175 ; Abbott-85761 ; N-(5-substitué)-thiophène-2-alkylsulfonamides ; hydrazones de 2,6-di-tert.-butylphénol ; Zenaca ZD-2138 ; SB-210661 ; composé de 2-cyanonaphthalène substitué par pyridinyle L-739,010 ; composé de 2-cyanoquinoline L-746,530 ; des composés de indole et quinoline MK-591, MK-886 et BAY x 1005 ;
(b) des antagonistes de récepteur pour leucotrines LTB₄, LTC₄, LTD₄ et LTE₄ choisis parmi le groupe comprenant un composé de phénothiazine-3-one L-651,392 ; un composé amidino CGS-25019c ; un composé de benzoxazolamine ontazolast ; un composé de benzènecarboximidamide BIIL 284/260 ; des composés zafirlukast, ablukast, montelukast, pranlukast, verlukast (MK-679), RG-12525, Ro-245913, iralukast (CGP-45715A) et BAY x 7195 ;
(c) des inhibiteurs de PDE IV;
(d) des inhibiteurs de 5-lipoxygénase (5-LO) ; et des antagonistes de protéine d'activation de 5-lipoxygénase (FLAP) ;
(e) des inhibiteurs doubles de 5-lipoxygénase (5-LO) et des antagonistes de facteur d'activation de plaquettes (PAF) ;
(f) des antagonistes de leukotriène (des LTRA) de LTB₄, LTC₄, LTD₄ et LTE₄ ;
(g) cétirizine antagoniste de récepteur H₁ antihistaminique, loratadine, desloratadine, fexofenadine, astemizole, azelastine et chlorphéniramine ;
(h) des antagonistes de récepteur H₂ gastroprotecteur ;
(i) des agents sympathomimétiques vasoconstricteurs agonistes de α₁- et α₂-adrénocepteurs administrés par voie orale ou de façon topique pour une utilisation décongestive, choisis parmi le groupe comprenant propylhexédrine, phénylephrine, phénylepropanolamine, pseudoéphédrine, naphazoline, hydrochlorure, hydrochlorure d'oxymétazoline, hydrochlorure de tétrahydrozoline, hydrochlorure de xylométazoline et hydrochlorure d'éthylnorépinéphrine ;
(j) un ou plusieurs agonistes de α₁- et α₂-adrénocepteurs comme mentionné en (i) ci-avant en combinaison avec un ou plusieurs inhibiteurs de 5-lipoygénase (5-LO) comme mentionné en (a) ci-avant ;
(k) des agents anticholinergiques que sont le bromure d'ipratropium ; le bromure de tiotropium, le bromure d'oxytropium ; la pirenzépine ; et la télenzépine ;
(l) des agonistes de β₁- à β₂-adrénocepteurs choisis parmi le groupe comprenant métaprotérénol, isoprotérénol, isoprénaline, albutérol, salbutamol, formotérol, salmétérol, terbutaline, orciprénaline, bitoltérol et pirbutérol ;
(m) théophylline et aminophylline ;
(n) cromoglycate de sodium ;
(o) des antagonistes de récepteurs muscariniques (M1, M2 et M3) ;
(p) des inhibiteurs de COX-1 (des NSAID) ; et des NSAID d'oxyde nitrique ;
(q) un inhibiteur sélectif de COX-2 rofecoxib ;
(r) des mimétiques de facteur de croissance de type I similaires à l'insuline (IGF-1);
(s) ciclésonide ;
(t) des glucocorticoïdes inhalés avec effets secondaires systémiques réduits choisis parmi le groupe comprenant prédnisone, prédnisolone, flunisolide, acétonide de triamcinolone, dipropionate de beclométhasone, budésonide, propionate de fluticasone et furoate de mométasone ;
(u) des inhibiteurs de tryptase ;
(v) des antagonistes de facteur d'activation de plaquettes (PAF);
(w) des anticorps monoclonaux actifs vis-à-vis d'entités inflammatoires endogènes ;
(x) IPL 576 ;
(y) des agents de facteur de nécrose anti-tumoral (TNFα) choisis parmi le groupe comprenant étanercept, infliximab et D2E7 ;
(z) des DMARD choisis parmi le groupe constitué par leflunomide ;
(aa) des peptides TCR ;
(bb) des inhibiteurs d'enzyme de conversion interleukine (ICE) ;
(cc) des inhibiteurs de IMPDH ;
(dd) des inhibiteurs de molécule d'adhérence incluant des antagonistes de VLA-4 ;
(ee) des cathepsines ;
(ff) des inhibiteurs de kinase MAP ;
(gg) des inhibiteurs de déshydrogénase de phosphate de glucose-6 ;
(hh) des antagonistes de Kinin-B₁ et B₂-récepteurs;
(ii) de l'or sous la forme d'un groupe aurothio en combinaison avec des groupes hydrophiles ;
(jj) des agents immunosuppresseurs choisis parmi le groupe comprenant cyclosporine, azathioprine et méthotrexate ;
(kk) des agents anti-goutte choisis parmi le groupe comprenant des colchicines ;
(ll) de l'oxydase de xanthine choisie parmi le groupe comprenant allopurinol ;
(mm) des agents uricosuriques choisis parmi le groupe comprenant probénécide, sulfinpyrazone et benzbromarone ;
(nn) des agents antinéoplastiques qui sont des médicaments antimitotiques choisis parmi le groupe comprenant vinblastine et vincristine ;
(oo) des secrétagogues d'hormones de croissance ;
(pp) des inhibiteurs de métalloprotéases en matrice (MMP) qui sont choisis parmi le groupe comprenant les stromélysines, les collagènes, les gélatinases, l'aggrécanase, la collagénase -1 (MMP-1), la collagénase -2 (MMP-8), la collagénase -3 (MMP-13), la stromélysine-1 (MMP-3), la stromélysine-2 (MMP-10) et la stromélysine-3 (MMP-11),
(qq) un facteur de croissance de transformation (TGFβ);
(rr) un facteur de croissance dérivé de plaquettes (PDGF) ;
(ss) un facteur de croissance de fibroblast choisi parmi le groupe comprenant un facteur de croissance de fibroblast basique (bFGF) ;
(tt) un facteur de stimulation de colonie macrophage granulocyte (GM-CSF) ;
(uu) la capsaïcine ;
(vv) des antagonistes de tachykinine NK₁ et NK₃ choisis parmi le groupe comprenant NKP-608C ; SB-233412 (talnetant) ; et D-4418 ;
(ww) des inhibiteurs d'élastases choisis parmi le groupe comprenant UT-77 et ZD-0892;
et
(xx) des agonistes de récepteur d'adénosine A2a.

24. Médicaments comprenant au moins un composé de la formule I selon l'une ou plusieurs des revendications 1 à 12 et/ou les sels, solvates et stéréoisomères utilisables pharmaceutiquement de ceux-ci, y compris leurs mélanges en toutes proportions, et au moins un autre ingrédient actif de médicament.

25. Jeu (kit) constitué par des packs séparés de
(a) une quantité efficace d'un composé de la formule I selon l'une ou plusieurs des revendications 1 à 12 et/ou des dérivés, sels et stéréoisomères de ceux-ci, y compris leurs mélanges en toutes proportions ; et
(b) une quantité efficace d'un autre ingrédient actif de médicament.
